# EUROPEAN PATENT SPECIFICATION

(11) **EP 4 137 582 B1**
(45) Date of publication and mention of the grant of the patent: **28.01.2026**
(21) Application number: 22190872.6
(22) Date of filing: 17.08.2022
(51) Int. Cl.: C12Q 1/6844

(54) **ROLLING CIRCLE REVERSE TRANSCRIPTION OF CIRCULAR RNA**
ROLLENDE ZIRKULÄRE REVERSE-TRANSKRIPTION MIT ZIRKULÄRER RNA
TRANSCRIPTION INVERSE PAR CERCLE ROULANT D'ARN CIRCULAIRE

(30) Priority: 17.08.2021 US 202163260323 P
(43) Date of publication of application: 22.02.2023
(60) Divisional application: 25225612.8
(73) Proprietor: NEW ENGLAND BIOLABS, INC., Ipswich, MA 01938-2723 (US)
(72) Inventor: GUAN, Shengxi, Ipswich, 01938-2723 (US); MAGUIRE, Sean, Ipswich, 01938-2723 (US); XU, Yan, Ipswich, 01938-2723 (US); UNLU, Irem, Ipswich, 01938-2723 (US)
(74) Representative: Griffin, Philippa Jane

(56) References cited:
- WO-A1-2019/005955
- LIU YAQIAN ET AL: "Direct detection of circRNA in real samples using reverse transcription-rolling circle amplification", ANALYTICA CHIMICA ACTA, ELSEVIER, AMSTERDAM, NL, vol. 1101, 26 December 2019 (2019-12-26), pages 169 - 175, XP085991200, ISSN: 0003-2670, [retrieved on 20191226], DOI: 10.1016/J.ACA.2019.12.027
- S. MOHR ET AL: "Thermostable group II intron reverse transcriptase fusion proteins and their use in cDNA synthesis and next-generation RNA sequencing", RNA, vol. 19, no. 7, 22 May 2013 (2013-05-22), pages 958 - 970, XP055149277, ISSN: 1355-8382, DOI: 10.1261/rna.039743.113
- ZHAO CHEN ET AL: "An ultraprocessive, accurate reverse transcriptase encoded by a metazoan group II intron", 6 November 2017 (2017-11-06), pages 183 - 195, XP093011527, Retrieved from the Internet <URL:https://rnajournal.cshlp.org/content/24/2/183> DOI: 10.1261/rna.063479.117
- MART�N-ALONSO SAMARA ET AL: "Reverse Transcriptase: From Transcriptomics to Genome Editing", TRENDS IN BIOTECHNOLOGY, ELSEVIER PUBLICATIONS, CAMBRIDGE, GB, vol. 39, no. 2, 8 July 2020 (2020-07-08), pages 194 - 210, XP086446241, ISSN: 0167-7799, [retrieved on 20200708], DOI: 10.1016/J.TIBTECH.2020.06.008
- MAGUIRE SEAN ET AL: "Rolling circle reverse transcription enables high fidelity nanopore sequencing of small RNA", PLOS ONE, vol. 17, no. 10, 10 October 2022 (2022-10-10), pages e0275471, XP093011530, DOI: 10.1371/journal.pone.0275471
- MARCEL BOSS ET AL: "A Fast and Easy Method for Specific Detection of Circular RNA by Rolling-Circle Amplification", CHEMBIOCHEM, JOHN WILEY & SONS, INC, HOBOKEN, USA, vol. 21, no. 6, 27 November 2019 (2019-11-27), pages 793 - 796, XP072202813, ISSN: 1439-4227, DOI: 10.1002/CBIC.201900514

## Description

### BACKGROUND

The variety and significance of RNA has expanded over time so that today many species of RNAs of varying abundance, sizes, structures, and functions have been described. Circular RNAs (circRNA) are found in all kingdoms of life; thousands have been identified across species from Archaea to humans. CircRNAs were previously thought to be insignificant byproducts of splicing errors. However, more recent studies suggest that most circRNAs found in nature are generated through back splicing *in vivo.*

CircRNAs are associated with multiple functions in the multicellular host. They participate in modulating certain protein-protein and protein-RNA interactions (Kristensen, et al., Nature Reviews Genetics, 20, 675-691 (2019)). CircRNAs are believed to act as sponges for microRNA (miRNA) and proteins and may additionally act as protein decoys, scaffolds, and recruiters. Some circRNAs act as translation templates in multiple pathophysiological processes. CircRNAs bind and sequester specific proteins to appropriate subcellular positions. Functions of circRNA include: providing a template for translation of proteins associated with cancer such as circ ZNF609 in mammalian myoblasts; regulation of gene expression such as circEIF3J; indirect regulation of miRNA target genes, e.g. CDR1/ciRS-7 in mammalian brain and miR7; and regulation of RNA binding protein dependent function, e.g. CircMbl in Drosophila and Mannan binding lectin (MBL) protein. Cancer studies have revealed that the degree of circRNA production is correlated with disease progression where 11.3% of circRNA is essential for cell proliferation. Moreover, circRNA levels increase in the brain is age-associated neurological disorders such as Alzheimer's disease and Parkinson's disease (see for example, Vo, et al. Cell (2019) 176, 869-881).

Endogenous circRNAs lack the free ends necessary for exonuclease-mediated degradation, rendering them resistant to several mechanisms of RNA turnover and granting them extended lifespans as compared to their linear mRNA counterparts. For this reason, circularization may allow for the stabilization of mRNAs that generally suffer from short half-lives and may therefore improve the overall efficacy of exogenous mRNA in a variety of applications including RNA vaccines and RNA based therapeutics (see for example: Salzman, et al. PLoS One. 2012, 7(2):e30733; Muller, et al. RNA Biol 2017, 14(8): 1018-1027; and Wessel Hoeft, et al. Nature Communications (2018) 9, Article 2629).

There is a continuing need to be able to learn more about naturally occurring circRNAs including their sequences. Sensitive analytical tools are required to detect and accurately sequence circRNAs that range in size from very small (20 nucleotides (nt)) to significantly large (10 kb or more). Recently with the advent of vaccines and therapeutics based on mRNAs, circularizing in vitro synthesized large linear RNA (greater than 100 bases) is one approach to stabilizing these molecules. Such methods result in the need for analysis and sequencing of large circRNAs to determine the quality of the synthetic product. Such sequencing methods should preferably be fast, efficient, reliable and show reduced bias.

Y. Liu et al., Analytica Chimica Acta, Vol. 1101, pages 169-175 (26 December 2019) describes direct detection of circRNA in real samples using reverse transcription-rolling circle amplification. S. Mohr et al., RNA, Vol. 19(7), pages 958-970 (22 May 2013) describes thermostable group II intron reverse transcriptase fusion proteins and their use in cDNA synthesis and next-generation RNA sequencing. Z. Chen et al., RNA, Vol. 24(2), pages 183-195 (6 November 2017) describes an ultraprocessive, accurate reverse transcriptase encoded by a metazoan group II intron. S. Martin-Alonso et al. 2020, Trends in Biotechnology, Vol. 39 (2), pages 194-210 (8 July 2020) describes reverse transcriptase: From Transcriptomics to Genome Editing. S. Maguire et al., PLOS ONE, Vol. 17(10), page e0275471, reports that rolling-circle reverse transcription enables high fidelity nanopore sequencing of small RNA. M. Boss et al., ChemBioChem, Vol. 21(6), pages 793-796 (27 November 2019) describes 'a fast and easy' method for specific detection of circular RNA by rolling-circle amplification. WO 2019/005955 describes improved reverse transcriptase and methods of use.

### SUMMARY

The present invention is defined in the independent claims and certain optional features thereof are defined in the dependent claims. The technical information set out below may in some respects go beyond the scope of the invention, which is defined exclusively by the appended claims. The additional technical information is provided to place the actual invention in a broader technical context, and to illustrate possible related technical developments. In so far as the terms "invention", "example" and "embodiment" are used herein, these terms will be interpreted in such a way that protection does not extend beyond the scope of the claims.

In general, methods, compositions and kits are provided that enable the detection, analysis and/or sequencing of small or large target RNA molecules whether synthetic, purified, or within a biological fluid or in cell lysate that may contain non-target RNA and other contaminating molecules, without the need for depletion or purification steps that diminish what might already be low concentrations of the target molecule. The methods, compositions and kits rely on the use of a Group II Intron reverse transcriptase (Intron-RT) that has strand displacing properties and can generate concatamers in cDNA by rolling circle transcription of circRNAs that may be naturally circular or circularized *in vitro* from linear RNA.

In one aspect, a reaction mixture is provided that includes (a) a circular RNA (circRNA); and (b) a Group II bacterial or archaeal Intron-RT. The circRNA in the reaction mixture is selected from: a eukaryotic circRNA, and a synthetic circRNA having an artificial sequence. The reaction mixture may further comprise a ribozyme or a DNA oligonucleotide where the oligonucleotide may be a primer or adapter. In one example, the oligonucleotide primer comprises a 3' end having a target specific complementary sequence for hybridizing to the circRNA; or a degenerate sequence at the 3' end, and optionally a 5' tail. The reaction mixture may also include one or more enzymes selected from the group consisting of a 5'- 3' RNA exonuclease, a 3'- 5' RNA exonuclease, a DNA polymerase, a ligase, a thermolabile proteinase and an endonuclease. For example, where a DNA polymerase is included in the reaction mixture, it may be any of Phi29, Taq, Bst, Bst large fragment, Bsu, Bsu large fragment, E.coli Polymerase I, Klenow, Deep Vent, Vent^{®}, Pfu, KOD, Tgo or 9°N^{™} DNA polymerase (all commercially available from New England Biolabs, Ipswich, MA).

In certain embodiments, the eukaryotic circRNA or the synthetic circRNA is a circularized linear RNA, where the circRNA may be circularized *in vivo* or *in vitro.* In one embodiment, the circRNA is a eukaryotic circRNA, in which case the eukaryotic circRNA in the reaction mixture may be isolated eukaryotic circRNA. The circRNA in the reaction mixture may be isolated (and optionally an enriched and/or purified preparation) from a cell or bodily fluid (e.g. blood, plasma or serum). Alternatively, the reaction mixture may contain a cell lysate or bodily fluid sample (e.g. blood, plasma or serum) comprising the circRNA, where the RNA may have been partially enriched or purified, or has been neither enriched nor purified.

In certain embodiments, the circRNA in the reaction mixture has a size in the range of 20 bases-50 kilobases, such as at least 200 bases, 400 kb, 1kb, 2kb, or 10kb. The reaction mixture may include a concatemeric first strand cDNA that is the product of the reaction between the eukaryotic circRNA in the sample and the bacterial or archaeal reverse transcriptase. Each concatemeric cDNA has multiple repeat units that are complementary copies of the circRNA, and the median length of the concatemeric cDNA is at least 3 times the length of the circRNA (e.g. at least 600 bases, 1.2kb, 3kb, 6kb, or 30kb).

In some embodiments the concatemeric cDNA in the reaction mix comprises at least 20 complementary copies of the circRNA, such as at least 100 copies. In certain examples, the concatemeric cDNA contains at least 500 nucleotides, such as at least 1000, 5000, or 10,000 nucleotides. Another feature of the concatemeric cDNA product is that the repeat units in a single cDNA share more than 90% sequence identity with each other, such as at least 95% or 98% sequence identity.

In general, a method is provided for identifying a circRNA in a sample by characterizing a first strand cDNA molecule, comprising: (a) incubating a sample comprising a circRNA with a Group II Intron-RT and dNTPs, to produce by rolling circle reverse transcription, a reaction product comprising concatemeric first strand cDNA molecules; and (b) characterizing the cDNA by (i) obtaining a sequence of the concatemeric first strand cDNA molecules, wherein the sequence reads represent a consensus complementary sequence of repeat units of the circRNA; or by (ii) amplifying the concatemeric first strand cDNA by a DNA amplification reaction by using primers.

In certain embodiments, the circRNA is isolated from a sample containing eukaryotic cells or bodily fluid; contained within a cell lysate or body fluid; circularized linear RNA; or synthesized *in vitro* creating an artificial sequence. The bodily fluid may be, for example, blood, serum, or plasma. In another example, the circRNA is circularized linear RNA that is a transcription product of a DNA.

In an example of the method, the step of sequencing the cDNA is preceded by a step of detecting the cDNA by amplification. In one embodiment, the methods described herein may include the step of amplifying the full length first strand cDNA concatemer using a randomly-primed amplification method, to produce an amplified concatemer. The first strand cDNA concatemer generated in this way may have a length of at least 500 bases; and contain at least 3 complementary copies of the circRNA. The concatemer cDNA or the double strand DNA amplification product of the concatemer cDNA can be sequenced by long-read sequencing.

A ribozyme or a DNA oligonucleotide such as a primer or an adapter may be included in the incubation step (a) with the circRNA and Intron-RT. In embodiments of the method, rolling circle reverse transcription can be performed at a temperature in the range of 20°C-60°C in which range the Intron-RT can reverse transcribe circRNA. However, it may be preferable to incubate the reaction mixture at a temperature in the range of 50°C-60°C (e.g. at about 55°C) so as to reduce the formation of RNA secondary structure and to enhance the efficiency of rolling circle reverse transcription. The RT reaction may proceed for about 30 minutes or 1 hour. In embodiments of the method, rolling circle reverse transcription can be preceded or followed by an enrichment step that involves enzymatic depletion of linear RNA using a 5'- 3' RNase and a 3'- 5' RNase. Alternatively or additionally, enrichment can be achieved by size separation of the concatemeric first strand cDNA from the non-concatemeric cDNA.

The amount of cDNA copies of the circRNA within a concatemer results in at least a 2 fold higher concentration than can be obtained using a retroviral RT.

In certain embodiments of the method, a DNA polymerase is included in the reaction mixture for rolling circle reverse transcription. Alternatively, the DNA polymerase may be used in a subsequent reaction. The DNA polymerase may be added to the cDNA concatemer to amplify the DNA for ease of quantitative detection or for sequencing. In these circumstances, Phi29 DNA polymerase may be selected for the amplification reaction, in which case, it may be desirable to add an endonuclease such as T7 endonuclease for removing branching of the amplified DNA. Alternatively, any other DNA polymerase may be used as desired for isothermal amplification or for PCR.

Sequencing may be performed on the cDNA directly, after second strand synthesis, or after DNA amplification. Consensus sequence reads are obtained by comparing the sequences for individual units in the concatemers to provide an accurate sequence determination for the circRNA. The consensus sequences also permit determination of the error rate of the Intron-RT for reverse transcription. This permits a determination of the error rate of secondary enzymes such as DNA dependent RNA polymerases and/or ligases on or after copying and/or joining nucleic acid sequences.

In one embodiment, a method is provided for assaying the transcription fidelity of an RNA polymerase, comprising: (a) transcribing a synthetic linear DNA with a DNA dependent RNA polymerase in a reaction mixture; (b) producing a circularized RNA (circRNA) from (a); (c) reverse transcribing the circRNA with a Group II Intron-RT to form a population of concatemeric cDNA; and (d) sequencing the population of cDNA to determine the transcription fidelity of the RNA polymerase. The error rate of the of the RNA polymerase can be determined from comparing the consensus reads from units in individual concatemers. In certain examples, the cDNA can be amplified with a DNA polymerase and/or sequenced by means of long-read sequencing.

In one aspect, falling outside the scope of the claims, a method is provided for amplifying a long linear RNA, which includes: performing first strand cDNA synthesis of a long linear RNA using an Intron-RT; and transcribing *in vitro* the cDNA using a DNA dependent RNA polymerase to make multiple copies of the long linear RNA. In one example, the long linear RNA contains modified bases. The long linear RNA may have a size of at least 1 kilobase (e.g. at least 2kb or at least 10kb). In some embodiments, the long linear RNA is capable of being reverse transcribed by Intron-RT in 30 minutes, e.g. at a reaction temperature of 55°C-60°C.

In general, a kit is provided that includes a (e.g., bacterial or archaeal) Group II Intron-RT, and reagents for circularizing a linear RNA, selected from any of: a splint adapter, an RNA ligase such as T4 RNA ligase, a ribozyme, or chemicals capable of circularizing the RNA. The oligonucleotide may include a degenerate sequence for use as a unique identifier that is incorporated at the completion or start of each rolling circle copy. The kit may further include at least one enzyme selected from the group consisting of a DNA dependent RNA polymerase, a 5'- 3' RNA exonuclease, a 3'- 5' RNA exonuclease, a DNA polymerase, a ligase, a thermolabile proteinase and an endonuclease. At least one of the enzymes selected from the group consisting of an Intron-RT, DNA dependent RNA polymerase, a 5'- 3' RNA exonuclease, a 3'- 5' RNA exonuclease, a DNA polymerase, a ligase, a thermolabile proteinase and an endonuclease may be lyophilized. The lyophilized enzyme may be positioned on the surface of a polymer, within a porous polymer matrix or in a cake within a tube. One or more of the enzymes may be in the same or different containers from the Intron-RT.

In one embodiment, falling outside the scope of the claims, a composition is provided that includes a Group II Intron-RT and a synthetic non-natural RNA oligonucleotide adapter complementary to a DNA splint oligonucleotide.

In another embodiment, falling outside the scope of the claims, a composition is provided that includes: a concatemeric single strand DNA, wherein the concatemer is at least 3 repeat units of a single sequence, wherein (i) the single sequence has a length in the range of 20 bases to 50 kilobases, (ii) the single sequence in each of the 3 repeat units differ no more than 10%; and (iii) the concatemer is a product of rolling circle reverse transcription of an RNA.

In another embodiment, falling outside the scope of the claims, a lyophilized Group II Intron-RT is provided, wherein the lyophilized transcriptase is associated with a polymer matrix. For example, the Intron-RT may be contained within a porous polymer matrix for example, where the Intron-RT is within the polymer matrix in a cylinder, or the Intron-RT is positioned on a surface of the polymer. In this example, the polymer may be bead shaped with the lyophilized Intron-RT on its surface.

In another embodiment, falling outside the scope of the claims, the Group II Intron-RT is combined with a colored dye to form a mixture wherein the colored dye is at a concentration in the range of 0.003% to 1% (w/v), such as in the range of 0.006% to 0.2% or 0.015% to 0.15%; and wherein the reverse transcriptase mixture does not contain Taq polymerase and does not contain RNA. In one example, the colored dye is one or a combination of xylene cyanol, tartrazine, orange G or a combination of two or more of xylene cyanol, orange G and tartrazine.

### BRIEF DESCRIPTION OF THE FIGURES

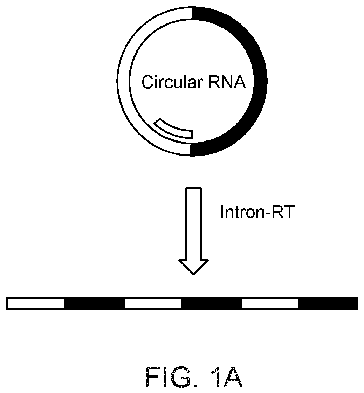

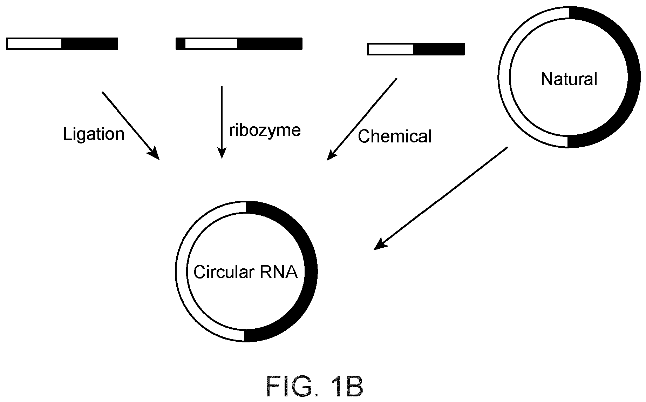

FIG. 1A-FIG. 1B shows cartoons of how rolling circle amplification (RCA) of a circular RNA (circRNA) molecule can be achieved using a Group II Intron reverse transcriptase (Intron-RT). An oligonucleotide primer may be used to initiate reverse transcription (FIG. 1A). The RNA is either naturally circular or is a linear RNA that has been circularized using a ribozyme, by ligation or by chemical means (FIG. 1B). The product of the Intron-RT is a concatemer of complementary single strand DNA copies of the target RNA. The black region and the white region of the circRNA are contiguous sequences in the circRNA.

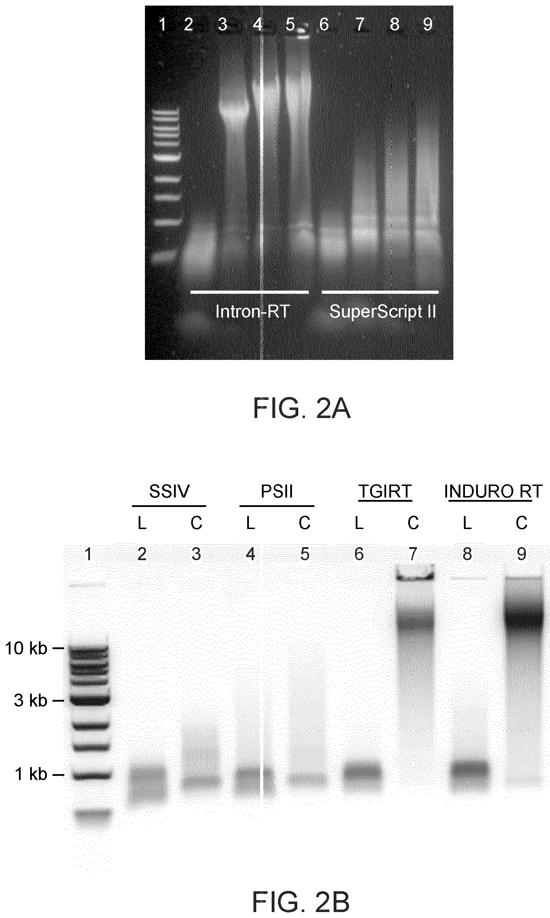

FIG. 2A-FIG. 2B shows that an Intron-RT generates not only larger multiple-repeat cDNA, but also at least a 2 fold greater yield for linear cDNA than obtained by Moloney Murine Leukemia Virus Reverse Transcriptase (M-MLV RT) variants from the same substrate circRNA.
FIG. 2A shows a time course for reverse transcription where Lane 1 is a 10 kb ladder (New England Biolabs, Ipswich, MA). For the Intron-RT generated cDNA, the reaction was incubated at 55°C for 0 minutes (lane 2), 20 minutes (lane 3), 1 hour (lane 4), and 2 hour (lane 5) respectively.
   For the SuperScript^{™} II RT generated cDNA (ThermoFisher Scientific, Waltham, MA), the reaction was incubated at 42°C for 0 minutes (lane 6), 20 minutes (lane 7), 1 hour (lane 8), and 2 hour (lane 9) respectively and not only the yield was noticeably less but also the cDNA size was much shorter in lanes 7-9. The cDNA yield for each reaction condition is 0.06 µg (lane 2), 2.5 µg (lane 3), 4.5 µg (lane 4), 4.4 µg (lane 5), 0.1 µg (lane 6), 0.5 µg (lane 7), 0.6 µg (lane 8), and 0.6 µg (lane 9) respectively.
FIG. 2B shows a similar pattern as FIG. 2A with additional examples of MMLV-RT's (exemplified by SuperScript IV (SSIV) and ProtoScript^{®} II (PSII) (New England Biolabs, Ipswich, MA) and Group II Intron-RTs exemplified by TGIRT^{™} (Ingex, Olivette, MO) and Induro^{™} RT (New England Biolabs, Ipswich, MA). Lane 1 is a 10 kb ladder (New England Biolabs, Ipswich, MA). L denotes linear RNA while C denotes circRNA.
FIG. 3A-FIG. 3C shows that Intron-RT can replicate circRNA having a broad range of sizes to form multiple-repeat cDNA. Lane 1 is a 10 kb size ladder. Lanes 2-5 show yield on increasing incubation times (0 minutes, 20 minutes, 60 minutes, and 120 minutes).
FIG. 3A shows concatemers formed from 1.3 kb circRNAs.
FIG. 3B shows concatemers formed from 0.8 kb circRNAs.
FIG. 3C shows concatemers formed from 0.4 kb circRNAs.

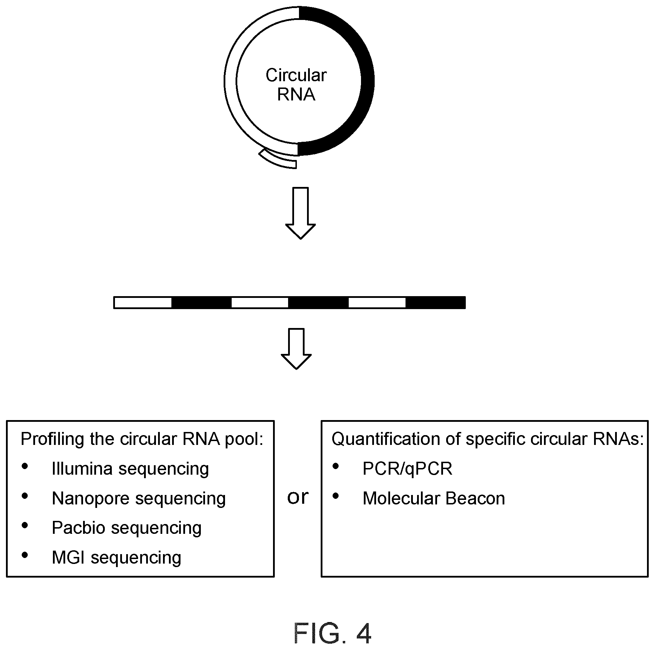

FIG. 4 shows a cartoon of a workflow in which a circRNA (shown in black and white) is primed with a DNA oligonucleotide to produce a linear concatemeric first strand cDNA product of rolling circle RNA amplification having 3 repeat units of the complementary sequence to the circRNA. The linear concatemer may be directly sequenced using for example sequencing platforms such as an Illumina sequencer or MGI sequencer, or a Long-read sequencer such as by Nanopore sequencing, or Pacific Biosciences sequencer. Alternatively, the concatemer can be detected by DNA amplification and/or quantified by quantitative amplification or with molecular beacons.
FIG. 5A-FIG. 5D: Intron-RT shows higher sensitivity than MMLV RT-derived ProtoScript II (PSII) and MMLV-RT variant Superscript IV RT in an RT-qPCR assay in detecting either an *in vitro* synthesized circRNA or a specific circRNA from total brain RNA.

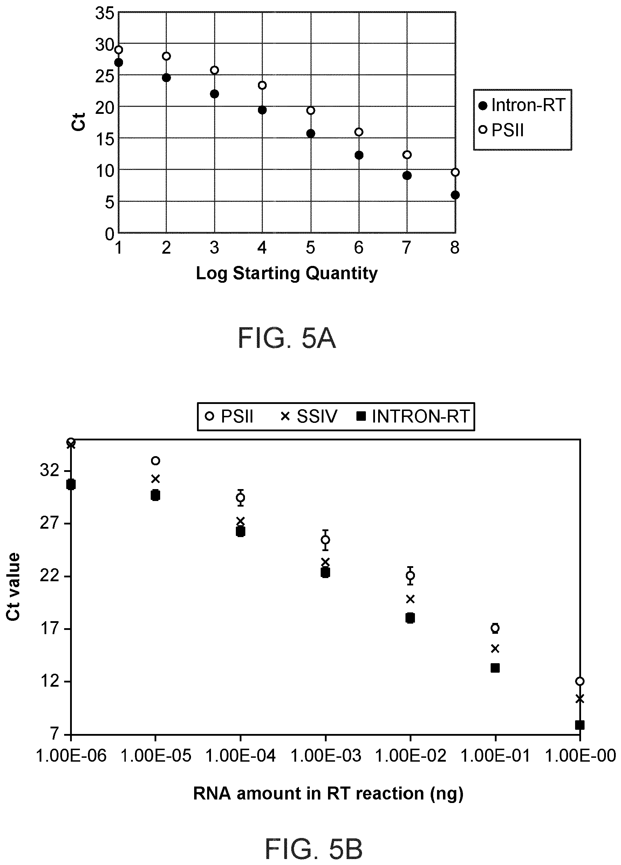

FIG. 5A: Various amount of an in vitro synthesized circRNA (0.5 kb) (0.0001 pg (1), 0.001 pg (2), 0.01 pg (3), 0.1 pg (4), 1 pg (5), 10 pg (6), 100 pg (7), and 1ng (8) was reverse transcribed with either Intron-RT, PSII or SSIV using a circRNA specific primer. qPCR was used to quantify the circRNA. Intron-RT exhibits lower Ct value (higher sensitivity) than PSII in all input. The results show as much as 8 fold (2³) increase in sensitivity using the Intron-RT compared with PSII.
FIG. 5B: Various amounts of an in vitro synthesized circRNA (1.3kb) (0.001 pg (1), 0.01 pg (2), 0.1 pg (3), 1 pg (4), 10 pg (5), 100 pg (6), and 1 ng (7)) were reverse transcribed with PS II, SSIV, or Induro RT using a circRNA specific primer. qPCR was used to quantify the circRNA. Induro RT exhibits lower Ct value (higher sensitivity) than other reverse transcriptases in all input. The results show as much as 16 fold (2⁴) increase in sensitivity using an Intron-RT compared with a viral RT.

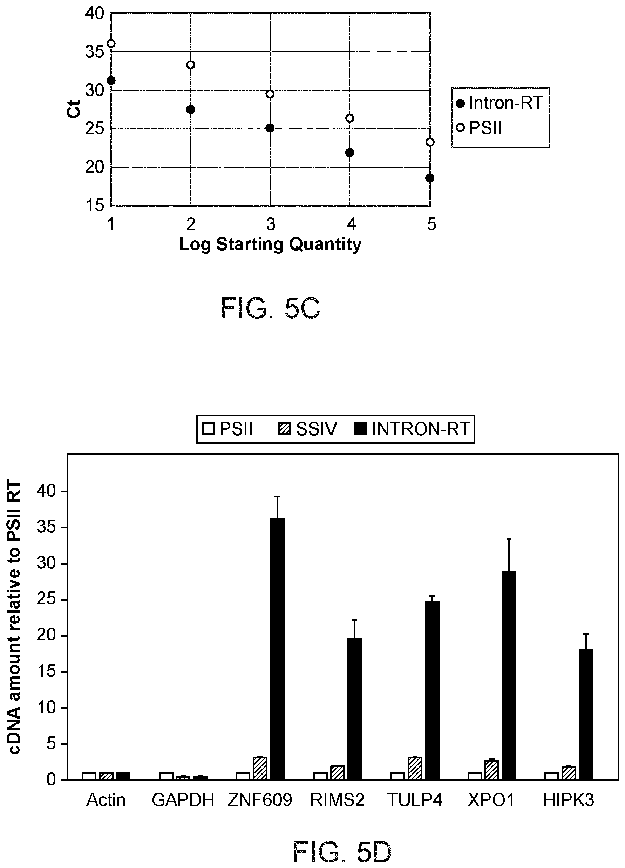

FIG. 5C shows that the use of an Intron-RT provides a higher sensitivity of detection for a specific circRNA (XOP1) in human brain via RT-qPCR than with a viral RT. The results show as much as a 32 fold (2⁵) increase in sensitivity using the Intron-RT compared to viral RT.
FIG. 5D tests the sensitivity of Intron-RT with additional endogenous circRNAs from human brain and shows that the use of Intron-RT provides a higher sensitivity of detection for all tested endogenous circRNAs from human brain (ZNF609, RIMS2, TULP4, XPO1 and HIPK3) via RT-qPCR than PSII, and SSIV. The results show as much as a 23 fold increase in sensitivity using the Intron-RT compared with viral RT.

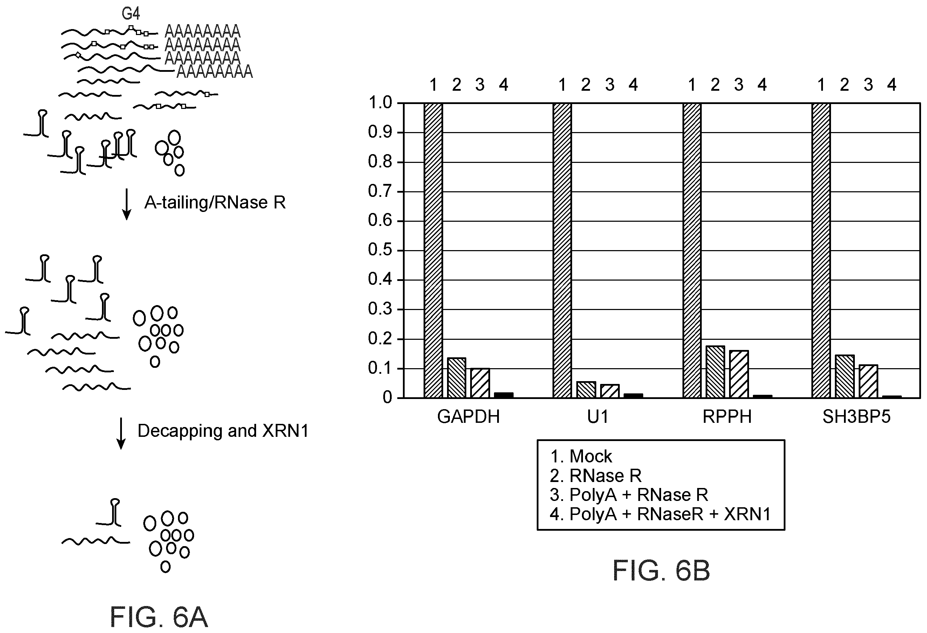

FIG. 6A-FIG. 6C shows a combination of a 5'->3' exoribonuclease (XRN-1) and a 3'->5' exoribonuclease (RNaseR) for depleting contaminating abundant linear RNAs can enhance circRNA enrichment from human brain total RNA. The workflow may be followed by rolling circle reverse transcription and sequencing using any available sequencing platform e.g. Illumina, Nanopore, PacBio and MGI sequencing and/or analysis using PCR, qPCR or molecular beacon methods.
FIG. 6A shows schemes of enrichment of circRNA prior to RCA using RNase R (a 3'->5' exoribonuclease) and XRN-1 (a 5'->3' exoribonuclease). Linear RNA is efficiently degraded thereby enriching for circRNA.
FIG. 6B shows that applying a 5'->3' exoribonuclease (XRN-1) and a 3'->5' exoribonuclease (RNaseR) together in (4) results in enhanced depletion of linear RNA (GAPDH, U1, RPPH and SH3BP5) in a total RNA preparation compared to no RNase (1), RNase R on its own (2), Poly A and RNase R (3).

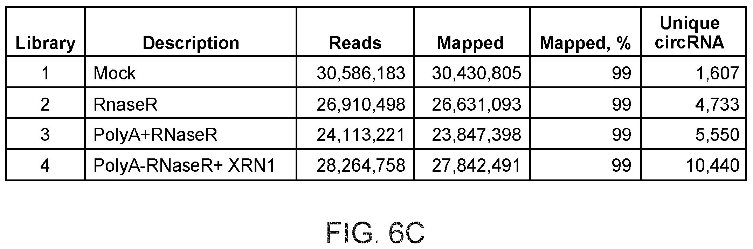

FIG. 6C shows the number of unique circRNA detected when the four different strategies from FIG. 6B (library 1, 2, 3 and 4) were used to deplete linear RNA to enrich circRNA. Applying RNase R and XRN-1 together enabled at least two fold more mapped unique circRNAs based on sequence reads compared with RNaseR alone or RNase R and poly A.

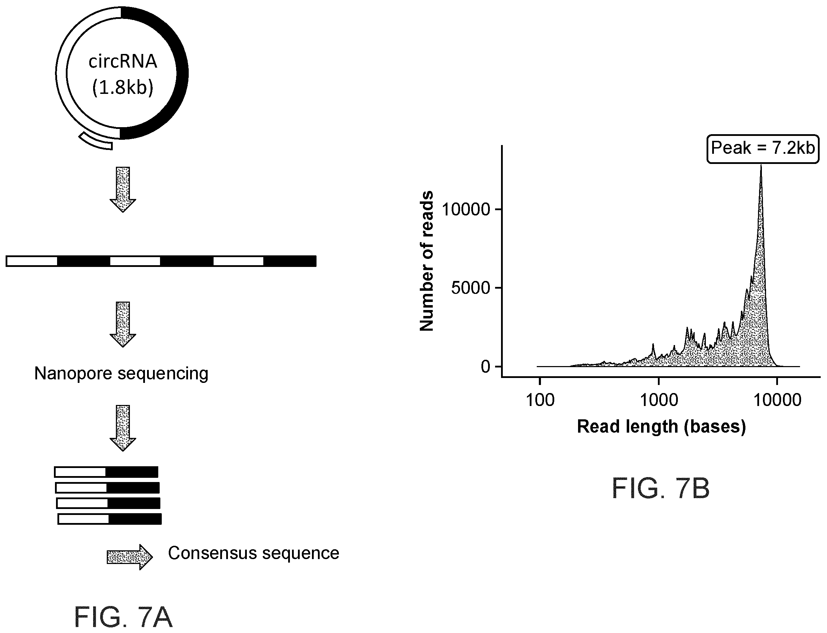

FIG. 7A-FIG. 7H shows that high quality sequence data can be obtained from a Nanopore sequencing platform using Intron-RT and *in vitro* circularized circRNAs with a single primer to generate a cDNA containing multiple repeats. The multiple repeats enable generating consensus sequences that correct artifactual errors from the nanopore sequencing platform.
FIG. 7A shows a workflow for forming cDNA concatemers from an *in vitro* synthesized circRNA (1.8 kb) and sequencing these via Nanopore sequencing.
FIG. 7B shows an example of the read length distribution of multi-repeat cDNA from Nanopore sequence. The major peak is 7.2 kb, which equals to approximately 4 copies of circRNA sequence (1.8 kb).

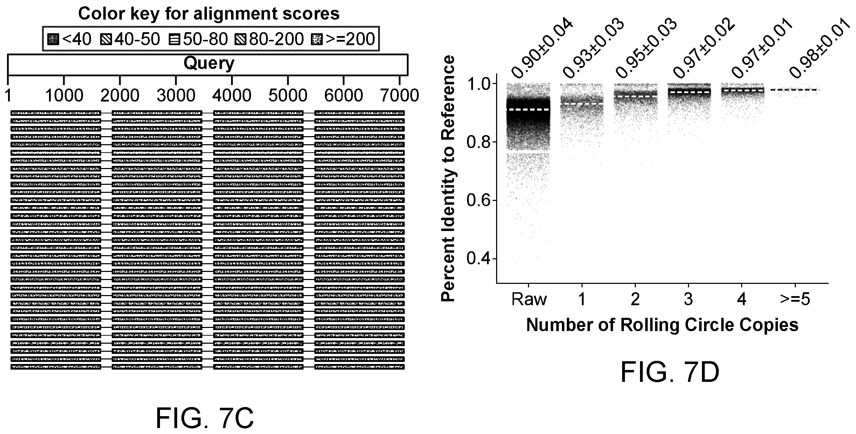

FIG. 7C shows cDNA has multiple repeat of circRNA sequence when nanopore data was aligned to the circRNA sequence.
FIG. 7D shows accuracy of the consensus reads (generated from the same cDNA with multiple repeat of circRNA sequence increases as the multi-repeat increases).
   Nanopore sequencing shown here results in improved sequencing accuracy, where no reference genome sequence is required, providing a whole picture of the circRNA in an easily executed method.

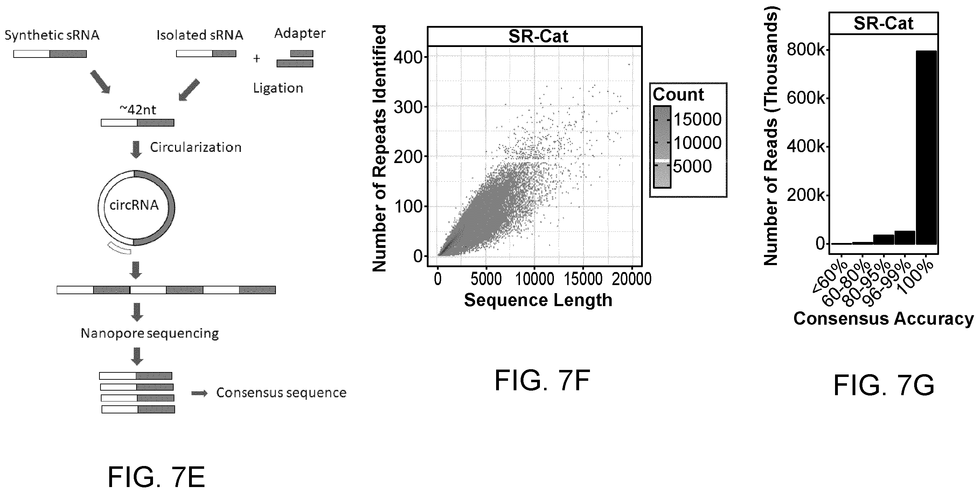

FIG. 7E shows a workflow for forming cDNA concatemers from an in vitro synthesized circRNA (42 nt) or from small RNAs isolated from human brain total RNA. For the small RNAs, after ligation of an RNA top strand adaptor sequence annealed to a bottom strand DNA splint to small RNAs, the ligated RNA was circularized. The rolling circle reverse transcription reaction happens with an oligonucleotide that hybridizes to the adaptor. The cDNA concatemers were sequenced via long-read sequencing (e.g. Nanopore sequencing) and consensus sequences generated.
FIG. 7F shows the number of repeats identified within individual concatemeric cDNA read was plotted against the read length. A strong correlation was found between read length and the number of repeats within the read. In this experiment, the majority of read lengths of cDNA from 42 nt circRNA was between 5000 and 10000 nt which corresponds to 100-200 copies/molecule.
FIG. 7G shows consensus sequences assembled from the repeats were mapped to the reference sequence (the synthetic small RNA with known sequence). The accuracy was determined by calculating the percent identity to the reference. The number of reads in each accuracy bin were plotted. The consensus reads were highly accurate with >96% of reads having at least 95% accuracy and >90% of reads with 100% accuracy.

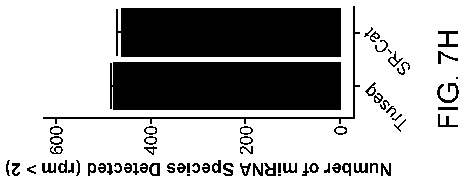

FIG. 7H shows the workflow illustrated in FIG. 7E with input of small RNAs isolated from total brain RNA (SR-Cat) can generate similar number of miRNA species as the TruSeq^{®} workflow (Illumina, San Diego, CA), which is based on the short-read sequencing platform.

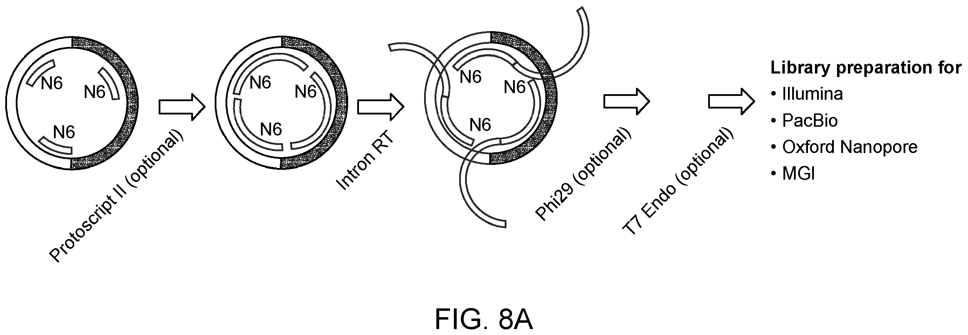

FIG. 8A-FIG. 8B shows a cartoon for a workflow in which the rate and/or yield of cDNA produced using Intron-RT for sequencing can be enhanced using additional enzymes.
FIG. 8A shows a workflow of profiling circRNA from total RNA that includes PSII that can optionally extend a short degenerate primer on circRNA to facilitate the Intron-RT rolling circle activity (RCA). Phi29 can further increase yield by amplifying the single strand cDNA. T7 Endonuclease may be used to remove branches that occurs during RCA of the RT and subsequent RCA of the DNA.

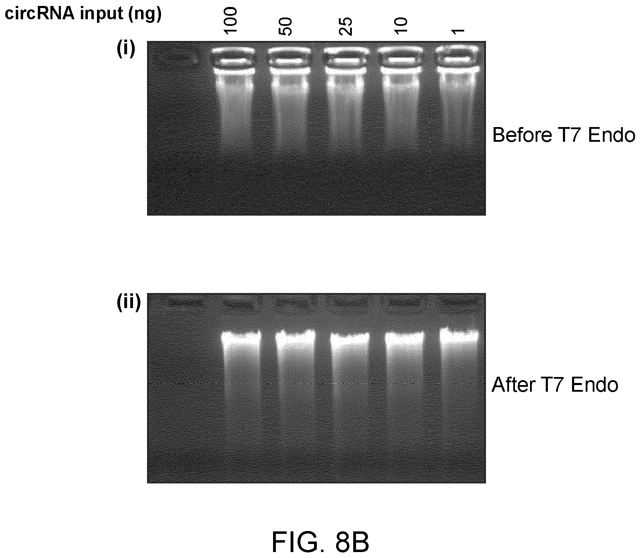

FIG. 8B (i) and (ii) shows that decreasing the amount of circRNA does not adversely affect the DNA product after amplification. As low as 1 ng of an in vitro synthesized RNA was shown here to be sufficient to produce a substantial amount of DNA for sequencing using the workflow in FIG. 8A (i) is in the absence of T7 endonuclease while (ii) includes T7 endonuclease as shown in FIG. 8A.

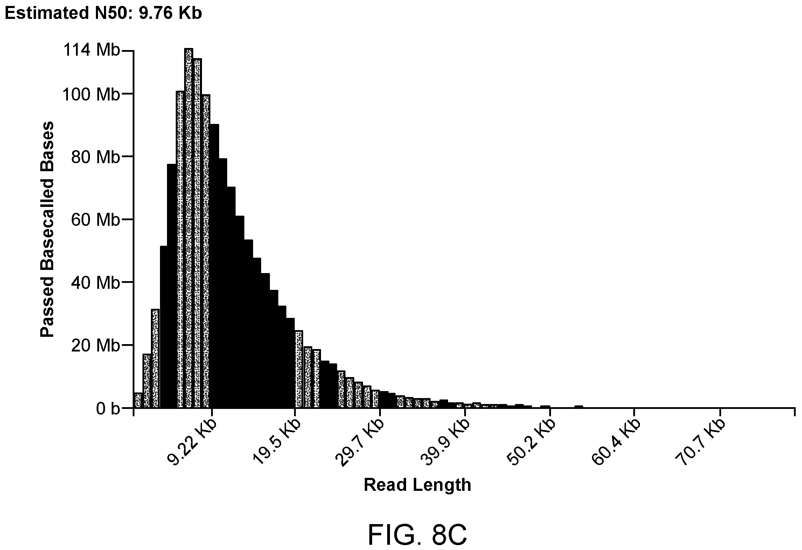

FIG. 8C shows that Intron-RT can generate concatemeric cDNA from circRNA in total human brain RNA where the cDNA has a median size of 9.75 kb.

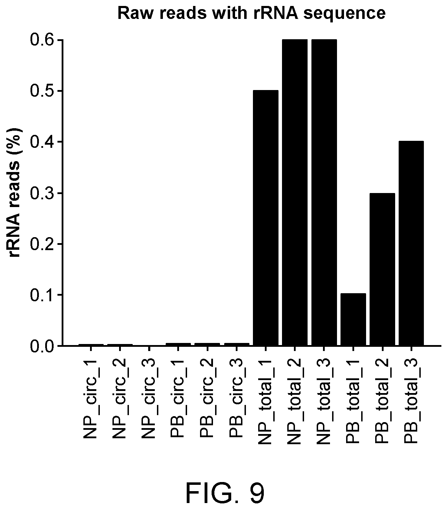

FIG. 9 compares 12 different libraries (NP: Nanopore, PB: PacBio) that were prepared from 6 different technical repeats of Intron-RT based circRNA sequencing from human brain RNA, to determine background effects of ribosomal RNA. Raw reads percentages attributable to rRNA are shown to be less than 1% in all cases. "Circ" corresponds to "circular-enriched" thus the RNA was ribo-depleted and RNaseR digested to enrich for circRNAs before entering the Intron-RT reaction. "Total" corresponds to non-depleted RNA directly used for Intron-RT reaction.

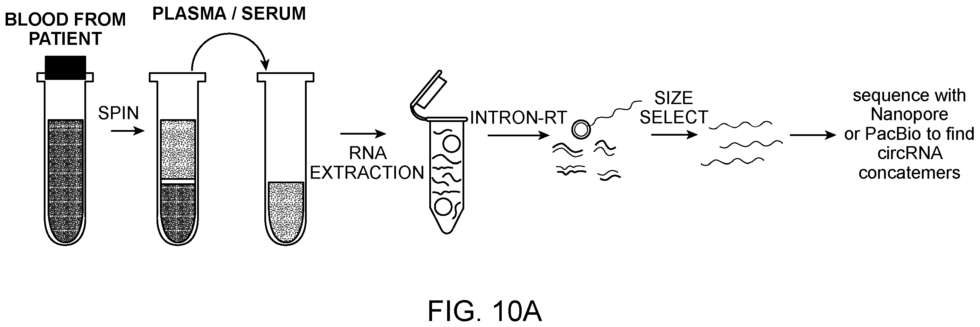

FIG. 10A-FIG. 10C shows how cell free circRNA can be detected from a sample of human plasma/serum. The circRNA sequencing was performed from human serum using total RNA based Intron-RT workflow.
FIG. 10A shows the workflow used to perform Intron-RT based circRNA sequencing with total RNA from human plasma/serum. This workflow does not require depletion of contaminating RNAs (e.g. tRNA and rRNA). Size separation is used instead. Any sample e.g. patient sample may be used as the source of circRNA. In this Figure, blood is used and is spun to prepare plasma/ serum, but any other patient sample/ bodily fluid may be used. Total RNA is extracted and incubated with Intron-RT to generate concatemeric cDNA from the circRNA, which can be identified by size and then sequenced, e.g. by nanopore or PacBio sequencing, to identify concatemers.

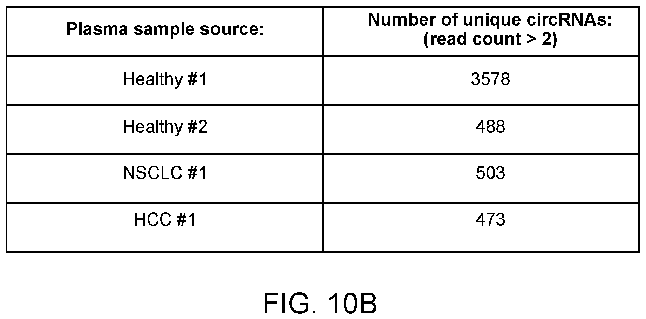

FIG. 10B shows the number of unique circRNAs in 4 plasma samples.

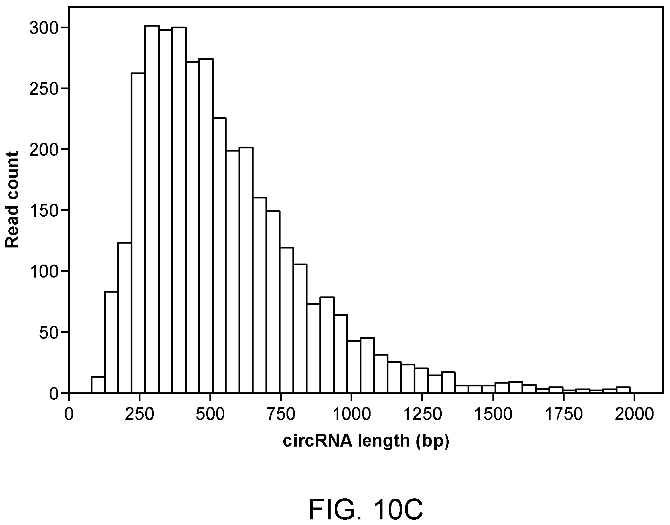

FIG. 10C shows the length distribution of different circRNAs detected from human plasma/serum. 3578 individual circRNA isoforms with more than 1 read count were detected from the human serum, with a median length of 483 bp.

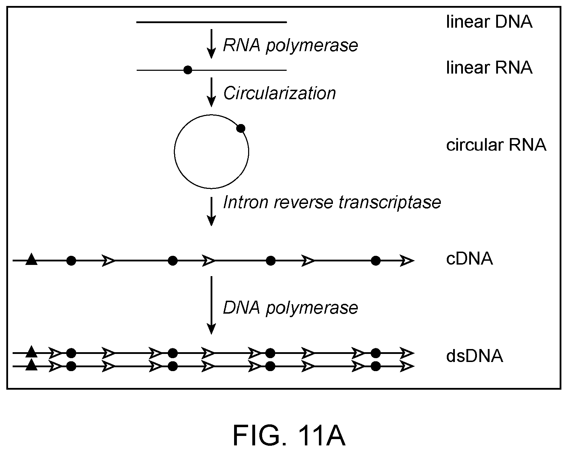

FIG. 11A- FIG. 11B show a workflow for identifying errors introduced by Intron-RT and/or DNA dependent RNA polymerases.
FIG. 11A: RNA polymerase synthesizes linear RNA from a DNA template. An example of an error introduced by RNA polymerase is shown as a circle. The linear RNA is then circularized by ligation, splicing using an oligonucleotide or by chemical means. A primer hybridized to the circRNA initiates Intron-RT rolling-circle amplification to create a cDNA strand that contains multiple concatemeric copies of the original circRNA. Intron-RT errors are shown as triangles, and each RT error only appears once per cDNA strand. RNA polymerase error (circles) are replicated during each cycle of rolling-circle amplification and appear in every monomer. Another primer can be annealed to the cDNA and extended by a DNA polymerase to produce double-stranded DNA, which is sequenced by a high-fidelity long-read sequencing platform such as Pacific Biosciences Single-Molecule Real-Time (SMRT) sequencing.

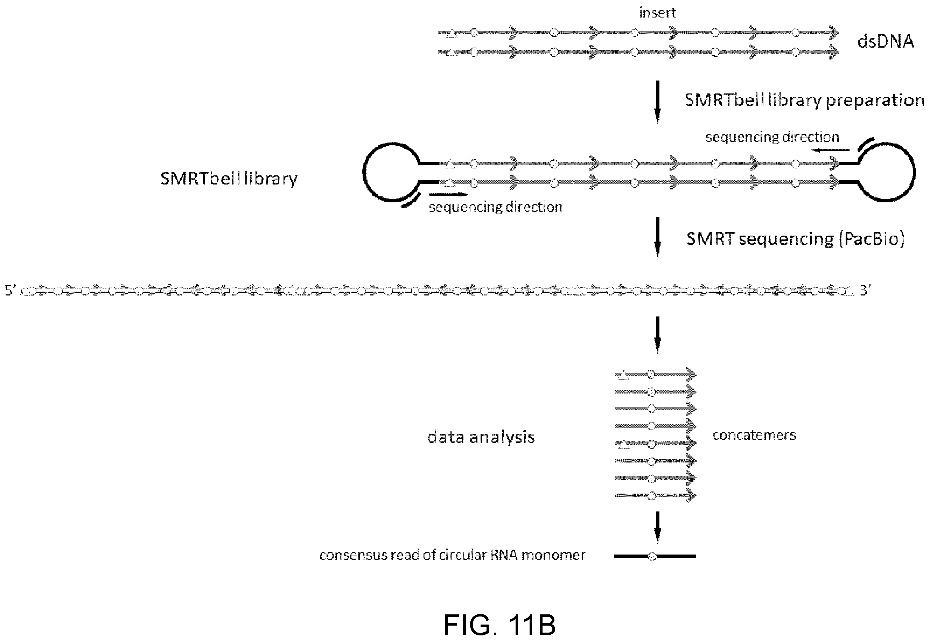

FIG. 11B describes the conceptual analytical steps to determine RNA polymerase and Intron-RT errors. RNA polymerase errors are shown as circles and are replicated across all monomers of each concatemeric cDNA, while Intron-RT errors only appear once per concatemeric cDNA strand. For each HiFi sequencing read, a highly accurate consensus sequence of each monomer is generated. The monomer consensus sequence is compared to the original RNA reference sequence to determine the errors generated by the RNA polymerase. Intron-RT errors are determined by comparing the high-fidelity Circular Consensus Sequencing (CCS) of the cDNA concatemer insert to the monomer consensus sequence of the same insert.

### DESCRIPTION OF EMBODIMENTS

Unless otherwise defined, all technical and scientific terms used herein have the same meaning as commonly understood by one of ordinary skill in the art to which this disclosure belongs. Still, certain terms are defined herein with respect to embodiments of the disclosure and for the sake of clarity and ease of reference.

Sources of commonly understood terms and symbols may include: standard treatises and texts such as Kornberg and Baker, DNA Replication, Second Edition (W.H. Freeman, New York, 1992); Lehninger, Biochemistry, Second Edition (Worth Publishers, New York, 1975); Strachan and Read, Human Molecular Genetics, Second Edition (Wiley-Liss, New York, 1999); Eckstein, editor, Oligonucleotides and Analogs: A Practical Approach (Oxford University Press, New York, 1991); Gait, editor, Oligonucleotide Synthesis: A Practical Approach (IRL Press, Oxford, 1984); Singleton, et al., Dictionary of Microbiology and Molecular biology, 2d ed., John Wiley and Sons, New York (1994), and Hale & Markham, the Harper Collins Dictionary of Biology, Harper Perennial, N.Y. (1991) and the like. As used herein and in the appended claims, the singular forms "a", "an", and "the" include plural referents unless the context clearly dictates otherwise. For example, the term "a protein" refers to one or more proteins, i.e., a single protein and multiple proteins. The claims can be drafted to exclude any optional element when exclusive terminology is used such as "solely," "only" are used in connection with the recitation of claim elements or when a negative limitation is specified.

The invention is defined by the claims. Aspects of the present disclosure can be further understood in light of the embodiments, section headings, figures, descriptions and examples, none of which should be construed as limiting the entire scope of the present disclosure in any way.

Each of the individual embodiments described and illustrated herein has discrete components and features which may be readily separated from or combined with the features of any of the other several embodiments within the scope of the claims. Any recited method can be carried out in the order of events recited or in any other order which is logically possible, within the scope of the claims.

Numeric ranges are inclusive of the numbers defining the range. All numbers should be understood to encompass the midpoint of the integer above and below the integer i.e. the number 2 encompasses 1.5-2.5. The number 2.5 encompasses 2.45-2.55 etc. When sample numerical values are provided, each alone may represent an intermediate value in a range of values and together may represent the extremes of a range unless specified.

In the context of the present disclosure, "non-naturally occurring" refers to a polynucleotide, polypeptide, carbohydrate, lipid, or composition that does not exist in nature. Such a polynucleotide, polypeptide, carbohydrate, lipid, or composition may differ from naturally occurring polynucleotides polypeptides, carbohydrates, lipids, or compositions in one or more respects. For example, a polymer (e.g., a polynucleotide, polypeptide, or carbohydrate) may differ in the kind and arrangement of the component building blocks (e.g., nucleotide sequence, amino acid sequence, or sugar molecules). A polymer may differ from a naturally occurring polymer with respect to the molecule(s) to which it is linked. For example, a "non-naturally occurring" protein may differ from naturally occurring proteins in its secondary, tertiary, or quaternary structure, by having a chemical bond (e.g., a covalent bond including a peptide bond, a phosphate bond, a disulfide bond, an ester bond, and ether bond, and others) to a polypeptide (e.g., a fusion protein), a lipid, a carbohydrate, or any other molecule. Similarly, a "non-naturally occurring" polynucleotide or nucleic acid may contain one or more other modifications (e.g., an added label or other moiety) to the 5'- end, the 3' end, and/or between the 5'- and 3'-ends (e.g., methylation) of the nucleic acid. A "non-naturally occurring" composition may differ from naturally occurring compositions in one or more of the following respects: (a) having components that are not combined in nature; (b) having components in concentrations not found in nature; (c) omitting one or components otherwise found in naturally occurring compositions; (d) having a form not found in nature, e.g., dried, freeze dried, crystalline, aqueous; and (e) having one or more additional components beyond those found in nature (e.g., buffering agents, a detergent, a dye, a solvent or a preservative).

Embodiments of the present invention permit a novel approach to detecting and sequencing circular RNA (circRNA) that include naturally circRNA, synthetic circRNA, and circularized linear RNA. The circRNA is reverse transcribed by rolling circle reverse transcription to produce cDNA containing concatemers of sequence units that are complementary to the circRNA.

A "concatemer" refers to a long continuous DNA molecule that contains multiple copies of the same DNA sequence linked in series formed by rolling circle reverse transcription of an RNA. In embodiments, concatemeric single strand DNA are described that contain at least 3 repeat units of a single sequence (e.g. at least 5, 10, 20, 50, or 100 repeat units), and the single sequence has a length in the range of 20 bases to 50 kilobases (e.g. greater than 10kb). Preferably the single sequence in each of the 3 or more repeat units differs by no more than 10%.

"Long-read sequencing" refers to sequencing that has been developed for analysis of long stretches of nucleic acids in a single read (greater than about 5000 bases or 10,000 bases). Examples of sequencing platforms suitable for long-read sequencing are commercially available from, for example, Pacific Biosciences (Menlo Park, CA) and Oxford Nanopore Technologies (Oxford, UK).

### CircRNAs

CircRNA can occur in low amounts in cells and in biological fluids. Embodiments of the methods of invention describe workflows that enable detection and identification of these RNAs via sequencing, as defined in the claims. These workflows are predicated on concatemer formation during reverse transcription using Group II Intron reverse transcriptase (Intron-RTs). Other embodiments described herein demonstrate how circRNA can be synthesized *in vitro* from linear RNAs and how target RNA molecules can be enriched by means of circularization without the requirement for depletion of non-target RNAs. CircRNA whether natural or synthetic, have been found to be more stable than linear RNAs. This property makes circRNA particular useful as a diagnostic target and also as a reagent for therapeutic and/or vaccine use.

CircRNAs have been detected in exosomes that may contain a complex cargo of contents derived from the original cell, including any or all of proteins, lipids, mRNA, miRNA and DNA. CircRNAs exert biological functions by acting as transcriptional regulators, microRNA (miRNA) sponges and protein templates. Moreover, emerging evidence has revealed that a group of circRNAs can serve as protein decoys, scaffolds and recruiters and play crucial roles in a variety of diseases, enabling them to potentially act as diagnostic biomarkers and therapeutic targets. Because circRNA are more stable than linear RNAs, there are advantages in circularizing linear RNA for sequencing, and for vaccine and therapeutic purposes. When the circRNA is combined with Intron-RTs, cDNA containing concatemers of complementary copies of the circRNA are formed. The concatemeric cDNA can be size separated from high concentrations of cDNA formed from other linear RNAs. The concatemeric cDNA may be accurately sequenced by analyzing the consensus sequences in the repeat sequences that make up the cDNA concatemers.

Linear RNAs can be circularized using splint adapters and a suitable RNA ligase , e.g. as described in FIG. 7D. Linear RNA can also be circularized by means of a ribozyme or by chemical means. Multiple RNAs can be ligated into a linear molecule and then circularized. Enzymatic ligation can be achieved by means of T4 RNA ligase *2. In vivo* or *in vitro* methods of circularization are described for example, in Muller, et al. RNA Biol. (2017) vol 14, 1018. Degenerate sequences that serve as unique identifiers may be inserted into a circularized molecule by means of an adapter or a primer.

### Intron-RTs have conserved structures and are suitable for rolling circle reverse transcription of circRNAs

Intron-RT refers to a family of reverse transcriptases that are functionally capable of performing efficient rolling circle reverse transcription of circRNAs to create concatemeric cDNAs. Examples of Intron RTs are provided below in SEQ ID. Nos 1-49 any of which may be used for rolling circle reverse transcription in the compositions, kits and methods described herein. It should be appreciated that other members of the Intron RT group for use herein may be obtained by metagenome analysis using functional, structural or sequence dependent features. Intron-RT as used herein also refers to variants of wild type enzymes where the variants may include naturally occurring and/or artificially introduced insertions, truncations, deletions and/or nucleotide mutations. In some embodiments Intron-RTs may be used with representative sequences from a sequence database such as disclosed below in SEQ ID NOs: 1-49 or comprising a sequence having at least 80%, 85%, 90% or 95% sequence identity to any of SEQ ID NOs: 1-49.

Intron-RTs from wild type sources are encoded by mobile Group II Introns that function in Intron mobility ("retrohoming") by a process that requires reverse transcription of a highly structured Intron RNA with high processivity and fidelity. Intron-RTs have been shown here to have higher fidelity, processivity, and strand displacement activity than retroviral RTs. To facilitate Intron-RT purification, Intron-RTs have been expressed as fusion proteins with a rigidly linked, non-cleavable solubility tag (see for example, Mohr et al. RNA (2013) vol 7, 958-70). Group II intron reverse transcriptases that are commercially available for reverse transcribing linear RNA include TGIRT^{™} (Ingex, Olivette, MO) and Induro (New England Biolabs), and MarathonRT (Kerafast, Boston, MA).

Intron-RTs have certain characteristic structural features including an N-terminal extension (NTE) typically containing a conserved sequence block (RT0) and two structurally-conserved insertions (RT2a and 3a) between the universally-conserved RT sequence blocks (RT1-7) (Lentzsch, et al. JBC (2019) vol 294, P19764-19784). The RT1-7 corresponds to the fingers and palm of retroviral RTs; thumb, with predicted α-helices corresponding to those in the HIV-1 RT thumb; DNA-binding domain and DNA endonuclease domain.

Present embodiments rely on the use of Intron-RT (for example, see SEQ ID NO: 1-49 or variants or mutants thereof) and are characterized by the conserved structures described above, even where the sequence varies. These reverse transcriptases were shown here to be especially suited for rolling circle reverse transcription of circRNA of any desired size including small and large RNAs and to consistently produce multiple copies of the circRNA in the form of linear concatemeric cDNA where each unit of the cDNA concatemer represents one copy of the circRNA. These reverse transcriptases are also suitable for reverse transcribing linear RNA without concatemer formation. Nonetheless, the enzymes have advantages over retroviral reverse transcriptases for linear RNA because of their processivity (length of RNA substrates transcribed), fidelity (low error rate), and ability to bypass modified nucleotides as well as to tolerate contaminants in the reaction mix. However, until now, the use of these reverse transcriptases have been restricted to linear RNAs. Reverse transcription of circRNA using this class of reverse transcriptases has provided exciting new technologies described herein where some features include the following:
(a) Intron-RTs can strand displace DNA that becomes hybridized to RNA during rolling circle reverse transcription enabling the Intron-RT to make concatemers of cDNA containing multiple complementary copies of the circRNA. The processivity of the Intron-RT results in long cDNAs that contain multiple cDNA copies of the circRNA making possible size separation of the concatemeric cDNA from other cDNAs copied from linear RNA that might arise in a biological sample. This advantageous feature is absent in retroviral reverse transcriptases. Since these latter reverse transcriptases cannot strand displace, they are unable to generate long cDNAs.
(b) Intron-RTs can bypass base modifications in the circRNA such as psU, m6A, 5hmC, 2'-O-mU and m1A to achieve rolling circle reverse transcription to produce cDNA concatemers. This is a useful feature that enables for example, quality control of circular mRNA that contain modified bases in vaccine production and also for discovery and/or analysis of natural occurring RNAs that contain modified bases such as some viral or archaeal RNAs.
(c) Intron-RTs are tolerant to inhibitors that may be present in the preparation of circRNA. Examples of inhibitors that do not prevent rolling circle reverse transcription with Intron-RT include various salts, 2% paraffin and 2% Tween 20. Other inhibitors that some Intron-RTs show tolerance for, include 0.02% Formulin, 10% ethanol, 10% isopropanol, 10% DMSO, 100mM Guanidinium chloride, 2% Igepal and 1.25M Urea. Intron-RT can form concatemeric cDNA in the presence of inhibitors such as might be found in body fluids or cell samples. This feature makes possible for the first time the detection of rare circRNAs in for example, blood that would otherwise be undetectable against the substantial background of contaminating non-target linear RNAs and other blood components.
(d) Intron-RTs can rapidly form concatemers in a wide range of circRNA sizes within 60 minutes or less at temperatures in the range of 25°C-65°C. In some embodiments, the Intron-RT may be able to synthesize cDNA from circRNA or linear RNA at 2 kb/minute at temperatures of 50°C-60°C. In some embodiments, a 12 kb cDNA may be synthesized in 10 minutes at a temperature in the range of 50°C-60°C.
(e) Intron-RTs can be readily stored either in a lyophilized form at room temperature, or in a storage buffer at -20°C where the storage buffer optionally contains a reducing agent such as DTT and optionally a detergent although preferably the buffer contains neither additives.

### Yield of cDNAs obtained from circRNAs.

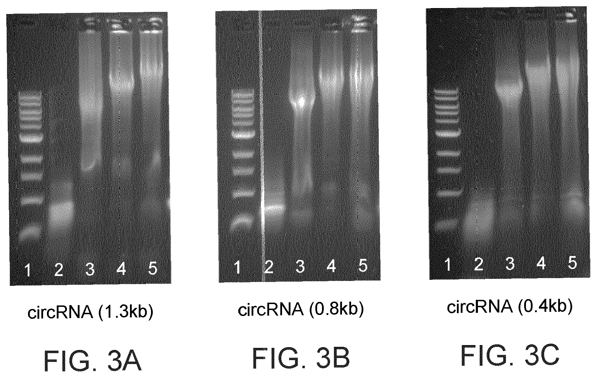

A comparison of the amount of cDNA produced by reverse transcription using commercially available retroviral reverse transcriptases and Intron-RTs show that the yield from Intron-RTs is much higher than from the retrovirus RTs. A variety of reverse transcriptases have been obtained from different sources including modifications of these. The advantages of Intron-RTs compared to viral RTs (e.g. M-MLV RT, PSII, and SuperScript IV (SSIV) (ThermoFisher Scientific, Waltham, MA) is illustrated in FIG. 2A-FIG. 2B and FIG. 5A-FIG. 5D with respect to yield and in FIG. 3A-FIG. 3C with respect to size of the circRNA. FIG. 2A-2B and FIG. 5A-FIG. 5D show a greater than 2 fold, 3 fold, 4 fold, 5 fold, 6 fold, 7 fold and as much as 8 fold increase in cDNA could be obtained using Intron-RT compared with viral RTs using the same starting material and incubation times as well as equivalent concentrations of the reverse transcriptase.

Similar difference in yield could be observed with whole RNA at a concentration of 0.0001 pg- 1 ng of a 0.5 kb circRNA from a pure preparation of circRNA or from total brain RNA (see for example, FIG. 5A-FIG. 5D). Again, Intron-RT significantly enhanced the sensitivity of the detection than viral RT (PSII).

In embodiments of the method, sequencing revealed that more than 70%, more specifically greater than 80% of the cDNA were concatemers yielding consensus sequences for unique circRNAs.

### Concatemer length of cDNA from circRNA

A population of concatemeric first strand cDNA molecules may be obtained from circRNA by rolling circle reverse transcription where the median length of the molecules is at least 0.5 kb. In some embodiments, the median length is in the range of 1.5 kb-50 kb, where for example, some molecules in the population may be in excess of 10kb or 20 kb in length. In various embodiments, at least 90% of the molecules may have a length that is at least 1.5 kb, at least 2 kb, or at least 3kb. The number of concatemeric first strand cDNA molecules in the population may vary. However, in some embodiments, the population may comprise at least 100, at least 1,000, at least 5,000, or at least 10,000 of the concatemeric first strand cDNA molecules and each molecule may have, on average, at least 3 units, e.g., at least 4, or at least 5 units, as described herein. In some cases, the sequences of the concatemeric cDNA may vary in a sample, reflecting the diversity of sequences of circRNAs in the sample. Within a molecule, each unit corresponding to one copy of the circRNA may be at least 25 nt, at least 50 nt, at least 200 nt, at least 500 nt, or at least 1,000 nt. In these embodiments, the units may range in size in the population, reflecting the lengths of the circRNAs in the sample.

### Reaction mix containing oligonucleotides for Rolling circle reverse transcription of circRNAs using Intron-RT

The ability to form cDNA concatemers from circRNA was here observed for each of a plurality of Intron-RTs tested. In embodiments, an oligonucleotide is utilized to initiate reverse transcription.

In some embodiments, a reaction mix for producing concatemeric first strand cDNA is provided, as defined in the claims. In these embodiments, the reaction mix comprises: a circRNA (or a sample comprising circRNA) wherein the circRNA is selected from: a eukaryotic circRNA, and a synthetic circRNA having an artificial sequence (e.g., one or more RNAs that that have been circularized in vitro, or a sample comprising RNA isolated from a cell or bodily fluid from a eukaryote, e.g., a protozoan, an invertebrate, a plant, or a mammal, where the sample comprises circRNAs); and a Group II bacterial or archaeal Intron-RT; where the combination is non-natural. The reaction mix may also comprise dNTPs.

In addition the reaction mix may comprise an oligonucleotide. The oligonucleotide in the reaction mix may be an adapter, ribozyme or other DNA molecule suitable for enhancing rolling circle reverse transcription of circRNAs. Alternatively, or in addition, the reaction mix may comprise an oligonucleotide that is a primer. Examples of primers for use with Intron-RT include a primer where the 3' end (e.g. at least 8nt in length) hybridizes to the circRNA and the 5' end of the primer is either complementary to the circRNA and does not have a 5' tail (e.g. a target-specific primer may be relatively short, e.g., less than 20, less than 15 or less than 10 nt in length) or it does have a 5' tail that is not complementary. Where the primer has a tail, the tail may be at least 8 nt at the 5' end , or the tail may be short, e.g., 6 or less, 5 or less or 4 or less nt. 5' tails may contain a sequence that provides a binding site for a PCR primer.

Another example of a primer is one that has a degenerate sequence at the 3' end (e.g., a sequence of at least 4, at least 5 or at least 6 degenerate bases). These primers may be relatively short, e.g., less than 10 nt in length although longer degenerate or sequence specific primers may be used. The oligonucleotide may be a primer containing a degenerate sequence that hybridize at its 3' end to the circRNA to initiate reverse transcription and has a 5' tail suitable for down-stream priming of DNA amplification. Where degenerate primers are used for amplification, embodiments of the method provide for an endonuclease such as T7 endonuclease to remove branched DNA molecules that can result from primer hybridization to cDNA concatemers as shown in FIG. 7B. Where specific primers are used to initiate reverse transcription, branching does not generally occur.

The oligonucleotide primer may have a complementary sequence to the circRNA and further include a unique identifier sequence commonly comprising a degenerate base sequence. In some embodiments, the short length of the primer can impose an upper limit on the temperature of the reverse transcription reaction. This effect may be circumvented by extension of the primer. In some embodiments, a retroviral reverse transcriptase such as ProtoScript II may be used to extend a short complementary primer (such as a degenerate primer) hybridized to the circRNA for enhancing Intron-RT reverse transcription. In some embodiments, multiple primers (e.g. hexamers) hybridize to the circRNA at different locations. In any embodiment, the reaction mix does not need to comprise a template switching oligonucleotide. Template switching oligonucleotides typically have a stretch of riboguanosines (e.g., rGrGrG) at the 3' end.

### Amplification post-reverse transcription

The cDNA concatemers that result from Intron-RT may be amplified in entirety prior to sequencing. The preferred amplification is whole genome amplification using for example Phi29 or any other DNA polymerase that is commonly used for long range isothermal amplification (see for example, Examples 6, 9 and 10) as the average size of the concatemers may be in excess of 1.5 kb, 3 kb and as much as 9 kb or more. If qPCR is performed on the cDNA or amplicons thereof, only a portion of the concatemer is amplified to quantify circRNAs.

### Advantages of rolling circle reverse transcription with Intron-RT over reverse transcription of linear RNAs

Although there have been some reports of rolling circle reverse transcription of RNA using retroviral RT, these enzymes were found to be inefficient with respect to yield of cDNA when compared with Intron-RT. SomaGenics (US 9,493,818) reported the use of adapter ligation to artificially circularize very small linear RNAs (miRNAs) for PCR amplification that relied on the adapter to provide specific primer hybridizing site. The reported method utilized a retroviral RT called SuperScript^{™} II (ThermoFisher Scientific, Waltham, MA) to reverse transcribes circRNA with low efficiency to produce a linear cDNA. While not wishing to be bound by theory, it is believed that the observed inefficiencies of retroviral RTs arise from the difficulties in passing through primer bound to the RNA substrate owing to low affinity binding of the enzyme for the RNA substrate and limited if any strand displacement properties.

An advantage of Intron-RT is its flexibility in accomplishing rolling circle reverse transcription of circRNA having a wide range of sizes e.g. 20 bases to 50 kb. For example, a long cDNA (median size >5 kb) was generated from a 42 nucleotide circRNA thereby containing more than 100 units of sequence complementary to a unique circRNA within 15 minutes (see FIG. 7E-FIG. 7G). The multiple units in a single cDNA made possible synthesis of double stranded DNA for sequencing with long read sequencers. This in turn enabled different unique circRNAs to be identified in complex mixtures of RNA. Examples 3,4, 6-8, show results for total RNA from brain tissue while Example 9 shows analysis of different unique circRNAs from serum.

Advantages of concatemer formation in cDNAs from circRNAs by Intron-RTs over single copy cDNAs include:
(a) Small amounts of circRNA can be detected, amplified and/or sequenced because of the amplification of the cDNA copy number in concatemeric cDNA molecules.
(b) Greater sensitivity of detection using Intron-RT with circRNA in total brain RNA with RT-qPCR. In general, a sensitivity of greater than 2 fold is expected using an Intron-RT compared with a retroviral RT over a range of concentrations of circRNA as low as 1 pg or less. For example, FIG. 5B shows as much as 16 fold increase in sensitivity using Intron-RT versus PSII. FIG. 5D shows as much as 23 fold increase in sensitivity compared to PSII (see FIG. 5D) where the concentrations of circRNA in FIG. 5B were 0.01pg, 0.1pg, 1pg , 10pg, 100pg, or 1ng.
(c) Highly accurate sequence data (greater than 80% for example at least 90% sequence accuracy) can be obtained for the circRNA by means of aligning and comparing the sequence of individual units from any single or multiple consensus sequence(s) of cDNA.
(d) The formation of long concatemeric cDNA (e.g. greater than 500 nt) from circRNA enables size separation of the concatemers from cDNA products from small linear RNA (such as tRNA or rRNA) and other linear RNA (such as mRNA), thereby permitting enrichment and/or purification of the circRNA from samples without the need for rRNA depletion. The removal of linear RNA using a mixture of RNases as described herein further enhances the sensitivity of detecting rare species of circRNA from biological material.
(e) Whole concatemeric cDNA sequencing can be achieved using long-read sequencing platforms such as Pacific Biosystems or Oxford Nanopore where a single cDNA may contain hundreds of complementary copies of the circRNA that maybe each as short as 20 nucleotides as in circularized microRNAs. The availability of multiple complementary copies of RNA in concatemeric cDNA may also enhance RT-QPCR sensitivity particularly for biological samples.
(f) Intron-RTs are useful reagents in determining the error rate of RNA dependent RNA polymerases and DNA dependent RNA polymerases that may be used routinely, or newly discovered by metagenome analysis or existing sequence databases.

### Obtaining accurate sequence data by consensus sequencing

Uses of concatemeric cDNA containing at least 4 copies of the substrate circRNA provides advantages that include: (a) improving the accuracy of sequencing data; (b) distinguishing circRNA from linear RNA; and (c) detection of error artefacts in synthetic circRNA.
(a) Sequence accuracy. The long concatemeric single strand cDNA produced by Intron-RT could be directly sequenced by long read sequencing platforms such as Oxford Nanopore or Pacific Biosciences sequencers that did not require an intermediate amplification step. Amplification of circRNA species by Intron-RT to produce cDNA concatemers is particularly useful for detecting rare circRNAs species. Subsequent whole concatemer sequencing that provides a consensus sequence for the multiple repeats provides a sensitive and accurate analysis of the circRNA in a sample. Biological samples including circRNAs largely contain linear RNAs some of which are fragmented naturally or can be fragmented in vitro using RNAses. Naturally occurring circRNAs have been correlated with various pathologies such as cancer and Alzheimer's disease and as such are useful biomarkers. Detecting these rare molecules and monitoring their up regulation and/down regulation represents an important diagnostic tool. Until the present embodiments, there appeared to need a more sensitive and accurate method of detecting and analyzing these naturally occurring rare circRNA molecules.
(b) Distinguishing circRNA from linear RNA. After the rolling circle reverse transcription, only the circRNA can generate concatemeric cDNA. The linear RNA can only generate single copy cDNA. Hence, from the sequencing result, the concatemer property of the cDNA confirms the nature of circle of the circRNA.
(c) Error artefacts in synthetic circRNAs. Synthetic circRNAs have been the focus of increased interest in the development of RNA therapeutics and vaccines because these RNAs are stable without the need for capping required to stabilize linear RNAs in this context. However, *in vitro* circularization of linear RNAs has associated problems such as introduction of erroneous fragments of RNA or mutations or truncations at the RNA termini prior to circularization. The sensitivity of concatemer formation followed by sequencing permits review of circularized RNA populations to determine their quality regarding the extent of artefacts.

The ability to form concatemers of cDNA from the circRNA has an added advantage for enhancing accuracy of RNA sequence determination. The consensus sequence from aligned cDNA sequence units obtained from the concatemers can reveal nucleotide variations that result from experimental error in the consensus sequence in contrast to actual variants.

### Some Uses of rolling circle reverse transcription

The convenience of forming concatemers of large circRNAs may be provide a means of quality control for mRNA that is synthesized and circularized for vaccine production or as therapeutic molecules for modulating a phenotype of an organism. The convenience of forming or identifying small circRNAs in total RNA or RNA in blood, plasma, or other biological fluid is the ability to recognize rare molecules that occur at low concentrations without the cost of further loss of sample through depletion reactions to reduce background. Moreover, the concatemer formation in cDNA is an amplifying effect that allows for further second strand DNA amplification and considerable enhancement of signal in diagnostic tests and sequencing experiments. Uses also include testing novel DNA dependent RNA polymerases for error rates where the reverse transcriptase error rate can be reliably subtracted. This is also shown with triangles and circles in FIG. 11A and FIG. 11B.

More specifically, an embodiment of the methods include the following: (i) incubating a reaction mix comprising RNA (e.g. a total RNA sample isolated from a cell or bodily fluid, where the sample contains circRNAs), an Intron-RT, dNTPs, and a primer that has a degenerate 3' end and may not have a 5' tail, to produce a product comprising concatemeric first strand cDNA molecules; (ii) amplifying the product first strand cDNA molecules using a randomly-primed amplification method (e.g., using Phi29 polymerase and random primers, or any suitable randomly primed WGA method), to produce an amplification product; and/or sequencing the amplification product, e.g., using a long range sequencing platform such as Oxford Nanopore sequencer. This method has the potential to revolutionize how circRNAs are analyzed, because the method can be PCR-free. In addition, the method eliminates template switching, which is an inefficient process in this context. Finally, the method eliminates the need for selection of molecules of a defined size range (i.e., molecules that are about 1 kb in length; see Zhang, et al Nat. Biotech. 202139: 836-845).

### Intron-RT Kits

A reagent kit for use in analyzing circRNAs may include an Intron-RT preparation as defined in the claims and having the following features:
The Intron-RT preparation containing an Intron-RT may be stored in a suitable storage buffer or in a lyophilized form, in a storage container such as a tube or on a matrix such as a paper, beads or a plastic substrate.

The Intron-RT may be a fusion protein with a second protein domain such as maltose binding domain (MBP), chitin binding domain (CBD), SNAP-tag^{®} (New England Biolabs, Ipswich, MA) or other suitable protein binding domain for immobilizing the Intron-RT on the substrate. The Intron-RT may be fused to a second RNA binding domain for enhancing its binding to RNA.

The Intron-RT preparation includes reagents for circularizing linear RNA such as any of a ribozyme, an RNA ligase, a splint adapter, or chemicals capable of circularizing the RNA; and may further include one or more of an oligonucleotide for circularization of an RNA, and/or a primer.

The Intron-RT preparation will be preferably RNA-free prior to use.

The Intron-RT preparation may further include additional proteins such as a 3'-5' exonuclease such as RNase R. The Intron-RT preparation may also include a 5'-3' exonuclease such as XRN-I.

The Intron-RT preparation may further include a reversible binding aptamer for inhibiting the reverse transcriptase activity prior to the desired reaction time.

The Intron-RT may additionally include one or more of: DNA dependent RNA polymerase, a DNA polymerase such as Phi29 or other polymerase, an exonuclease, an endonuclease such as T7 endonuclease, a ligase and/or random or specific primers and dNTPs. Any of these reagents may be present in the Intron-RT preparation or may be provided in a separate container either individually or together.

The Intron-RT preparation or kit may further include a T4 RNA ligase, a ribozyme, an adapter and/or suitable chemicals for circularizing a target RNA. The Intron-RT preparation may further include in a separate container, a Thermolabile Proteinase K (New England Biolabs, Ipswich, MA) for removing enzymes at a particular time in the workflow and/or a DNAse for removing contaminating DNA from a sample.

### Examples of Intron-RTs

SEQ ID NO: 1 
MMNDGNDWIVDIDLEKFFDTVNHDKLMTIIGRTIKDGDVISIIRKYLVSGIMIDDEYEDSIVGTPQGGNLSPLLANIMLNELDKEMEK
RGLNFVRYADDCIIMVGSEMSANRVMRNISRFIEEKLGLKVNMTKSKVDRPSGLKYLGFGFYFDTRAHQFKAKPHAKSVAKFKKRM
KELTCRSWGVSNSYKVEKLNQLIRGWINYFKIGNMKRLCKELDSRIRYRLRMCIWKHWKTPQNRAKKLMKLDVPRWAAFKIAYCG
DKYASLAHNGWVHKAISLKRLTSFGLVSMLDYYTERCVKC
SEQ ID NO: 2 
MIRNGKYKPNPVRRVEIPKETKGEFRKIGVPTVVDRVIQQAITQELTPIYEEQFSENSFGFRPKRGAHGALKQCQKNVNDGYVYVVD
MDLEKFFDTVSQSKLIEVLSRTIKDGRLISLIHKYLNAGVIANGMFERTEIGIPQGGPLSPLLSNVMLNELDKELERRGHRFVRYADDC
MIFCKSKKSAERTLENILPFIEGKLFLKVNTMKTKVAHISKVKYLGYSFYRYKGKCRFRVHPKSVSKMKDKIRELTDRNNGMSNVIREE
RYQQYVRGWVEYFRLADMKSLLKTTDEWARRRIRAVYWKQWKKIKTRYRMLKALGMEHWKALELAWSRKGYWRMAQVLNQV
FSNKIIAKLGYTSLLDYYLIVYEN
SEQ ID NO: 3 
MRTGAIDELQELRRKIYLTAKSEKRKCFWGLFCHVTKIETLDKAYKLARENNGSAGVDGVTFADIERYGTYNFIKEIREELINGTYLPTR
NRKVEIPKSNGKTRTLGIGTIKDRVVQGALKLILEPIFEADFSECSYGFRPKKNQHQAVNRVAKGIVRGFTKVIDVDLTAYFDNINHKIL
MDKIALRVNDSKIMRLIKLMLKINGKVGVPQGSVISPLFSNIYLNAIDRMFEKAKQGTSRKGYENMEYCRYADDTVILVNGHEAVD
WLVSKSYRRLREELAKLKINLNVEKTKIVDMSQGETFGFLGFDFRLVKSKGKKMVLLKPKKKKVTELISKVKTHLVQSRNKTVTEMIEE
LNPILRGWVNYYRIGHCSRTLTYIKIWVERKVRRFARKAQKKFGFGWKEWSSEIIYGKWGLFSDYQVRYFSS
SEQ ID NO: 4 
MIKTSIELQELRRRIYIKAKAEKDWKFWGLYIHVTKMETLAEAYKLAKGNNGAPGIDGVTFEDIENEGAGQFLKKIQEELTSETYLPMK
NRIKDIPKGNGKTRTLGIPTIRDRVVQGALKLILEPIFEADFQDGSYGYRPKRTAHQAVERTAKAVIQGKTQVIDVDLSSYFDNVQHHI
LLGKVAKRINDRKVMRLLKLILKANGKIGLPQGGLISPLLSNLYLNEIDKMLEKAKEFTKKKDGYIHIEYVRYADDLVILIDGYHKWNRL
MEFTMFRLKQEFEKIGVKINTEKTKIVNLLHGEIFSFLGFDYRRTKTKSGKWGVRKTPKMKARTKLLEKIKGIFKSQKSQPLDKVIKLINP
ILRGWVNYFRIGNSSQCFGYVKDWIQKKIRRHLMRARKLKGFGWDRWSNKWLYEKLNLYGDYKIRYFQG
SEQ ID NO: 5 
MNVKTNDILGTVRYEPLEKAQELCEKLYLAAKNSETRRFHAIYDKVYRMDILNSAWNQVKANKGTKGIDNESISDILMKGEDIVISEIH
QNLKERKYRPKQVERVYIPKPDGRMRPLGIPTIRDRIVQTATKHLIEPIFEADFIDCSYGFRPNRCAHEALEEIRKTMNAGYTIVLDADI
KGFFDNIDHDKLLEFVHRRISDRRILKLIRKWLKCGIMDSGIVKESDMGTPQGGVISPLLANIYLHEFDKFWTQQTRVKGKLVRYADD
FVIMFRTEKEAELGLKLIKAKMEELGLELNEDKTKIVDTRGGKEGFDFLGFHHRMTKSWKYKRYYAQKRPGDKSVKSIKTKIRNFLGQ
RAILNRSIDEVVKEINPVIRGWMNYFRFGNSADKFTQIDRYLHERLALWWSKKHQKSGRRWKSDFTWEKFKECGIQLLSGNVLYWS
YYSNA
SEQ ID NO: 6 
MKEITERRYYTLKDKITKIRNMKVAAKHVLDKGGSAGVDRIDTVEFKENYSIHMRELYREFLEDRYQPKPALRVFIPKSDGRQRPLGIP
TVKDRIAQAAVRGILEPIYEKEFCDCSFGFRKGKSQMDAINQIEEYKEQGYKWVLDADIKGFFDNIDHKLLIEFIKQKVTDGWVIEIIKL
WLTMGVMKDGKYIPTEKGTPQGGVISPLLANIFLHEFDKVMTERGYKLVRYADDFVVMTKSKRKAKRAYEVVKEIITEKLKLELHPEK
TVITNFGEGFVFLGFEFIAWRYKRPRKKSLEKFKDKVRKVTKRNQPWKVDSIIKRLNAKIYGWANYFGHGNVKTLFRSLDEWIRMRL
RSYIEKKKAVMNQNKRIPNSFFRKKGLVSLLTRLS
SEQ ID NO: 7 
MKTDNTLLEEMLSDTNLELAFTQVKRNKGASGVDGMEVAELKDYLDKHLEEIKDSIRNKTYKPQPVRRVEIPKPDGGIRNLGVPTVL
DRFVQQAIAQVLIPIYEPIFSDNSFGFRPNRCCEMAIIKALEYMNEGYQWIVDIDLEKFFDTVNHDKLISLVMKDVKSGEIVSLIRKYLV
SGIMIDNEYKESVVGTPQGGNLSPLLSNIVLNELDKEMEARGLRFTRYADDCIILVGSSKAADRVMENISKFIEKKLGLKVNMTKSKVS
KPNDIKYLGFGFYYDSFSSMWKAKPHEKSIATLQTKLRRLTNRSWSVSWEYRILKIRQLVNGWINYYRIGNFIKVCRKLDAQIRFRIRM
YLWKKWKTIGNREKQLRKLGALPWQAKTWANSRKSYARCASTFLRTRITNDLLYRKGLPSMVAQYQLKHISV
SEQ ID NO: 8 
MIISEMQHKLATWAASDPTRQFTRLLRLIVNRDWLAEAARIVLASNGARTPGIDGIDKHKMQRGLDDKLDQLQNDLLQGTYCPQP
VKRIYIKKANGKLRPLGIPTLTDRIVQRAMLMVMDPIWESDFHRCSYGFRPERSVHHAVRTVKIQLQDGSDTKGRWVIEGDLASYFD
 
TVHHKLLLKCVRRRIADNRFVDLLWRILKAGHVDKGLFRASSEGVPQGGVLSPLLSNIMLHEFDMWMEGNYLNKKVRKDRWAWN
FGIQQGRPITIRENREWKPAVAFSRYADDFVVIVKGNKAHAEAIREQCRMFLEGKLKLTLNMEKTHITHVNDGFVFLGHRIIRKRGKT
GRMSVVTTIPYEKAKAFAHKLSKALSGDHDLSKVDMVDRLNRKLSGWAAFYKFTDNTARTFQRIDRVVFWKLAHWLAQKYRSRIKP
LMRNWCRAPEKGKAKTWVLFGISEQGNRVGKALHRLVGSPKMQFRWRNPETNPYIKREENRSTITSRYRDVAMAMGRA
SEQ ID NO: 9 
MRPLGISSSDDKLVEEVIKMILESIYDPTFSNYSHGFRPGRSCHTALLQLQQNFTGVKWFVEGDIKSYFDTIDHHSLVNILRRRIKDEAF
IELIWKFLNAGYMENWEYNATFSGIAQGSGISPILANIYLNEFDRFMEDYKKGFDKGMGRKRNNEYSRKQSYRQRCKAKIRKEWDG
YSDTEKQEAVRQLAELKRAFQKIPMGDPMDTGYKRIQYVRYADDFLIGVIGSKEDAEKIKSDIGRFFEETLKLELSAEKTLITNSDDKAR
FLGYDVTVCNDRALKKAKGKGTVKAYTGKIKLYLPKEKWVGKLLGYGVLKIVSRAGEKEVWKPLQRNDYIFLPVHEMVRKYNAQIRG
IYNYYRLASNVSVLNKFHYVMEYSLYKTVAAKYRITMTKAKLKYTKNKEFKVPYKTQKGIKYAVLYNEGFRRVKYALGSYADIIPEYEQ
MNKPKELFFRYKANVCEMCGAYVPAVKVYQVKSMNDLDVNTEWGAIMNRKKRKTLVVCGDCYDRIHK
SEQ ID NO: 10 
MNVTNDRFKDRQLHIEDYRQMVSAEQKEYAEVFAYQRITENNDIIIDFWTENLMELILRKDNFNKAYKKVKSNKGAGGIDGMQVD
NLLPYLRENQDELVQQIKLGKYKPNPVRRVEIPKEEKGKVRMLGIPTVVDRVIQQAMAQELTPIYEEQFSDSSFGFRPNRGQHDALK
RCQKYVEEGYVYVVSMDLEKFFDTVNHSKLIEVLSRTIKDGRVIALIHKYLYAGVVRSGLFERTGEGFPQGGPLSPLLGNIMLNELDKE
LEQRGHKFVRYADDVVIFCKSKKSAERTLDNIVPYVEGKLFLKVNRAKTTISHVSKIKYLGYAFYRYKGKCRFRVHPKSVMKMKDKLRV
LTNKSTGWDNDYRRQKLTEFVRGWINYFSMADMRGLMGEVDEWLRRRIRAIYWKQWKKVKTRYRILKALKLPEWKVHELANCRK
GYWRATIMLNSVLTNKIIARLGYISMSDYYLKICEN
SEQ ID NO: 11 
METNTEEGLTPKLTQLRAKLNFKAKQEPNFRFYTLYGHICNMEVLETAWKQVLKNRGTCGYDGVTLEDVKLRGVNEFLEEIRKSLVF
RTYRADPVKRVYIPKSDGSLRPLGIPTVKDRVVQMALLLIIEPIFEADFLDCSYGFRPDKSTHQAIDAMVQAINEGKHEIYDADLEKYFN
SIPHDKLIKAVEKRIVDNRVLNLIKMWLKAPIWEKGKPMDRNNVGVQQGGVISPILSNLYLHWFDKIFCSSKGPREWANAVLIRYAD
DIVIMAKFIGPKIKEWIQKVIEERFCLKINPAKTKIVKLKDNKAFVNFIGFTIRILRSGKVQIQPKKEAIKKAKLRIRELTSVKWGYLEIVKVI
KRLNLFLVGWGKYFNKGSPSDAYQEINWYVEQRLRNFLERRSQKGYKKPKIIKWHACLKGMGLYTLTRSSLDKCRKPFKRRFM
SEQ ID NO: 12 
MAVLLVTGPIFEVDLFRWQYGFRTGVDAKMALRRIHFGIVDRGARDVVDADLSDYFDTIPHGDLMRCVARRIADGSVLSVIRDWL
NAPVIERTSNGERQSTEAKDSHRGTPQGGVISPLLANLYFRRFMLAWYGHGYADKYQAEIVNYADDFVILCREGRGEGAMEAMRH
LMGKLGLTVNEKKTRLVKLPDERFDFLGYTVGRFYGLGGRPYWGTRPSKKSIKRLKDAVHDATTRRWNALDVESRVTELNPLLRGW
AGYFNQGPVTRIYEDLDRYVARRLRVWLMRKRGKTGTGYRQYSDQFLYESLGLIRLRTLAANRSNAKV
SEQ ID NO: 13 
MTKASISLQELRRKIYRKAKIEKQWRFWGLYCHVCKKEVLREAYRLAKANDGAPGNDGKSFEDIEAEGVEGFLEGIGQELLNRTYRPL
PNRKVEIPKGNGKTRILGIPTVKDRVVQGALKLLLEPIFEADFKECSYGYRPKRHAHQAIDRVTKGILYGLTRVVDVDLSGYFDNIRHHI
LLEKIARRVQDDDIMHLVKLILKANGKKGVPQGGIISPLLSNLYLNEVDEMMERAREVTRSKGYENLDFIRYADDMVILVHWHPKED
WILQKVQKRHKEELDKLEVEMNLEKTKVVDLKKGGSFSFLGFDFRLTRNREGKAYVSKTPRKKKCIEIGKRVRAVLKANWNKPLKEVI
QAVDAIIRGWVNYFRIGNSNSAFNKVRDYLEMKVRKFVLRRKKLKGFGWKMWSREEIYGKWGLYNDYRIRYVVPKANSS
SEQ ID NO: 14 
MQTKLHRWAVADHGRRFDDVYNFVSDPATLVVALQRVAGNAGARTAGVDGVTAVHVSMAGPEVFLDHVRFLLKAGTFRPLPVR
EKMIPKTGGKLRRLGIPTITDRVVQAALKLVLEPIFEADFRPCSYGFRPNRRAHDAIAEIHHLATAGYRWVLDADIEACFDRIDHPALM
GRVRARVKDKRVLTLVKAFLKAGIMTELGERNETTSGTPQGGILSPLLANIALSVLDEFFAEQWAAAGTGQQRRQRRAKGLATWQL
VRYADDFLVLVHGNQEHVEDLRDQVSTVLATMGLRLSESKTRIAHLADGVDFLGFRIVWQRKRGTSKWHVYTFIADKPVQALKRKI
 
KSLTRRLSHMSYRETLVRINYIQRGWANYFKHAVAKHTFSHLQNFIWWRVINWVMRRNRMTWRAIQRWLRTPQGWRPIVFDGV
ELFKIAKVSVTRYRYRGTKIPNPWQPTPTSPPPTA
SEQ ID NO: 15 
MCANRGAPGVDGVTVDAVETSGVDAFLQDLSQRLRTHTYRPSVLRRVQIPKPGRPGEFRPLSIPTVADRVVMTAAKLVLEPVFEAQ
FTEASYGFRPKRSAIDACEAVRVAANQRREWVLEADIRDCFGTIDHEALMAQVARRVVDRPMLKLIRAWLRMGVLEGGVTSPTGA
GTPQGSPISPLLANIALHVLDEAWQNEGCRLGVLVRYADDFVVLSPTEQRAEQARELAARVLERLGMRLHPEKTGIVRLTRGGQGF
DFLGFHHRKMESWKWRGRYYLQRWPSARAMRVLRDKVRAATARSKTERPVSAVVADLNPVLRGWAAYFRNGNSGRKFNVVDG
YVHERLAIFASAKHRLAGRNWTARFTYGWITRLGVYRLTGNVHRATAHASR
SEQ ID NO: 16 
MEGAKSNTVSAIDGRQRKLGGQQGQTADGHLGMVGAELQEYPEAPTHMRIDPERKLDTKPDLLERICDRNNMRQAYVKVARNG
GAGGVDGMSVKELDTWLHDNYDQLVLRILAGKYRPQPVRRVKIPKEEKGKLRLLGIPTVIDRMVQQAAVQVLTPIYEPMFSDASFG
FRPNRSAHDALLRIKELADARDVWICSIDLERFFDTVNQSKLIQVMSNVVKDGRVVSLMHRFLKAGVMVDGVVMPTDEGTPQGG
PLSPLLANVLLNELDRELERRGHHFVRYADDLIILKRSRKAAERAMGTVTKFIEGRLFLKVNRDKSYVAHIASHDPEVKYLGYGFFRYSG
ELHFRVHSKSAAKLKAKVKEVTSRSNGWSLDYRRKRLSWLVNGWVSYFRLADMTKLLRSVDEWLRRRIRCVYWKCWKKISTKYKAL
MKLGVSRNLAWQWANSRKAYWRIAGSAILARALNNTKLDELGWEFLYQRCLLVKC
SEQ ID NO: 17 
MIAENLLEQFLSTSNFLTAYRRIASKKAAGGLDGITVEEFGHRLDQHITKLQKDIRERRYIPQPAAVTYIPKFNEENEWRELGLPSVADK
VVQAAMLEVVEPLAEKMFLDCSYAYRPGTGHYKAIRRVENSLNNRKKTWVVQRDIDNFFDTVDHNRLMEQFSALVQGEPTMVEL
VALWCRMGLVEKNGRWRNVQAGIRQGGVISPLLANLYLHPLDVFATKLGVDWIRYADDYVILGESQEEVVSSDVQIVEFLKDSLGL
MLNRDESSPKHIDEGFTFLGVRFCGKERAIDTKKIKKMKKKIRWLLAQKMVKTPEEVISKLTNQVESWQRYYSFLNPTRQFSEIDCFIE
KEFLTLATSKIQTGKWDRIPPQGLSFPSLIADGKRDGFKKWEGLWAQAIKTVETDDVNHDTARINHSVEKKISRKRQKCRKESGESG
NLIVTTPGHFVGKRGERIVVMSKQRIVSELPVVRLSGLTLSGRGVSISGDVVELCMKKDVYIHFVDNLGKIIAVVGPPGGSSGEVSLLQI
TERDKEKGLTLAKMFILGKVKNQFALLKYYFKYPLNRENGFGKIFVERRQFLSDGIEKIKNATVLSDPETFRQQMMGWEGAFGAAY
WEIVGHLFRNGVQFSGRVRHGATDLVNSALNYGYGILYGDCLNAVIRTGLNPMAGFLHSYQTGKPTLIYDLIEEFRPFAVDRGIFTML
NRGERLEQGDDGMLATETRKKISKSVISRLSNEVWFHGRRLTLREVIQEQAYNIKRHLSDKAQYHPFLGRW
SEQ ID NO: 18 
MAVHSIDRNTWLTKLERIKLLSSKNQDIKFNNLGHIIDLKMLEEQYKELDSKKAIGMDGITKEDYGKKLKANLLSLLTRIRKGQYQAKP
ARIVKIPKEDGGKRPLVISCFEDKIIESAVSKILNSVFEPIFLKYSYGFRPKLNAHDALRELNRLTYNFNKGAIVEIDITKCFNTIKHCELMEF
LRKRISDKKFLRLIMKLIETPIIENGTIVTNKEGCRQGSIVSPILANVFLHYVIDSWFAKISEENLIGQTGMVRYADDMVFVFESEADAKR
FYDVLPKRLNKYGLNINEAKSQMIKSGRDHAANLAKQGKKIASYNFLGFTCYWGKSRFGTTWRLKYTSRRDRFTEKLKGLRKYLRGQ
LNTLDKTQTLSQVIRVIR
SEQ ID NO: 19 
MNVTESRFKNRQLHIEDYLQMVSAEQKEYAEVFDYSKITEKSGVITDYWTNNLLDLILRKDNLNNAYKQVKKNGKGGNDGMQVD
ELLPFLRENQDTLIRKIREGKYKPNPVRRVEIPKETEGEFRKLGVPTVVDRVIQQAIAQELSPVYEKQFSENSFGFRPKRGAHDALRQC
QKNVNDGYVYVVDMDLEKFFDTVCQSKLIEVLSRTIKDGRVISLIHKYLNAGVIAKGMFERTEVGMPQGGPLSPLLSNVMLNELDKE
LESRGHRFVRYADDCMIFCKSRKSAERTLKNIIPFIEGKLFLKVNRKKTEVSHISKVKYLGYSFYRYKGKCRFRVHSKSVVKMKNRIRELT
DRNKGISNEVREKRYQEYVRGWVEYFRLADMKEFLRKTDEWARRRIRAVYWKQWKKIKTKYRMLKALGLEDWKAKELANSRKGY
WRMAKVLNQIFSKKIIAKLGYTSMLDYYLIICEN
SEQ ID NO: 20 
MNVTESRFKNRQLHIEDYLQMVSAEQREYAEVFDYSKITEKSGVITDYWTNNLLELILRKDNLNNAYKQVKRNRGKGGIDGMQVDE
LLPFLRENQETLIQEIKGGKYKPNPVRRVEIPKETKGEYRKLGVPTVVDRVIQQAIAQELSPIFEEQFSENSFGFRPKRGAHDALRQCQK
 
NVNDGYVYVVDMDLEKFFDTVCQSKLIEVLSRTIKDGRVISLIHKYLNAGVIANGKFERTEVGMPQGGPLSPLLSNIMLNELDKELER
REDRFVRYADDCMIFCKSRKSAERTLKNIIPFIEGKLFLKVNRKKTEVAHISKVKYLGYTFYRYKGKCRFRVHQKSVVKMRNKIRELTDR
NKGISNAERERKYQEYVRGWVEYFRLADMKGLLKITDEWARRRIRAVYWKQWKKIKTKYRMLKALGMEHWKAKELACSRKGYWR
MAKVLNQIFSKKIIAKLGYTSMLDYYLVVCEN
SEQ ID NO: 21 
MDSKDMQRLQTTQQRGYPLNREMEFQKTTEVHSISSASEDGRNEVQRYTSKMLEMIVERGNMEAAYKRVVANKGSHGVDGME
VDELLPYLKENWATIKQQLLEGKYKPQPVRRVEISKPDGGVRLLGIPTALDRLIQQAIAQILNRVYNHTFSDSSYGFRPGRSAKDAIKA
AEAYINEGYTWVVDMDLEKFFDRVNHDIIMSKLEKRIGDKRVLKLIRRYLESGVMINGIKVSTEEGTPQGGPLSPLLANIMLDELDKEL
EKRGHKFCRYADDCNIYVKSRSAGNRVMKSIKKFIESKLKLKINEAKSAVDRPWRRKFLGFSFYTKENEVRIRIHEKSIKRFKEKVREITN
RNKGISMENRIKRLNQITTGWVNYFGLADAKSIMKTLDEWIRRRLRACIWKQWKKIKTKHDNLVKLGVEEQKAWEYANTRKGYW
RISNSPILNKTLTNKYFESIGYKSLSQRYLIVHNS
SEQ ID NO: 22 
MRIPKVVMDNLVEMSKRDENYVFERIYRNLYNEEFFLEAYAKLAKKEGNMTEGTDGKTIDGMSIDRIRKLIEQLKDESYQPNPSRREY
IPKKPTGQRPLGIPSMDDKLVQEVARRILEGLYEPIFRDSSHGFRPNLSCQTAVDKIQKTFTGVKWFVEGDIKGFFDNIDHHTLINLLR
KRIKDERFIRLIWKFLKAGYLEDWVYHKTFSGTPQGGVISPLLANTYLHELDKYMEQYAEEFNKGKKRKRTHDYRALEWQLTKMKKS
DEEWDLMSTSEKKEHTKAYKALIKKRNEMNATDPFDTDFRRINYVRYADDFLVGVIGSKEDAEKTKQDIKAFLSNKLKLELSVEKTLIT
HAHKEKARFLGFDIVTSYSTQPTRNKDGKLQRQNNGKVKLYVPKEKWVNKLLDRNILRINGNDWKMVHRPELINNDDLEIISIYDAE
LRGFYEYYKIANNVSVLNKMAYIMEYSMYKTFARKYKISVRKVIDKFSVNGVFTVQYETKKGMRSKTLISSFTRVEKASTESKIDNLPN
GWVYSGITSLIDRMKARVCEYCGATDVDLEMHHVRKLKDLKKKRSLQKWEVIMIARNRRQLALCARGQGNDCHMKLHKGQL
SEQ ID NO: 23 
MYFAKETLAGHVGLDKLMQTSLRGIRQKAKENEKHRFQNLYQLLNEDTLMEAWKELNKKASAGVDEITAKEFKLNLRANIKEIVSNL
KEKRYRTKLVRRVNIPKGKNESRPLGIPALSDKLVQRAVAKILEVIYEEDFLDCSYGYRPKIGAHKGIKDLTKELQFNKYSYIVEADIKGFF
NNINHEWLIRMIEERVDDSALVGLIRKWLKAGILDANHLVIHPATGTPQGGIISPILANIYLHYALDLWFEKGVKRRCEGEAYLCRYAD
DFVCAFRYKKDADRFYKALGKRLRKFELELAEEKTNIISFSRFRKVENTHFDFLGFEFRWGVSLKGKDIIKRCTSKKKLRASLQNFKEWC
KENRNNRLRKITEMLNKKLRGYFNYYGLIGNYESLWKFYTIAIETLYKWLNRRSQRKSFNWSEFTRLMKWYGVLKPKIVESSDHQLEF
QY
SEQ ID NO: 24 
MKNSKEMRKLQKTSYKEDWTWQVEVELQETMGVHSISTAFESGKDDGKLYTNNLLEKILDSENLRLAFRRVKSNKGSHGVDGMKV
DELPQYLKQNIISLKKSILEGTYRPHPVRRVEIPKPDGGIRLLGIPTVVDRMIQQAVAQVLTPIFEKTFSENSYGFRPKRDAKQAIRKAKE
YMEQGYKWVVDIDLAKYFDTVNHDKLMALVAREIKDKRVLKLIRLFLQSGVLINGVVIETEEGCPQGGPLSPLLSNIMLTELDRELEKR
GHKFCRYADDNNIYVKSKKSGDRVMHSITEFIENKLKLKVNKDKSAVDRPWKRKFLGFSFYQWYGKIGIRVHEKSINKFKAKIRNITSR
SNAWDMNYRMLKLRQCIVGWLNYFRIADIARLTKELDEWTRRRLRMCFWKQWKKIKTKHDNLKNLGIKESKAWEFANTRKSYWR
IANSPILASTLTNSYLEKNRIYKYS
SEQ ID NO: 25 
MDTSSLMEQVLSKDNLNAAYLQVVRNKGAAGVDGMTVEELGAYLSENGESIREKLRTRKYKPQPVRRVEIPKPEGGTRKLGVPTVV
DRFVQQAVAQVLTPIFEEQFHDHSYGFRPNRCAQQAVLKALEMMNDGHSWVVDIDLAKFFDTVDHDKLMTIFGRTIKDGDVISVV
RKFLVSGVMIDDEYEDTIVGTPQGGNISPLLANVMLNEVDKELEARGLDFVRYADDLIIMVGSRQAADRVMKSITRFIEERLGLKVN
GEKSKVDKPKGIKYLGFGFYYDSFAKGYKARPHPKAAAKFREQMKKLTCRSWGVSNGYKVKKLNQLIRGWINYFKIGSMKGLCERLD
SQIRYRLRMCIWKHWKTPQNRAKNLIKLGISRKYAWSTAYTGARIAYVCQRGAMNVAVTKERLTRFGLVSMSDYYAERRVTC
SEQ ID NO: 26 
MDTSSLMEQILSKDNLNAAFLQVVRNKGAAGVDGMEYTELKDYLSENGETIKEQLRTRKYKPKPVRRVEIPKSDGGVRNLGVPTVT
DRFVQQAIAQVLTPIFEEQFHEHSYGFRPNRCAQQAVIKALEMMNDGHSWIVDIDLAKFFDTVDHDKLMTIFGRTIKDGDVISIVRK
FLVSGIMIGDEYEDSIIGTPQGGNISPLLANIMLNELDKELEARGLDFVRYADDCIIMVGSRQAAERVMKSVTRFIEDRLGLKVNATKS
KIDKPKGIKYLGFGFYYDSFAKEYRAKPHAKSIVKFKARMKELTCRSWGVDNAYKVKKLNELIRGWINYFKIGSMKGLCAKLDSNIRFR
LRMCIWKQWKTPQNRAKNLIKLGISRKFAWSTAYYGAHIAHVCQGGAMNMAVTKERLTRFGLVSMLDYYVERRVTC
SEQ ID NO: 27 
MDTSSLMEQILSSGNLNRAYLQVVRNKGAEGVDGMKYTELKEHLEKNGEIIKEQLRTRKYKYQPVRRVEIPKPDGGVRNLGVPTVT
DRFIQQAIAQVLTPIYEEQFHEHSYGFRPNRCAQQAILTALDMMNDGNDWIVDIDLEKFFDTVNHDKLMTIIGRTIKDGDVISIIRKYL
VSGIMIDDEYEDSIVGTPQGGNLSPLLANIMLNELDKEMEKRGLNFVRYADDCIIMVGSEMSANRVMRNISRFIEEKLGLKVNMTKS
KVDRPSGLKYLGFGFYFDTRAHQFKAKPHAKSVAKFKKRMKELTCRNWGVSNSYKVEKLNQLIRGWINYFKIGSMKTLCRKLDGNIR
YRLRMCIWKHWKTPQNRAKNLMKFDVPRWAAFKIAYCGDRYARLAHNGWIQKAISTKGLTSFGLVSMLDYYTERCVTC
SEQ ID NO: 28 
MDTSSLMEQILSSENLNRAYLQVVRNKGAEGVDGMKYTELKEHLEKNGDIIREQLRTRKYKPQPVRRVEIPKPDGGVRNLGVPTVT
DRFIQQAIAQVLTPIYEEQFHDHSYGFRPNRCAQQAILTALDMMNDGNDWIVDIDLEKFFDTVNHDKLMTIIGRTIKDGDVISIVRKY
LVSGIMIYDEYEDSIVGTPQGGNLSPLLANIMLNELDKEMEKRGLNFVRYADDCIIMVGSEVSANRVMRNISRFIEEKLGLKVNMTKS
KVDKPQGLKYLGFGFYFDSRAHQFKAKPHAKSVAKFKKRMKELTSRSWGVSNSYKVEKLNQLIRGWINYFKIGSMKTLCKEMDSRIR
FRLRMCIWKHWKTTQNRIKNLMKLGVDKDTAWITAYTGKRIAYVCQRRVMNFAINKERLTRFGLVSMLDYYTERCVTC
SEQ ID NO: 29 
MDTSSLMEQILSKDNLNTAYLQVVRNKGAEGVDGMTYTELKEHLEKNGEIIREQLRTRKYKPQPVRRVEIPKPDGGVRNLGVPTVT
DRFIQQATAQVLTPIYEEQFHDHSYGFRPNRCAQQAVIKALEMMNDGYDWIVDIDLEKFFDTVNHDKLMTLIGRTIKDGDVISIIRKF
LVSGIMVDDEYKDSVIGTPQGGNLSPLLANVMLNELDKEMEQRGLNFVRYADDSIIMVGSEMSARRVMRNLAKFIEEKLGLKVNM
TKSKVDRPRGLKYLGFGFYFDSRARQFKAKPHARSVVKFKAKMRQLTCRSWGISNSYKVKKLNELIRGWINYFKIGSMKTLCKEMDS
QIRYRLRMCIWKHWKTPQNRAKNLMKLDVPRWAAFKIAYCGDRYARLAHNGWVQKAISNKRLTSFGLVSMLDYYTDRCVTC
SEQ ID NO: 30 
MRGKGETSVELLETILSNQNMNEAYLRVYKNKDASGVDGVTVDELKQYLKENKDELRQRIRTRKYQPQAALRVEIPKENGKMRKLG
IPTVVDRVVQQAIHQILSPIFEKQFSEFSYGFRPKRSCEMAIVKSLEFLNAGYEWIVDIDLERFFDTVHHDKLMRIISNTISDGDVISLIRK
YLVSGVMVNGKYEETSVGTPQGGNLSPLLSNIMLNELDKELESRELQFVRYADDALIFVKSEKAASRVMKSIVRFIEKNLGLIVNTEKS
KISRPEDLKFLGFGYYYNSKDERYQTKPHPISVQKLQRKLRHLTNRNWSVPLDYRILKLKQVIFGWVNYFRITNMKGVMKQVDKKLR
SRIRVIIWKQWKIPKKQIKSLVQLGIPEEEAKGLTYCRKGYRYIGLSKVVQRAMSNQRLKKRGVPSSLERYLKVRTAI
SEQ ID NO: 31 
MEMEQRGEQSISFSRVLIAEKTTSAVPNRERVVNSSETERNERMDTSSLMEQILSKENLNTAYLQVVRNKGAEGVDEMKYTELKEH
LAKNGEIIKEQLRTRKYKPQPVRRVEIPKSDGGVRNLGVPTVTDRLVQQAIAQVLTPIYEEQFHDHSYGFRPNRCAQQAIITALDMM
NDGYDWIVDIDLEKFFDTVNHDKLMTLIGKTIKDGDVISIIRKFLVSGIMVDDEYKESVIGTPQGGNLSPLLANIMLNELDKEMEQRG
LNFVRYADDCIIMVGSEMSAKRVMRNLTKFIEEKLGLKVNMTKSKVDRPNGLKYLGFGFYFDSRAHQFKAKPHEKSVAKFKTKMKQ
LTCRSWGVSNTYKVQKLNELIRGWINYFKIGSMKTLCAKMDSNIRYRLRMCIWKHWKTSQNREKNLVKLGIDRNTARRVAYTGARI
AYVCNKGAVNVAINNKRLASFGLISMLDYYTERCVTC
SEQ ID NO: 32 
MVKSSETERKERMDTSNLMEQILSSDNLNRAYLQVVRNKGAEGVDGMKYTELKEHLAKNGEIIKEQLRTRKYKPQPVRRVEIPKPD
GGVRNLGVPTVTDRFVQQAIAQVLTPIYEEQFHDHSYGFRPNRCAQQAILTALDMMNDGNDWIVDIDLEKFFDTVNHDKLMTLIG
RTIKDGDVISIVRKYLVSGIMIDDEYENSVIGTPQGGNLSPLLANIMLNELDKEMEKRGLNFARYADDCIIMVGSEMSANRVMRNISR
FIEEKLGLKVNMTKSKVDKPQGLKYLGFGFYFDSRAHQYKAKPHAKSVEKFKKRMKELTCRSWGVSNSYKVEKLNQLIRGWVNYFKI
 
GSMKHLCREMDKHIRFRLRMCIWKQWKTPQNRAKNLMKLGVPQWAAKRTSYAKGFARVCRGSDVCQAISNKRLTSFGLVSMLD
YYTERCVTC
SEQ ID NO: 33 
MEEQGIFIERILSNENMNRAYKQVKKNKGAAGIDGMECADLLSHLKVNGQQLRESIRNQSYKPMPVKRVEIPKADGSKRKLGIPTVT
DRMVQQAAAQVLTPIYERKFHENSYGFRPGKSAQKAVLKAVEYMNEGYNWVVDIDLEKFFDTVEHDKLISVLNKEIKDGKILSLIRKF
LVSGVMVGEQIEETEIGTPQGGNISPLLANILLNELDWELESRGLKFARYADDCIIFVKSEKAARVMESVTKYVENVLRLKVNRGKSK
IGRPTEIQYLGFAFYYQFQEKKYKIKVPKKSLDKVMKKVRKLTSRKWGVSNSYKAQKIAEVVRGWINYFKIGSILTATRRLDTVIRYRFR
MCIWKHWKNPKTRYKNLVKLGVSKKNAKSAAGFHGYARVCRTETICYAMSNARLKKFGLLSAEEYFCQTRC
SEQ ID NO: 34 
MELLEQILRNQNMNEAYLRVYKNKGASGIDGVTVDELKQYLKENKDELRQRIRTRKYQPQAALRVEIPKENGKMRKLGIPTVVDRV
VQQAIHQVLSPIFEKQFSEFSYGFRPKRSCEMAIIKSLEFLNDGHDWVVDIDLERFFDTVHHDKLMRIIANTIDDGDVISLIRKYLVSGV
MVKGKYEETPVGTPQGGNLSPLLSNIMLNELDKELEGRGLRFVRYADDSLIFVKSEKAADRVMKSIVRFIEDKLGLIVNSEKSKISRPKE
LKFLGFGYYYDGKNKRYQVKPHLSSIQKFQRKLRKLTKRNWSVSLDYRILKLKQVIIGWVNYFRIGNMKNATERIDMKLRSRIRVIIWK
QWKVPRKQIKSLTQLGIPKEEAKGLTYCRKGYRYIGLSKVIQRAISNKRLKQRGLPSALEHYLKVHTVI
SEQ ID NO: 35 
MDTSSLMEQILSRDNLNAAYLQVVRNKGAEGVDGMKYTELKGHLEKNGEIIREQLRTRKYTPQPVRRVEIPKPDGGVRNLGVPTVT
DRFVQQAIAQVLTPVYEEQFHDHSYGFRPNRCAQQAIITALDRMNDGYDWIVDIDLEKFFDTVNHDKLMTLMGKTIKDGEVISIIRK
FPASGIKEDDEYRESVIGTRQGGNLSPLLANIMRNELDKEMEQRGMNFVRYADDCINMVGSEMSAKRVMRNITRFIEEKLGLKVNT
TKSKVDKPQGIKYLGYGFYFDSRAHQYKAKPHAKSAAKFKARMKQLTCRSWGVSNIYKVQKLNELIRGWINYYKIGSMKTICARLDG
SIRYRIRMCIWKHWKTPQNRARNLKKLGVPEWAARRTAYAKGYARVCRASDVCEAINNKRLASFGLISMLDYYTERCVTC
SEQ ID NO: 36 
MELLEQILSNKNMNEAYIRVYKNGASGVDGVTVDELKQYLKENKDELRQRIRTRKYQPQAALRVEIPKENGKMRKLGIPTVVDRVV
QQAIHQVLSPIFEEQFSEFSYGFRPKRSCEMAIIKSLEFLNDEHDWVVDIDLERFFDTVHHDKLMRIISNTIKDGNVISLIRKYLVSGVM
VNGKYEDTPVGTPQGGNLSPLLSNIMLNELDKELEKRGLRFVRYADDALIFVKSEKAANRVMSTIVRFIEEKLGLIVNVEKSRISRPKEL
KFLGFGYYYDANNNNYKVKPHPSSVQKFQRKLRQLTKRNWNVPLAYRILKLNQVIVGWVNYFRVANMKKTTERIDTKLRSRIRVIIW
KQWKVAKKQIKSLIRLGIPEEEAKGLTYCRKGYRYIGLSKVVQRAISNKRLKQRGVPSALERYLKVHTVI
SEQ ID NO: 37 
MYGETLLDKILDTENLNQAYKKVKRNKGSAGTDGMTVQELGAYIALNKKELISQIRQRKYQPQPVLRVEIPKPDGGIRLLGIPTVKDRL
VQQAIAQVLTPMFDKKFSEHSYGFRPNRYAEMAIIQTLEYLNDGHEWIVDIDLERFFDTVNHDRLMNLISRTIQDGDIISLIRKFLVSG
VQIDEDYKETVIGTPQGGNLSPLLSNIMLNELDTELESRGLRFVRYADDCIIMVKSEMAARRVMRSVTRFIEEKLGLIVNTTKTKVTRP
NDPNMKFLGFGFFKDPQVEVYKAKPHQKSVDNFKFKLKQLTRKNWSVETKFQVERINQVVRGWVNYFKIGFMKKALERIDSHTRF
RLRMCIWKKWKTSKNRRKNLIKLGMDRHTAYKYSYSSKGAVRIAYSWVLTKTITNKRLTQFGLLSGVQHYRKVHA
SEQ ID NO: 38 
MDTSSLMEQILSKDNLNTAYLQVVRNKGAEGVDGMKYTELKEHLAKNVEIIKEQLRTRKYKPQPVHRVEISKPDGGVRNLGVPTVT
DRFVQQAIAQVLTPIYEKQFHDHSYGFRPNRCAQQAIITALDMMNDGYDWIVDIDLEKFFDTVNHDKLMTLIGKTIKDGDVISIIRKF
LVSGIMVDDEYRESVIGTPQGGNLSPLLANIILNELDKEMEQRGLNFVRYADDCIIMVGSEMSARRVMRNLTKFIEEKLGLKVNMTK
SKVDRPRGLKYLGFGFYFDSRTHQYKAKPHAKSVARFKARMKQLTCRSWGVSNSYKVQKLNELIRGWINYFKIGSMKTLCAKLDSNI
RYRLRMCIWKHWKTPQNRAKNLMKLGIEKNRAFHVAYAGYRIAYVCNKGAVNVAISNKRLTQFGLVSMLDYYTKRCVTC
SEQ ID NO: 39 
MDTSSLMEQILSKENLNTAYLQVARNKGAEGVDDMKYTELKEHLEKHGETIKEQLRARKYKPQPVRRVEIPKPDGGVRNLGVATVT
DRFVQQAIAQVLTPIYEEQFHDHSYGFRPNRCAQQAIITALDMMNDGSAWIVDIDLEKFFDTVNHDKLMTLIGRTIKDGDVISIIRKF
LVSGIMDDDEYKESVIGTPQGGNLSPLLANIMLNELDKEMEQRGLNFVRYADDCIIMVGSEMSAKRVMRNLTKFIEEKLGLKVNMS
KSKVDRPSGLKYLGFGFYFDSRAHQYKAKPHAKSVAKFKARMKQITCRSWGVGNDYKVKKLNELIRGWINYFKIGSMKTLCAKMD
NNIRYRLRMCIWKHWKTPQNRAKNLIKLGVPGWSAWKMAYLHGYAKPARCRDVHQAINNKRLASFGLISMLDYYTERCYLLS
SEQ ID NO: 40 
MNTENLLKQILHPNNLNQAYLQVKRNKGAAGVDGMTINELGHYLKENGEEIKDQIRTRSYQPKPVKRIEIPKADGGVRNLGVPTVV
DRFIQQAMAQVLTPIYEEKFHENSYGFRPGRCAEMAIIKSLEFMNDGYTWIVDIDLEKFFDKVNHDKLMRLISNTIKDGDVISLIRKFL
VSGVMMDDEYKESVIGTPQGGNLSPLLSNIMLNELDQELEARGLNFVRYADDCLILVKSEKAANRVMKSMTKYLEETLGLKVNVTK
SKVERPSGIKFLGFGFFWDKNAYQFKAKPHQISIMRVKEKLKRLTRRSWSVSFDYRLKKLKQLIIGWVNYFKIAKMRTVCGNLDKNIR
FRLRMCIWKQWKKVQTKYRSLMRLGIDKDKAWEWANTRKGYARVARSFILCRTITNERLKRRGLVSLLDHYQTVHI
SEQ ID NO: 41 
MYEGKLLDKILDIDNLNQAFKQVKRNKGAAGVDGMTVQELGAYMALNKDEIISQIRQRTFNPQPVLRVEIPKPNGGVRLLGIPTVK
DRVIQQAIAQILTPMFDKTFSEYSYGFRPNRYAEMAIIQTLDYLNDGHDWIVDIDLERFFDTVNHDRLMNLVSRTVDDGDVISLIRKF
LVSGVQIDEEYKETIIGTPQGGNLSPLLSNIMLNELDIELEGRGLRFVRYADDCIIMVKSEMAAKRVMRSVTKFIEEKLGLIVNSTKTKV
TRPNDPNMKFLGFGFFKDYQSDLYKAKPHQKSVENFQYKLKQLTRKNWSVETKFQVQRINQVIRGWVNYFKVGYMKTVLGKIDA
HIRVRLRTCIWKKWKTAKNRRKNLIKLGMERYNAYKYSHTSKGAVRIAYSWVLSTTITNKRLARFGLISCVQHYNKVHA
SEQ ID NO: 42 
MDTSSLIEQILSSKNLNEAYLQVFRNKGAEGVDGMKFTELKEHLVKNGEEIKEQLRSRKYKPQPVRRVEIPKPDGGVRNLGVPTVTDR
FIQQAIAQVLTPIYEEQFHDHSYGFRPNRCAQQAIMTALDMMNDGNTWIVDIDLEKFFDTVNHDKLMTIIGRTIKDGDIISIVRKFLV
IGIMVDNEYEESVIGTPQGGNLSPLLANIMLNELDKEMEKRGLNFVRYADDCIVMVGSEMSANRVMRNLTRFIEEKLGLKVNMTKS
KVGRPQGLKYLGFGFYFDSHAHGYKVKPHAKSVEKFKVRMKQLTRRNWGVSNSYKVEKLNQLIKGWINYFRIGEMKWICKDMDA
KIRRRLRMCIWVHWKTPENRAKNLMKLGMYRQKAYAIAYSGSRVARLSEGALNYVITNQRLTTFGLVSMLDYYTDRCVTC
SEQ ID NO: 43 
MDTSNLMEQILSSDNLNRAYLQVVRNKGAEGVDGMKYTELKEHLAKNGETIKGQLRTRKYKPQPARRVEIPKPDGGVRNLGVPTV
TDRFIQQAIAQVLTPIYEEQFHDHSYGFRPNRCAQQAILTALNIMNDGNDWIVDIDLEKFFDTVNHDKLMTLIGRTIKDGDVISIVRK
YLVSGIMIDDEYEDSIVGTPQGGNLSPLLANIMLNELDKEMEKRGLNFVRYADDCIIMVGSEMSANRVMRNISRFIEEKLGLKVNMT
KSKVDRPSGLKYLGFGFYFDPRAHQFKAKPHAKSVAKFKKRMKELTCRSWGVSNSYKVEKLNQLIRGWINYFKIGSMKTLCKELDSRI
RYRLRMCIWKQWKTPQNQEKNLVKLGIDRNTARRVAYTGKRIAYVCNKGAVNVAISNKRLASFGLISMLDYYIEKCVTC
SEQ ID NO: 44 
MPPCYPTMTVDQTTGAVTNQTETSWHSINWTKANREVKRLQVRIAKAVKEGRWGKVKALQWLLTHSFYGKALAVKRVTDNSGS
RTPGVDGITWSTQEQKTQAIKSLRRRGYKPQPLRRVYIPKANGKQRPLGIPTMKDRAMQALYALALEPVAETTADRNSYGFRRGRC
TADAAGQCFLALAKAKSAEHVLDADISGCFDNISHEWLLANTPLDKGILRKWLKSGFVWKQQLFPTHAGTPQGGVISPVLANITLDG
MEELLAKHLRGQKVNLIRYADDFVVTGKDEETLEKARNLIQEFLKERGLTLSPEKTKIVHIEEGFDFLGWNIRKYNGVLLIKPAKKNVKA
FLKKIRDTLRELRTATQEIVIDTLNPIIRGWANYHKGQVSKETFNRVDFATWHKLWRWARRRHPNKPAQWVKDKYFIKNGSRDWV
FGMVMKDKNGELRTKRLIKTSDTRIQRHVKIKADANPFLPEWAEYFEKRKKLKKAPAQYRRIRRELWKKQGGICPVCGGEIEQDMLT
DIHHILPKHKGGSDDLDNLVLIHANCHKQVHSRDGQHSRSLLKEGL
SEQ ID NO: 45 
MALLERILARDNLITALKRVEANQGAPGIDGVSTDQLRDYIRAHWSTIRAQLLAGTYRPAPVRRVGIPKPGGGTRQLGIPTVVDRLIQ
QAILQELTPIFDPDFSPSSFGFRPGRNAHDAVRQAQGYIQEGYRYVVDMDLEKFFDRVNHDILMSRVARKVKDKRVLKLIRAYLQAG
VMIEGVKVQTEEGTPQGGPLSPLLANILLDDLDKELEKRGLKFCRYADDCNIYVKSLRAGQRVKQSIQRFLEKTLKLKVNEEKSAVDRP
 
WKRAFLGFSFTPERKARIRLAPRSIQRLKQRIRQLTNPNWSISMPERIHRVNQYVMGWIGYFRLVETPSVLQTIEGWIRRRLRLCQW
LQWKRVRTRIRELRALGLKETAVMEIANTRKGAWRTTKTPQLHQALGKTYWTAQGLKSLTQRYFELRQG
SEQ ID NO: 46 
MYAITKECLEKGDVPRFKGLVEIASSDVVIVSAIHKIKSNQGNSTAGTDGKTISDILTLNYDEAINFVKRCFKKYTPNPIRRVHIPKPGKK
EKRPLGILTIADRIIQECVRMVIEPILEAQFFQHSYGFRPYRDAKQAIERCVFICNRIGYNWVIEGDIKGFFDNVNHTILIKQLWHMGIR
DRRMLMIIKAMLKAGVIKETKINEMGTPQGGIISPLLANVYLHKLDQWITREWEEKKMRNGTTIRTAKYKSLRDHSTITKPEFYVRYA
DDWILFTNSRGNAEKWKYRIKKYLKENLKLELSDDKTLITNIKKKPMKFLGFKIKMIPHGKGGKYIGYASADTEKIKGKVEQIRKDLRKL
KFATNQEWLITDINRINSKIRGIINYYSSAPSVNRDVRPFKEKLKYASYKALKRYGAKWIPANQCYNLAPLYPDRTEQVPAIKINGRWL
GIMSIGFATWIKTNQKNQKETPYTAEGRRIRLQNTGKKPLTVRAQWLLDSGYLNLIQGVKSSKIYNFEFFMNRCYAFNRDKGKCKIC
GDILQPFNTRTHHINTKLPLNEINKVSNLVTVCDKCHTLIHLKDLSDVNLSRLKTNAIRKLEEYRKCVH
SEQ ID NO: 47 
MPKKSKTLCVDDLRHAEYYGMQGTFDELYQKSQSGEVFENLMDLILSRDNILLAYRNIKANKGSYTAGTDKKNITDIGSQTPDDVVK
RVRFIVTGSEHGYRPKPVRRKDIPKPNGKTRPLGIPCIWDRLIQQCIKQIMEPICEAKFCNNSYGFRPNRSVEHAINRTYTMLQMMN
LHYVIEFDIKGFFDNVNHSKLIRQIWSLGIHDKTLIFIIKRILTAPIKMPDNTTVLPNKGTPQGGIISPLLANIVLNELDWWIASQWEENP
IAISRGRERIIGKTKVFDKSHGYRIMKNTEMKEMHIIRYADDFRIFCRTKEDAVRTKEAVTAWIEERLKLEVSPEKTRIVNTRKRWSEFL
GFKIRVRLKHHKYVVQSAICDKKVEIERAKLVEQAKNIAKPREKKSCLSEIQLYNSMVLGIQNYYQLATCISIDCRELHRRVMTVLTNRL
NTETGSMLKHEGGTITQAEKERFGQSKMIRYVSGIDQMIYPIAFIKNKIPMAKRSIVCSYTKEGRAPIHTELNLNQYVLKGLREKISVG
HSTEYHDSKISLFSAQKGKCAISGEEFADAEHVAVWLKVPRALGGFERYKNMVLIHKKYLILLQELPQAVIKDLIKTLNITKKMLVKINS
LREQANLSAII
SEQ ID NO: 48 
MDSKDMQRLQTTQQRGYPLNREMEFQKTTEVHSISSASEDGRNEVQRYTGKMLEMIVERGNMEAAYKRVVANKGSHGVDGMG
VDELLPYLKENWATIKQQLLEGKYKPQPVRRVEIPKPDGGKRLLGIPTVLDRLIQQAIAQILNKVYNHTFSDSSYGFRPGRSAKDAIKA
AEAYINEGYTWVVDMDLEKFFDRVNHDIIMSKLEKRIGDKRVLKLIRRYLESGVMINGIKVSTEEGTPQGGPLSPLLANIMLDELDKEL
EKRGHKFCRYADDCNIYVRSRSAGNRVMKSIKKFIESKLKLKVNEAKSAVDRPWRRKFLGFSFYTKENEVRIRIHEKSIKRFKEKVREIT
NRNKGISMENRIKRLNQITTGWVNYFGLADAKSIMKTLDEWIRRRLRACIWKQWKKIKTKHDNLVKLGVEEQKAWEYANTRKGY
WRISNSPILNKTLTNKYFESIGYKSLSQRYLIVHNS
SEQ ID NO: 49

MAVPKKVLKRQILRNIEYYGLQEVFDELYQKSLENRKFKNLMELILMEENIKLAYRNMKKNDGSTTPGIDGKTIEHLAKMTEKEVIELV
RNKLEWYTPKAIRRVEIDKGNGKKRPLGIASIEDRLIQQCILQVLEPICEAKFHDRSNGFRPNRGVENALAQAEKLIQSNKLYIVVDIDIK
GFFDNVSHGKLLKQLWTIGIQDKKLISIISAMLKGEIAGIGFPEKGTAQGSIISPLLSNVVLNELDWWIASQWEFMPTRHVYKEAIKAN
GTQSKSKKYRALRSSTLKECFIVRYADDFKIFCRKHKDAVVMFEATKQWLKTRLGLDISPEKSKIVNLKHSYSEFLGFRIKVHKKGKDTK
CKPPVDKYVVKSHISEKALKKIKTNAKERIIAIQKTNGSRAGEFAIRDYNSFVMGVQNYYSMATCVNPDMQTLAYEIKTSIKIRLNTRV
KRRTNEVLTPYLSERYGKSKELRFINGVPLVPIGYVQHRVPLHKKAVVNKYTAEGRKEIHKQLETVDIERVHELMKNPVSDETVEFND
NCVSLFVAQRGKCAICKLPLTLENMRCIRKIPKSCGGDDKYNNLIIVHKEMERLIKSQDKKEIKRILNKYKLESNQRRKVNTIRKNAGLE
EIDVKALDKEL

### EXAMPLES

### Example 1: Group II Intron reverse transcriptase (Intron-RT) generates multiple-repeat copies of circular RNA (circRNA) in the resulting linear cDNA, and therefore produces a higher yield of cDNA than retroviral reverse transcriptases that generate single copies of the circRNA

Rolling circle reverse transcription of circRNA (0.4 kb, sequence shown below) was carried out with an Intron-RT or retroviral reverse transcriptase (SuperScript^{™} II, ThermoFisher Scientific, Waltham, MA). The reaction using Intron-RT was performed as following: 2 µl of Induro^{™} buffer (New England Biolabs, Ipswich, MA), 1 µl of dNTP (10 mM of each), 1 µl of circRNA specific primer (10 µM, sequence shown below), 1 ug of circRNA and 100 ng of Intron-RT. The reaction was incubated at 55°C for 0 minutes, 20 minutes, 1 hour and 2 hours, respectively. The reaction for SuperScript II is under the following condition: 2 µl of SupersScript II buffer (5X), 1 µl of dNTP (10 mM of each), 1 µl of circRNA specific primer (10 µM), 1 µg of circRNA and 0.5 µl of SuperScript II RT. The reaction was incubated 42°C for 0 minutes, 20 minutes, 1 hour and 2 hours. The results are shown in FIG. 2A. The cDNA yield for each reaction condition was determined to be at least 2 fold greater and much as 4 fold using Intron-RT than was obtained for SuperScript RT where the concentrations of cDNA obtained were as follows: 0.06 µg, 2.5 µg, 4.5 µg, 4.4 µg, 0.1 µg, 0.5 µg, 0.6 µg and 0.6 µg, respectively.

The circRNA was *in vitro* synthesized following the protocol published by Wesselhoeft RA, et al., Nature Commun 9, 4475 (2018).
circRNA (0.4 kb) (SEQ ID NO: 50) 
AAAAUCCGUUGACCUUAAACGGUCGUGUGGGUUCAAGUCCCUCCACCCCCACGCCGGAAACGCAAUAGCCGAA
AAACAAAAAACAAAAAAACCCCCCUCUCCCUCCCCCCCUAACGUUACUGGCCGAAGCCGCUUGGAAUAAGGCCG
GUGUGCGUUUGUCUAUAUGUUAUUUUCCACCAUAUUGCCGUCUUUUGGCAAUGUGAGGGCCCGGAAACCUG
GCCCUGUCUUCUUGACGAGCAUUCCUAGGGGUCUUUCCCCUCUCGCCAAAGGAAUGCAAGGUCUGUUGAAUG
UCGUAAAAAAACAAAAAACAAAACGGCUAUUAUGCGUUACCGGCGAGACGCUACGGACUU
Primer sequence:
   TCCAGAGGAACTGCTTCCTTC (SEQ ID NO: 51)

In a second example, the retroviral reverse transcriptases were SuperScript IV (SSIV), ProtoScript^{®} (PSII) (New England Biolabs, Ipswich, MA), and the Intron-RTs were TGIRT^{™} (Ingex, Olivette, MO) and Induro. Reverse transcription of 1 kb RNA was initiated with primers specific to the RNAs that were either circular (C) (SEQ ID NO: 52) or linear (L) (SEQ ID NO: 53). Reverse transcription with retroviral RTs (SSIV and PSII), and Intron-RTs (TGIRT and Induro) were carried out according to manufacturers' protocols. For the Intron-RTs, 1 pmol of linear or circRNA was incubated with 2 µl of 10 µM primers (SEQ ID NO: 51 and SEQ ID NO:54 respectively) in a volume of 28 µl at 65°C for 5 minutes. The reaction was cooled down to 4°C with 0.1°C /second ramp and 2 µl 10mM dNTPs, 8 µl 5X Intron-RT buffer and 200 ng Intron-RT were added to the reaction followed by an incubation at 55°C for 1 hour. When the reverse transcription reactions reached completion, 1.5 µl of Thermolabile Proteinase K (New England Biolabs, Ipswich, MA, P8111S) was added to all the RT reactions and incubated for 15 minutes at 37°C and 20 minutes at 55°C. cDNAs were purified with SPRI beads and eluted in 26 µl water. 24 µl of this elution was mixed with 3 µl 10X RNaseH buffer, 1.5 µl RNaseH (New England Biolabs, Ipswich, MA), and 1.5 µl RNase I_{f} (New England Biolabs, Ipswich, MA) to degrade the template RNA and incubated at 37°C for 30 minutes and 70°C for 20 minutes. 10 µl of the reactions were run on 1% agarose gel together with 10 kb ladder (New England Biolabs, Ipswich, MA). cDNA (>10kb) containing multiple repeat complementary sequences to circRNA was observed using Intron-RTs, while the majority of the cDNA produced by MMLV-RT variants (SSIV and PSII) is around 1kb, equivalent to single copy of circRNA due to the poor strand displacement activity. The cDNA copies for linear RNA was single copy for all reverse transcriptase products. The results are shown in FIG. 2B.
circRNA (1.3 kb) (SEQ ID NO: 52) 
AAAAUCCGUUGACCUUAAACGGUCGUGUGGGUUCAAGUCCCUCCACCCCCACGCCGGAAACGCAAUAGCCGAA
AAACAAAAAACAAAAAAACCCCCCUCUCCCUCCCCCCCUAACGUUACUGGCCGAAGCCGCUUGGAAUAAGGCCG
GUGUGCGUUUGUCUAUAUGUUAUUUUCCACCAUAUUGCCGUCUUUUGGCAAUGUGAGGGCCCGGAAACCUG
GCCCUGUCUUCUUGACGAGCAUUCCUAGGGGUCUUUCCCCUCUCGCCAAAGGAAUGCAAGGUCUGUUGAAUG
UCGUGAAGGAAGCAGUUCCUCUGGAAGCUUCUUGAAGACAAACAACGUCUGUAGCGACCCUUUGCAGGCAGC
GGAACCCCCCACCUGGCGACAGGUGCCUCUGCGGCCAAAAGCCACGUGUAUAAGAUACACCUGCAAAGGCGGC
ACAACCCCAGUGCCACGUUGUGAGUUGGAUAGUUGUGGAAAGAGUCAAAUGGCUCUCCUCAAGCGUAUUCAA
CAAGGGGCUGAAGGAUGCCCAGAAGGUACCCCAUUGUAUGGGAUCUGAUCUGGGGCCUCGGUGCACAUGCUU
UACAUGUGUUUAGUCGAGGUUAAAAAACGUCUAGGCCCCCCGAACCACGGGGACGUGGUUUUCCUUUGAAAA
ACACGAUGAUAAUAUGGCCACAACCAUGGGAGUCAAAGUUCUGUUUGCCCUGAUCUGCAUCGCUGUGGCCGA
GGCCAAGCCCACCGAGAACAACGAAGACUUCAACAUCGUGGCCGUGGCCAGCAACUUCGCGACCACGGAUCUCG
AUGCUGACCGCGGGAAGUUGCCCGGCAAGAAGCUGCCGCUGGAGGUGCUCAAAGAGAUGGAAGCCAAUGCCC
GGAAAGCUGGCUGCACCAGGGGCUGUCUGAUCUGCCUGUCCCACAUCAAGUGCACGCCCAAGAUGAAGAAGU
UCAUCCCAGGACGCUGCCACACCUACGAAGGCGACAAAGAGUCCGCACAGGGCGGCAUAGGCGAGGCGAUCGU
CGACAUUCCUGAGAUUCCUGGGUUCAAGGACUUGGAGCCCAUGGAGCAGUUCAUCGCACAGGUCGAUCUGUG
UGUGGACUGCACAACUGGCUGCCUCAAAGGGCUUGCCAACGUGCAGUGUUCUGACCUGCUCAAGAAGUGGCU
GCCGCAACGCUGUGCGACCUUUGCCAGCAAGAUCCAGGGCCAGGUGGACAAGAUCAAGGGGGCCGGUGGUGA
CUAAAAAAAACAAAAAACAAAACGGCUAUUAUGCGUUACCGGCGAGACGCUACGGACUU
Primer sequence: SEQ ID NO: 51
Linear RNA (SEQ ID NO: 53) 
GGGUCUAGAAAUAAUUUUGUUUAACUUUAAGAAGGAGAUAUACAUAUGAAAAUCGAAGAAGGUAAAGGUCA
CCAUCACCAUCACCACGGAUCCAUGGAAGACGCCAAAAACAUAAAGAAAGGCCCGGCGCCAUUCUAUCCUCUAG
AGGAUGGAACCGCUGGAGAGCAACUGCAUAAGGCUAUGAAGAGAUACGCCCUGGUUCCUGGAACAAUUGCUU
UUACAGAUGCACAUAUCGAGGUGAACAUCACGUACGCGGAAUACUUCGAAAUGUCCGUUCGGUUGGCAGAAG
CUAUGAAACGAUAUGGGCUGAAUACAAAUCACAGAAUCGUCGUAUGCAGUGAAAACUCUCUUCAAUUCUUUA
UGCCGGUGUUGGGCGCGUUAUUUAUCGGAGUUGCAGUUGCGCCCGCGAACGACAUUUAUAAUGAACGUGAA
UUGCUCAACAGUAUGAACAUUUCGCAGCCUACCGUAGUGUUUGUUUCCAAAAAGGGGUUGCAAAAAAUUUU
GAACGUGCAAAAAAAAUUACCAAUAAUCCAGAAAAUUAUUAUCAUGGAUUCUAAAACGGAUUACCAGGGAUU
UCAGUCGAUGUACACGUUCGUCACAUCUCAUCUACCUCCCGGUUUUAAUGAAUACGAUUUUGUACCAGAGUC
CUUUGAUCGUGACAAAACAAUUGCACUGAUAAUGAAUUCCUCUGGAUCUACUGGGUUACCUAAGGGUGUGG
CCCUUCCGCAUAGAACUGCCUGCGUCAGAUUCUCGCAUGCCAGAGAUCCUAUUUUUGGCAAUCAAAUCAUUCC
GGAUACUGCGAUUUUAAGUGUUGUUCCAUUCCAUCACGGUUUUGGAAUGUUUACUACACUCGGAUAUUUGA
UAUGUGGAUUUCGAGUCGUCUUAAUGUAUAGAUUUGAAGAAGAGCUGUUUUUACGAUCCCUUCAGGAUUAC
AAAAUUCAAAGUGCGUUGCUAGUACCAACCCUAUUUUCAUUCUUCGCCAAAAGCACUCUGAUUGACAAAUAC
GAUUUAUCUAAUUUACACGAAAUUGCUUCUGGGGGCGCACCUCUUUCGAAAGAAGUCGGGGAAGCGGUUGC
AAAACGCUUCCAUCUUCCAGGGAUACGACAAGGAUAUGGGCUCACUGAGACUACAUCAGCUAUUCUGAUUAC
ACCCGAGGGGGAUGAUAAACCGGGCGCGGUCGGUAAAGUUGUUCCAUUUUUUGAAGCGAAGGUUGUGGAUC
UGGAUACCGGGAAAACGCUGGGCGUUAAUCAGAGAGGCGAAUUAUGUGUCAGAGGACCUAUGAUUAUGUCC
GGUUAUGUAAACAAUCCGGAAGCGACCAACGCCUUGAUUGACAAGGAUGGAUGGCUACAUUCUGGAGACAUA
GCUUACUGGGACGAAGACGAACACUUCUUCAUAGUUGACCGCUUGAAGUCUUUAAUUAAAUACAAAGGAUAU
CAGGUGGCCCCCGCUGAAUUGGAAUCGAUAUUGUUACAACACCCCAACAUCUUCGACGCGGGCGUGGCAGGU
CUUCCCGACGAUGACGCCGGUGAACUUCCCGCCGCCGUUGUUGUUUUGGAGCACGGAAAGACGAUGACGGAA
 
AAAGAGAUCGUGGAUUACGUCGCCAGUCAAGUAACAACCGCGAAAAAGUUGCGCGGAGGAGUUGUGUUUGU
GGACGAAGUACCGAAAGGUCUUACCGGAAAACUCGACGCAAGAAAAAUCAGAGAGAUCCUCAUAAAGGCCAAG
AAGGGCGGAAAGUCCAAACUCGAGUAAGGUUAACCUGCAGGAGG
Primer sequence: TTCCAATTCAGCGGGGGCCACC (SEQ ID NO: 54)

### Example 2: Intron-RT generates cDNA with large multiple repeats of circRNA of different sizes

CircRNAs having a range of sizes: (A) 1.3 kb (SEQ ID NO: 52) (B) 0.8 kb (SEQ ID NO: 55) and (C) 0.4 kb (SEQ ID NO: 56) were reverse transcribed by rolling circle reverse transcription using the primer (SEQ ID NO: 51) (FIG. 3A-FIG.3C). The sequences for each circRNA are shown below. The reaction for Intron-RT was carried out under the following condition: 2 µl of Intron-RT buffer (5X), 1 µl of dNTP (10 mM of each), 1 µl of circRNA specific primer (10 µM, sequence shown below), 1 µg of circRNA and 100 ng of Intron-RT. The reaction was incubated at 55°C for 1 hour.
circRNA (0.8 kb) (SEQ ID NO: 55) 
AAAAUCCGUUGACCUUAAACGGUCGUGUGGGUUCAAGUCCCUCCACCCCCACGCCGGAAACGCAAUAGCCGAA
AAACAAAAAACAAAAAAACCCCCCUCUCCCUCCCCCCCUAACGUUACUGGCCGAAGCCGCUUGGAAUAAGGCCG
GUGUGCGUUUGUCUAUAUGUUAUUUUCCACCAUAUUGCCGUCUUUUGGCAAUGUGAGGGCCCGGAAACCUG
GCCCUGUCUUCUUGACGAGCAUUCCUAGGGGUCUUUCCCCUCUCGCCAAAGGAAUGCAAGGUCUGUUGAAUG
UCGUUGGAGGUGCUCAAAGAGAUGGAAGCCAAUGCCCGGAAAGCUGGCUGCACCAGGGGCUGUCUGAUCUGC
CUGUCCCACAUCAAGUGCACGCCCAAGAUGAAGAAGUUCAUCCCAGGACGCUGCCACACCUACGAAGGCGACAA
AGAGUCCGCACAGGGCGGCAUAGGCGAGGCGAUCGUCGACAUUCCUGAGAUUCCUGGGUUCAAGGACUUGGA
GCCCAUGGAGCAGUUCAUCGCACAGGUCGAUCUGUGUGUGGACUGCACAACUGGCUGCCUCAAAGGGCUUGC
CAACGUGCAGUGUUCUGACCUGCUCAAGAAGUGGCUGCCGCAACGCUGUGCGACCUUUGCCAGCAAGAUCCA
GGGCCAGGUGGACAAGAUCAAGGGGGCCGGUGGUGACUAAAAAAAACAAAAAACAAAACGGCUAUUAUGCGU
UACCGGCGAGACGCUACGGACUU
circRNA (0.4 kb) (SEQ ID NO: 56) 
AAAAUCCGUUGACCUUAAACGGUCGUGUGGGUUCAAGUCCCUCCACCCCCACGCCGGAAACGCAAUAGCCGAA
AAACAAAAAACAAAAAACCCCCUCUCCCUCCCCCCCUAACGUUACUGGCCGAAGCCGCUUGGAAUAAGGCCG
GUGUGCGUUUGUCUAUAUGUUAUUUUCCACCAUAUUGCCGUCUUUUGGCAAUGUGAGGGCCCGGAAACCUG
GCCCUGUCUUCUUGACGAGCAUUCCUAGGGGUCUUUCCCCUCUCGCCAAAGGAAUGCAAGGUCUGUUGAAUG
UCGUAAAAAAACAAAAAACAAAACGGCUAUUAUGCGUUACCGGCGAGACGCUACGGACUU
Primer sequence: (SEQ ID NO: 51)

### Example 3: Intron-RTs show higher sensitivity than retroviral RTs (ProtoScript II (PSII) and SuperScript IV (SSIV)) in detecting an in vitro synthesized circRNA or specific circRNAs in human brain RNA via RT-qPCR

An *in vitro* synthesized circRNA (1. 3 kb) (SEQ ID NO: 52) with various input (0.001 pg (1), 0.01 pg (2), 0.1 pg (3), 1 pg (4), 10 pg (5), 100 pg (6) and 1 ng (7) was reverse transcribed with retroviral RTs (PSII, SSIV), and an Intron-RT (Induro) using a circRNA specific primer (SEQ ID NO: 51). The RT reactions for PSII and SSIV were carried out according to manufacturers' protocols. For the Intron-RT, 2 µl input RNA was incubated with 2 µl 10 mM of the specific primer in 25 µl total volume at 65°C for 5 minutes then cooled down to 4°C, 8 µl 5X Induro Buffer, 2 µl 10 mM dNTPs, 1 µl RNase inhibitor (New England Biolabs, Ipswich, MA) and 200 ng Intron-RT were added to the reactions and incubated for 1 hour at 55°C. qPCR was performed with Luna^{®} Universal qPCR Master Mix (New England Biolabs, Ipswich, MA) according to manufacturer's protocol. The results of the qPCR are shown in FIG. 5A and FIG. 5B as Ct values on y-axis.

### Human brain RNA

1 µg of total human brain RNA was reverse transcribed using a random hexamers primer. PSII and SSIV reactions were carried out according to manufacturers' recommendations. For Intron-RT, 1µg total human brain RNA was incubated with 4 µl of 60 µM random hexamer and oligo dT mix in 25 µl total volume at 65°C for 5 minutes. Then the reaction was cooled down to 4 °C with a 0.1 °C/ sec ramp. 8 µl 5X Induro buffer, 2 µl 10 mM dNTPs, 1 µl RNase inhibitor (New England Biolabs, Ipswich, MA) and 200 ng Intron-RT were added to the reactions and incubated for 10 minutes at 23°C and 5 minutes at 30°C for initial extension of the hexamers, then 1 hour at 55°C.

qPCR was performed with Luna Universal qPCR Master Mix according to manufacturer's protocol with the primers listed below to detect endogenous circRNAs from human brain (ZNF609, RIMS2, TULP4, XPO1, HIPK3), The results are shown in FIG. 5C and FIG. 5D, together with two linear RNAs ACTIN for normalization and GAPDH as a linear RNA comparison.
ZNF609 Forward primer (Circ) - CAGCGCTCAATCCTTTGGGA (SEQ ID NO: 57)
ZNF609 Reverse primer (Circ) - GACCTGCCACATTGGGTCAGTA (SEQ ID NO:58)
RIMS2 Forward primer (Circ) - TCTGTCACGGAAAAGTCGCA (SEQ ID NO: 59)
RIMS2 Reverse primer (Circ) - TGATCCGGCTACCTGTTTGT (SEQ ID NO:60 )
TULP4 Forward primer (Circ) - GGAGTGGTTGGGGTGACTTT (SEQ ID NO: 61)
TULP4 Reverse primer (Circ) - TCAACTGCCATACGAAGCGT (SEQ ID NO:62 )
HIPK3 Forward primer (Circ) - TATGTTGGTGGATCCTGTTCGG (SEQ ID NO:63 )
HIPK3 Reverse primer (Circ) - GACCAAGACTTGTGAGGCCATA (SEQ ID NO: 64)
XPO1 Forward primer (Circ) - GGAAAATGTGATAAAAACAAGGTGG (SEQ ID NO: 65)
XPO1 Reverse primer (Circ) - GACTCTTGTCCAAGCATCAGGA (SEQ ID NO: 66)
ACTIN Forward primer (Lin) - TCCCTGGAGAAGAGCTACG (SEQ ID NO: 67)
ACTIN Reverse primer (Lin) - GTAGTTTCGTGGATGCCACA (SEQ ID NO:68)
GAPDH Forward primer (Lin) - AAGGTGAAGGTCGGAGTCAAC (SEQ ID NO: 69)
GAPDH Reverse primer (Lin) - GGGGTCATTTGATGGCAACAATA (SEQ ID NO: 70)

### Example 4: Enrichment of circRNAs by degrading contaminating linear RNA with the combination of a 5' to 3' and a 3' to 5' RNase in total RNA

To test the effectiveness of two exoribonuclease for depletion of linear RNA, the following four reactions were set up: 1) Mock without any treatment; 2) RNase R only; 3) Poly A polymerase + RNase R; and 4) Poly A polymerase + RNase R + XRN-1. Linear RNA with A-tailing by Poly A polymerase has been shown to improve the linear RNA degradation by RNase R (Xiao et al, Nucleic Acids Research, (2019) 47, 8755-8769).

| 1: Mock | 2: RNase R | 3: Poly A pol + RNase R | 4: Poly A pol + RNase R + XRN-1 |
|---|---|---|---|
| 20 ul reaction: | 20 ul reaction: | 20 ul reaction: | 10 ul reaction: |
| 2 ug of total RNA | 2 ug of total RNA | 2 ug of total RNA | 2 ug of total RNA |
| 2 ul Poly(A) Buffer | 2 ul Poly(A) Buffer | 2 ul Poly(A) Buffer | 1 ul MMLV Buffer |
| 1 ul 10 mM ATP | 1 ul 10 mM ATP | 1 ul 10 mM ATP | 1 ul 10 mM ATP |
| | | 5 units Poly(A) polymerase | 5 units Poly(A) polymerase |
| | | | |
| 37°C for 30 minutes | 37°C for 30 minutes | 37°C for 30 minutes | 37°C for 30 minutes |
| | | | Add: |
| | | | 10 ul Adjust Buffer |
| | | | 0.5 ul RNase R |
| | | | 79.5 ul H₂O |
| | | | |
| | | | 37°C for 15 minutes |
| RNA cleanup and elute in 89.5 ul H₂O | RNA cleanup and elute in 89.5 ul H₂O | RNA cleanup and elute in 89.5 ul H₂O | RNA cleanup and elute in 14 ul H₂O |
| Add: | Add: | Add: | Add: |
| 10 ul RNase R Buffer | 10 ul RNase R Buffer | 10 ul RNase R Buffer | 1 ul mRNA decapping enzyme (MDE) |
| | 0.5 ul RNase R | 0.5 ul RNase R | |
| | | | 1 ul T4 PNK |
| | | | 2 ul MDE Buffer |
| | | | 2 ul ATP (10 mM) |
| | | | |
| 37°C for 15 minutes | 37°C for 15 minutes | 37°C for 15 minutes | 37°C for 15 minutes |
| | | | Add: |
| | | | 3 ul NEBuffer 3.1 |
| | | | 2 ul XRN-1 |
| | | | 20 ul H₂O |
| | | | |
| | | | 37°C for 15 minutes |
| RNA cleanup and elute in 10ul H₂O | RNA cleanup and elute in 10ul H₂O | RNA cleanup and elute in 10ul H₂O | RNA cleanup and elute in 10ul H₂O |

Ihe reagents were obtained as follows: RNase R (Lucigen, Middleton, WI); Poly(A) polymerase, MMLV buffer; RNA cleanup: Monarch^{®} RNA Cleanup Kit; mRNA Decapping enzyme (MDE); T4 Polynucleotide Kinase (T4 PNK); XRN-1; NEBuffer 3.1 (New England Biolabs, Ipswich, MA).
Adjust Buffer:100 mM Tris-Cl, pH 8.0, 750 mM KCl.

### RT-qPCR

Reverse transcription was performed in the following 20 µl reaction: 4 µl of 5x PSII reaction buffer, 2 µl of 100 mM DTT, 1 µl of 10 mM dNTP, 1 µl of 60 µM RT primer (Reverse Primer), 1 µl RNase Inhibitor (New England Biolabs, Ipswich, MA), 200 units of PSII and 10 µl of eluted RNA from the enrichment procedure described above. The reaction was incubated at 25°C for 10 minutes, 42°C for 1 hour and 65°C for 20 minutes.

qPCR was performed with Luna Universal qPCR Master Mix according to manufacturer's protocol. The results are shown in FIG. 6B.

The primers for qPCR used to analyze the cDNA resulting from reverse transcription of circRNA were as following:

| **Gene** | **Forward Primers (5' to 3')** | **Reverse Primers (5' to 3')** |
|---|---|---|
| GAPDH | GGTGGTCTCCTCTGACTTCAACA (SEQ ID NO: 71) | GTTGCTGTAGCCAAATTCGTTGT (SEQ ID NO: 72) |
| U1 | CCACAAATTATGCAGTCAAGTTTCCCA (SEQ ID NO: 73) | CCATGATCACGAAGGTGGTTTTCC (SEQ ID NO: 74) |
| RPPH1 | CTAACAGGGCTCTCCCTGAGC (SEQ ID NO: 75) | GTTCCAAGCTCCGGCAAAGG (SEQ ID NO: 76) |
| SH3BP5 | TGAAGCAGCTCTCCCTACAGTG (SEQ ID NO: 77) | TGGCACAATGTTCTCCAGTTCC (SEQ ID NO: 78) |

### Illumina Sequencing

Illumina libraries were constructed with NEBNext^{®} Ultra^{™} II Directional RNA Library Prep Kit for Illumina^{®} (New England Biolabs, Ipswich, MA) according to the manufactory's protocol. The data was analyzed using CIRI2 (Gao, et al, Briefings in Bioinformatics, 2018, 19(5):803-810)(FIG. 6C). Using poly A -RNaseR and XRN-1, 10,440 unique circRNAs were identified from total brain RNA.

### Example 5: Consensus sequence generated by multi-repeat cDNA from rolling circle reverse transcription with Intron-RT enhances accuracy of nanopore sequencing result

Rolling circle reverse transcription was performed in an aqueous reaction mixture containing: 360 ng of purified circRNA template (1.8 kb, sequence shown below), 1x Induro Buffer, 1 mM DNTPs, 1 µM RT primer, 10 mM DTT, 100 ng Induro RT (an Intron-RT) and 20 units Murine RNase Inhibitor in a final volume of 20 µL. Reactions were incubated at 50°C for 30 minutes. The cDNA reaction product was purified using NEBNext^{®} Sample Purification Beads (New England Biolabs, Ipswich, MA) with a 1:1 volume ratio, following the manufacturer's directions. 500 ng of cDNA was resuspended in 50 mM NaCl and the EP oligo was added to a final concentration of 1 µM, in a final volume of 20 µL. The oligos were annealed to the cDNA by heating to 60°C for 5 minutes to create a double stranded cDNA end with an A overhang suitable for the Oxford Nanopore Ligation Sequencing Kit (Oxford Nanopore Technologies, Oxford, UK). A ligation reaction was then performed by adding 20 µL of the Oxford Nanopore sequencing adapter as well as 50 µL of Blunt/TA ligation master mix (New England Biolabs, Ipswich, MA) in a final volume of 100 µL and reacted for 30 minutes at room temperature. Reactions were then purified and prepared for sequencing following the Oxford Nanopore Ligation Sequencing protocol.
RT Primer:
   /5Phos/ACTTGCCTGTCGCTCTATCTTCTTTGTTTTTTCCCTGCAGGTTAACCTTACTC (SEQ ID NO: 79)
EP oligo:
   CCTGCAGGGAAAAAACAAAGAAGATAGAGCGACAGGCAAGTA (SEQ ID NO: 80)
circRNA (~1.8kb) (SEQ ID NO: 81) 
GGAUUCUAAAACGGAUUACCAGGGAUUUCAGUCGAUGUACACGUUCGUCACAUCUCAUCUACCUCCCGGUUU
UAAUGAAUACGAUUUUGUACCAGAGUCCUUUGAUCGUGACAAAACAAUUGCACUGAUAAUGAAUUCCUCUGG
AUCUACUGGGUUACCUAAGGGUGUGGCCCUUCCGCAUAGAACUGCCUGCGUCAGAUUCUCGCAUGCCAGAGA
UCCUAUUUUUGGCAAUCAAAUCAUUCCGGAUACUGCGAUUUUAAGUGUUGUUCCAUUCCAUCACGGUUUUG
GAAUGUUUACUACACUCGGAUAUUUGAUAUGUGGAUUUCGAGUCGUCUUAAUGUAUAGAUUUGAAGAAGA
GCUGUUUUUACGAUCCCUUCAGGAUUACAAAAUUCAAAGUGCGUUGCUAGUACCAACCCUAUUUUCAUUCUU
CGCCAAAAGCACUCUGAUUGACAAAUACGAUUUAUCUAAUUUACACGAAAUUGCUUCUGGGGGCGCACCUCU
UUCGAAAGAAGUCGGGGAAGCGGUUGCAAAACGCUUCCAUCUUCCAGGGAUACGACAAGGAUAUGGGCUCAC
UGAGACUACAUCAGCUAUUCUGAUUACACCCGAGGGGGAUGAUAAACCGGGCGCGGUCGGUAAAGUUGUUCC
AUUUUUUGAAGCGAAGGUUGUGGAUCUGGAUACCGGGAAAACGCUGGGCGUUAAUCAGAGAGGCGAAUUAU
GUGUCAGAGGACCUAUGAUUAUGUCCGGUUAUGUAAACAAUCCGGAAGCGACCAACGCCUUGAUUGACAAGG
AUGGAUGGCUACAUUCUGGAGACAUAGCUUACUGGGACGAAGACGAACACUUCUUCAUAGUUGACCGCUUGA
AGUCUUUAAUUAAAUACAAAGGAUAUCAGGUGGCCCCCGCUGAAUUGGAAUCGAUAUUGUUACAACACCCCA
ACAUCUUCGACGCGGGCGUGGCAGGUCUUCCCGACGAUGACGCCGGUGAACUUCCCGCCGCCGUUGUUGUUU
UGGAGCACGGAAAGACGAUGACGGAAAAAGAGAUCGUGGAUUACGUCGCCAGUCAAGUAACAACCGCGAAAA
AGUUGCGCGGAGGAGUUGUGUUUGUGGACGAAGUACCGAAAGGUCUUACCGGAAAACUCGACGCAAGAAAAA
UCAGAGAGAUCCUCAUAAAGGCCAAGAAGGGCGGAAAGUCCAAACUCGAGUAAGGUUAACCUGCAGGGAAAA
AACAAAAAACAAAACGGCUAUUAUGCGUUACCGGCGAGACGCUACGGACUU
Primer sequence:
   ACTTGCCTGTCGCTCTATCTTTGTTTTTTCCCTGCAGGTTAACCTTACTC (SEQ ID NO: 82)

In FIG. 7E, FIG. 7F, and FIG. 7G, the reaction condition was the same as in FIG. 7A- FIG. 7D except that the template is 5 pmol of circRNA (42 nt). The results in FIG. 7B show a peak size of 7.2 kb corresponding to a median repeat number of 4 units/ concatemer cDNA. The accuracy of consensus sequence increases as the copy number of repeat within the concatemeric cDNA increases. Consensus sequence from cDNA with 4 copies of repeats can generate 97% accuracy when compared with the reference sequence (FIG. 7D). FIG. 7F shows a median length that represents a median copy number of greater than 100 (FIG. 7F) with greater than 90% percent of the reads identical to the reference sequence (FIG. 7G).

### Example 6. Preparation of a circRNA library for sequencing

Randomized primer (N6) were first annealed to the circRNA. In this example, PSII was used to elongate the N6 primer (FIG. 8A). In this example, elongation of primer facilitated stability of the elongated primer hybridization prior to raising the temperature for reverse transcription by Intron-RT. In other examples however, it was found that primer extension was not necessary or advantageous.

Since the PSII has weak strand displacement activity, the elongation of the primer stops when the strand reaches another annealed primer. The Intron-RT subsequently added to the reaction continued reverse transcription in a rolling circle to produce concatemers due to the strong strand displacement activity of the Intron-RT. If the amount of the multiple-repeat cDNA was relatively low due to low input of starting RNA, Phi29 (New England Biolabs, Ipswich, MA) was used to amplify the cDNA followed by T7 Endonuclease (New England Biolabs, Ipswich, MA) to debranch the product. The Phi29 and T7 Endonuclease reactions were performed according to the manufacturer's instructions. Library preparation was then carried out according to sequencing platform of choice using the standard protocols described by New England Biolabs, Ipswich, MA (see for example, New England Biolabs product E7805S for Illumina sequencing platform and New England Biolabs product E7180S for Oxford Nanopore Sequencing Platform). An example of a workflow for the above is shown in FIG. 8A.

### Optional circRNA enrichment

CircRNA in human brain RNA (1 µg) could be enriched using the circRNA enrichment steps described above including rRNA depletion and linear RNA depletion. The rRNA depletion was performed using NEBNext^{®} rRNA Depletion Kit v2 (Human/Mouse/Rat) (New England Biolabs, Ipswich, MA). The linear RNA depletion was performed according to the method above. As an alternative, circRNA in human brain could be size separated after rolling circle reverse transcription as described below to obviate the need for depletion of unwanted RNA prior to reverse transcription.

### Optional Hexamer elongation by PSII

Human brain RNA (1 µg) or the enriched circRNA was reversed transcribed with PSII to elongate the randomized Hexamer (N6) primer. The final reaction condition for 20 µl is: 4 µl of 5x PSII reaction buffer, 2 µl of 100 mM DTT, 1 µl of 10 mM dNTP (N0447S), 1 µl of 10 µM N6 primer, 200 units of PSII and human brain RNA. The reaction was incubated at 25°C for 10 minutes.

### Rolling circle reverse transcription

If PSII was used to extend the N6 and rolling circle reverse transcription was performed by adding the 20 µl of the following components to the reaction: 8 µl of 5 X Induro buffer, 200 ng of Intron-RT (Induro), 2 µl 10 mM dNTP and 8 µl H₂O. The reaction was incubated at 55°C for 40 minutes.

If PSII was not used to extend the N6, rolling circle reverse transcription was performed as following in a 20 µl reaction: 4 µl of 5 X Group II Intron-RT buffer, 100 ng of Intron-RT, 1 µl 10 mM dNTP and human brain RNA. The reaction was incubated at 25°C for 10 minutes followed by 55°C for 40 minutes.

### Size selection of cDNA product

Size selection was carried out with beads resuspension buffer MgCl500-PEG5 according to the paper (Stortchevoi, et al, Journal of Biomolecular techniques, 31: 7-10). The product was eluted in 10 µl water.

### DNA amplification with Phi29

The DNA amplification with Phi29 was carried out with the following conditions in a 50 µl reaction: 5 µl of 5X Phi29 DNA polymerase reaction buffer, 5 µl of 10 mM dNTP, 2.5 µl 1 mM phosphorothioate N6, 1 µl of product from rolling circle reverse transcription and 20 units of Phi29 DNA polymerase. The reaction was incubated overnight at 30°C. Then 20 µl of T7 Endonuclease I was added to the reaction to debranch the DNA. The effect of debranching with an endonuclease on the final cDNA product is shown in FIG. 8B.

### Nanopore library preparation and sequencing

The Nanopore library was prepared according to the Ligation Sequencing Kit and the sequencing was performed with Spot-ON^{®} Flow Cell, R9 version on GridION^{®} machine (Oxford Nanopore Technologies, Oxford, UK) according to the manufacturer's protocols. The N50 (median) length and distribution of lengths of cDNA obtained from circRNA in human brain is shown in FIG. 8C.

### EXAMPLE 7: Cellular circRNA profiling from total human brain RNA without ribodepletion or linear RNA depletion with RNAse pretreatment

The experimental protocols described here are similar to Example 6, except that the input RNA here is total human brain RNA without circRNA enrichment. This example demonstrated that, due to the size difference, the concatemeric cDNA derived from rolling circle reverse transcription of circRNA could be readily separated from non-concatemeric cDNA derived from linear RNA. As shown in Fig 9, in the final sequencing results, a very low percentage of reads of rRNA (<1%) were detected, even though rRNA constitutes more than 80% of the starting total brain RNA. This result suggested that the Intron-RT mediated rolling circle reverse transcription can be applied directly to total RNA without rRNA/linear RNA depletion.

### Example 8: Intron-RT mediated rolling circle reverse transcription enables high fidelity nanopore sequencing of small RNA

Small RNAs (sRNAs) are an important group of non-coding RNAs that have great potential as diagnostic and prognostic biomarkers for treatment of a wide variety of diseases. The portability and affordability of nanopore sequencing technology makes it ideal for point of care and low resource settings. Currently sRNAs cannot be reliably sequenced on the nanopore platform due to the short size of sRNAs and high error rate of the nanopore sequencer.

A highly efficient nanopore-based sequencing strategy for sRNAs is described here (see FIG. 7E) in which sRNAs are ligated to an RNA top strand adapter and bottom strand splint DNA and circularized by means of an RNA ligase. The circularized sRNA then undergoes rolling circle reverse transcription with an Intron-RT to generate concatemeric cDNA. After sequencing, the resulting tandem repeat sequences within the individual cDNA were aligned to generate highly accurate consensus sequences (see for example, the workflow in FIG. 7E, results in FIG. 7F-FIG. 7H).

For synthetic RNA oligos (sequence shown below), 5 pmol of RNA was used as input and the protocol starts at the circularization step. For human brain RNA, sRNAs (< 200 base pairs) were isolated from 10 µg of total human brain RNA, sRNAs (< 200 base pairs) were isolated from total RNA using RNA XP beads (Beckman Coulter, Indianapolis, IN) following the manufacturer's protocol. 50 ng of the isolated sRNA was used as input for a ligation reaction that would circularize the sRNA.

The RNA was first denatured by heating to 70°C for 2 minutes and placed on ice immediately. The ligation mixture was then added to the RNA. The ligation mixture contained 1X T4 RNA Ligase Buffer (New England Biolabs, Ipswich, MA), 20% PEG-8000, 0.05% Tween-20, 20 pmol of the annealed DNA splint (bottom strand) RNA adapter (top strand) (see below) and 200 units of T4 RNA Ligase 2 Truncated K/Q (New England Biolabs, Ipswich, MA) in a total volume of 20 µl. The ligation reaction was incubated at 25°C for 1 hour. Subsequently, 2 units of USER^{®} Enzyme (New England Biolabs, Ipswich, MA) and 4 units of DNase I (New England Biolabs, Ipswich, MA) were added to the reaction mixture and incubated at 37°C for 30 minutes to remove the bottom DNA splint strand of the adapter. The T4 RNA Ligase 2 Truncated K/Q was then heat inactivated at 75°C for 5 minutes and then cooled down to 4°C. The reaction mixture was then diluted to a total volume of 40 µL with 1X T4 RNA Ligase Buffer, 1 mM ATP, 10 units of T4 Polynucleotide Kinase and 30 units of T4 RNA Ligase 1 (New England Biolabs, Ipswich, MA). The circularization reaction was then allowed to proceed for 1 hour at 25°C. Linear RNAs were then degraded by adding the following mixture of enzymes: 2 µL XRN-1, 1 µL 5'deadenylase (New England Biolabs, Ipswich, MA), RNase R (purified in house), ATP and Poly(A) Polymerase (New England Biolabs, Ipswich, MA). The reaction mixture was then diluted to 80 µL with 2X Intron-RT buffer (1X final concentration), 1 µM primer (supplemental table 1, oligo 2), 1 mM dNTPs and 100 ng of Induro RT. The reaction was incubated for 38°C for 5 minutes, 60°C for 30 minutes and then 95°C for 5 minutes. The resulting cDNA was purified using 96 µl of NEBNext Sample Purification Beads, following the manufacturer's directions, with a modified elution protocol. The elution was incubated at 37°C for 10 minutes with occasional vortexing.

Second strand synthesis was performed using Taq DNA polymerase (New England Biolabs, Ipswich, MA). A primer (supplemental table 1, oligo 5) was annealed to the adapter sequence and the 5'-3' exonuclease activity of the polymerase could remove primers annealed to the internal sequence of cDNA. The reaction mixture contained 1 µg of the rolling circle cDNA product, 1X ThermoPol^{®} Buffer (New England Biolabs, Ipswich, MA), 1 mM dNTPs, 10 pmol primer and 5 units of Taq DNA Polymerase in a total volume of 50 µL. The reaction was incubated for 95°C for 30 seconds, 62°C for 1 minute and 65°C for 20 minutes. These reactions were purified using 25 µl of NEBNext Sample Purification Beads. The Nanopore Sequencing was performed with the purified DNA.

Reads were filtered by length (> 1000 bp) and average quality (>= 7) and then converted to FASTA format. SPADE (23) was used to detect periodic repeats in the reads and to extract consensus sequences. Iterative testing was performed to find optimal parameter tuning and the final parameters were used as follows: K-mer size = 5, sliding window size = 1000, peak height threshold = 10, gap threshold = 200, margin = 200, letter consistency threshold = 0.5. All other parameters were used with their defaults. Custom R scripts (R Core Team, version 3.6.3. https://www.R-project.org/) were used to parse the resulting GenBank files from the SPADE output to collect the consensus sequences. Consensus sequences generated in this manner could have any random circular orientation, therefore, to phase them we generated all possible rotations of each consensus sequence and aligned the adapter to them using pairwise alignments with the Needleman-Wunsch algorithm as implemented in the R package Biostrings (R package, version 2.62.0. https://bioconductor.org/packages/Biostrings.) We chose the first rotation of the sequence that gave the longest un-gapped alignment anchored to either the start or end of the read. The adapter sequence was then trimmed from the rotated consensus sequences to yield the final trimmed consensus sequences.
Synthetic RNA:
   oligo:/5phos/rUrGrArGrGrUrArGrUrArGrGrUrUrGrUrArUrArGrUrUrUrArCrUrGrArCrCrArGrGrArCrGrArCrGr ArCrA (SEQ ID NO: 83)
RNA adapter:
   /5Phos/rUrArCrUrGrArCrCrArGrGrArCrGrArCrGrArCrA/3Phos/ (SEQ ID NO: 84)
Bottom DNA splint strand:
   UGUCGUCGUCCUGGUCAGUANNNNNN/3InvDT/ (SEQ ID NO: 85)
RT Primer:
   TGTCGTCGTCCTGGTCAGTA (SEQ ID NO: 86)
Second Strand Synthesis Primer:
   TACTGACCAGGACGACGACA (SEQ ID NO: 87)

The results are shown in FIG. 7F - FIG. 7H. FIG. 7F and FIG. 7G are results from synthetic RNA with known sequence to evaluate the number of repeats in the cDNA and the accuracy of the consensus sequence. Intron-RT mediated rolling circle reverse transcription produces concatemeric cDNA with multiple repeats (>100 copies) of the synthetic small RNA sequence (FIG. 7F). The consensus sequence can produce highly accurate small RNA sequence when compared to the reference sequence (FIG. 7G). FIG. 7H is results of small RNAs isolated from total brain RNA. The Intron-RT mediated method (SR-Cat) can generate similar number of miRNA species as the TruSeq^{®} workflow (Illumina, San Diego, CA), which is based on the short-read sequencing platform (FIG. 7H). This approach can be a rapid and accurate method of detecting and sequencing small RNAs from total RNA obtained from cells, tissues and/or body fluid from microorganisms, plants or animals.

### Example 9: Cell free circRNA profiling from human serum/plasma

This example shows that Intron-RT mediated rolling circle reverse transcription allows for circRNA profiling from samples with very low RNA input, such as biofluids (urine, serum, saliva).

The workflow is shown in FIG. 10A. Total RNA was isolated from 1.5 ml frozen human serum using miRNeasy Serum/Plasma Advanced Kit (Qiagen, Hilden, Germany) according to manufacturer's protocol. The obtained RNA was treated with DNase I (New England Biolabs, Ipswich, MA) for 15 minutes at room temperature to remove any DNA contamination. Then RNA was purified using Monarch^{®} RNA Purification Kit (New England Biolabs, Ipswich, MA) and eluted in 12 µl water. 10 µl from the purified serum RNA was mixed with 2 µl of 100 µM random hexamers (New England Biolabs, Ipswich, MA) in a total volume of 12 µl and incubated at 72°C for 5 minutes and cooled down to 4°C. Then, 8 µl of 5X Group II Intron-RT buffer, 200 ng of Group II Intron-RT, 2 µl 10 mM dNTPs and 16 µl molecular grade water were added to the reaction. For initial extension of the hexamers the reaction was incubated at 23°C for 10 minutes and 30°C for 5 minutes. The temperature then slowly (0.1°C/second) raised to 55°C and kept at 55°C for 1 hour. Finally, the Intron-RT was heat-inactivated at 95°C for 1 minute. The longer (10 kb+) concatemeric cDNAs were size-selected against cDNA from linear RNA with SPRI beads with MgCl500-PEG5 buffer according to the protocol described by Stortchevoi, et al, Journal of Biomolecular techniques, 31: 7-10. Size-selected cDNAs were then amplified using Phi29 and debranched as described in Example 6. Debranched DNA was sequenced on PacBio Sequel II instrument according to manufacturers' protocols. The reads were analyzed using was analyzed using isoCirc software published in Xin et al., Nat. Commun., 2021, 12(1):266. In the experiment shown in FIG. 10B, unique circRNA isoforms were detected in both healthy and cancerous human plasma. The length distribution of circRNA from Healthy Individual # 1 are shown in FIG. 10C. The median length of the circRNAs detected with our method was 483 bp.

### Example 10: High accuracy RNA sequencing with circularization and rolling circle reverse transcription and measuring RNA polymerase error rates

Circularization of RNA followed by rolling-circle reverse transcription achieved highly accurate RNA sequencing for identifying sequence heterogeneity, including single-nucleotide polymorphisms (SNP), and was used for measuring the error rates of RNA polymerases (see FIG. 12A and FIG. 12B).

The error rate of *in vitro* transcription of a synthetic template by T7 RNA polymerase was measured. The RNA template was either circularized by means of an insert sequence, flanked by permutated Intron-exon splicing elements, plus homology arms, for circularization, as described in: Wesselhoeft et al., Nature communications 1-10 (2018) doi:10.1038/s41467-018-05096-6, or by ligation (as described in Example 8, or Petkovic et al., 2015, Nucleic Acids Research, Volume 43, 2454-2465).

After circularization of the transcribed RNA, rolling circle cDNA synthesis was performed by Intron-RT, producing long concatemeric cDNA. All reagents are from New England Biolabs, Ipswich, MA. The cDNA synthesis reaction contained: 1 µg of circularized RNA, 1X RT Buffer, 200 nM specific primer, 100 ng Intron-RT and 1 mM each dNTP and incubated at 44°C for 1 minute. The reaction was stopped by the addition of 1 µl Thermolabile Proteinase K and incubated for 15 minutes at 25°C, 15 minutes at 37°C, and 1 minute at 95°C, followed by the addition of 1 µl RNaseA (diluted 1/10 in TE) and incubation for 15 minutes at 37°C. The single-stranded cDNA was purified by ethanol precipitation. Next, the concatemeric cDNA was replicated and made double-stranded by A-tailing and annealing an oligo-dT primer to the newly-formed poly-dA sequence at the 3' end. The oligo-dT can be extended by a DNA polymerase, such as Phi29 DNA polymerase or Taq DNA polymerase following standard protocols for primer extension. The double-stranded DNA product was then made into sequenceable libraries for long-read DNA sequencing, such as Pacific Biosciences Single-Molecule Real-Time (SMRT) sequencing using commercially available template preparation kits. The DNA libraries were sequenced on a Pacific Biosciences sequencer (Sequel II), to generate HiFi reads (high accuracy sequencing reads) of the concatemeric double-stranded cDNA. A consensus sequence was generated by aligning the sequences of all individual monomers from a single concatemer sequence. This consensus sequence of an individual monomer was compared to the template RNA sequence to determine RNA polymerase errors. Example error rates for T7 RNA polymerase using this method are provided below.

| **RNA Polymerase** | **Amplicon** | **Substitution Error Rate (per base)** |
|---|---|---|
| T7 RNA polymerase | DNA1 | 3.69E-05 |
| | DNA3 | 3.65E-05 |
| T7 RNA polymerase | DNA1 | 3.63E-05 |
| | DNA3 | 3.58E-05 |
| T7 RNA polymerase | DNA1 | 3.81E-05 |
| T7 RNA polymerase | DNA1 | 3.92E-05 |

Mutational spectrum of T7 RNA polymerase (average across all samples):

| rA-rC | rA-rU | rA-rG | rU-rA | rU-rC | rU-rG | rC-rA | rC-rU | rC-rG | rG-rA | rG-rC | rG-rU |
|---|---|---|---|---|---|---|---|---|---|---|---|
| 0.92% | 1.93% | 5.24% | 19.16% | 20.69% | 26.73% | 2.82% | 1.55% | 0.65% | 17.48% | 0.56% | 2.26% |

### Example 11: Measuring Intron-RT reverse transcriptase error rates

The error rate of Intron-RT reverse transcriptase was determined using the method described in Example 9. In this case, the HiFi sequencing reads of the cDNA concatemers were analyzed by comparing the high-accuracy CCS (circular consensus sequencing) reads of the full length cDNA concatemer to the high-accuracy consensus sequence of the individual RNA monomers (which corresponded to the sequence of the RNA strand that acted as a template for reverse transcription) to determine any errors made by the reverse transcriptase. The error rate of Intron-RT was found to be:

| **Reverse Transcriptase** | **Amplicon** | **Substitution Error Rate (per base)** |
|---|---|---|
| Intron-RT | DNA1 | 5.2E-05 |
| | DNA3 | 6.0E-05 |
| Intron-RT | DNA1 | 5.3E-05 |
| | DNA3 | 5.7E-05 |
| Intron-RT | DNA1 | 6.6E-05 |
| Intron-RT | DNA1 | 7.2E-05 |

### Example 12: Intron-RT can synthesize a CDNA from linear RNA having a size greater than 12 kb in 10 minutes

Using a 10 minute incubation at 50°C, 55°C or 60°C of total human RNAs - SHDA (1.9Kb), XRN (4.7Kb), HERCI (5.5Kb, SMG1 (9.3 kb), HERC1 (12.2Kb) and HERC1 (14.2Kb) were reverse transcribed with the Intron-RT-Induro. After first strand cDNA synthesis, an aliquot was amplified by PCR using LongAmp^{®} Taq 2X Mastermix (New England Biolabs, Ipswich, MA). It was found that at temperatures of 55°C-60°C, the Intron-RT (Induro) had a cDNA synthesis rate of about 2 kb/minute.

## Claims

1. A method for identifying a circular RNA (circRNA) in a sample by characterizing a first strand cDNA molecule, comprising:
(a) incubating a sample comprising a circRNA with a Group II Intron reverse transcriptase (Intron-RT) and dNTPs, to produce by rolling circle reverse transcription, a reaction product comprising concatemeric first strand cDNA molecules; and
(b) characterizing the cDNA by:
(i) obtaining a sequence of the concatemeric first strand cDNA molecules, wherein the sequence is a consensus complementary sequence of repeat units of the circRNA; optionally wherein the step of sequencing the cDNA is preceded by a step of detecting the cDNA by amplification;
or
(ii) amplifying the concatemeric first strand cDNA by a DNA amplification reaction by using primers.

2. The method of claim 1, wherein the circRNA is:
isolated from a sample containing eukaryotic cells or bodily fluid;
contained within a cell lysate or body fluid;
circularized linear RNA;
synthesized in vitro creating an artificial sequence; or
a circularized linear RNA that is a transcription product of a DNA.

3. The method of claim 1 or claim 2, wherein (a) further comprises:
enriching the circRNA in total RNA by degrading linear RNA with a 5'- 3' RNase and a 3'- 5' RNase;
combining DNA oligonucleotide or a ribozyme with the circRNA and Intron-RT, optionally wherein the oligonucleotide is a primer or an adapter;
and/or
forming the concatemeric cDNA at a temperature in the range of 20°C-60°C, such as wherein (a) comprises incubating the sample with the Intron-RT at a temperature in the range of 50°C-60°C so as to reduce the formation of RNA secondary structure.

4. The method of any of claims 1-3, wherein (b) further comprises:
amplifying the full length first strand cDNA concatemer using a randomly-primed amplification method, to produce an amplified concatemer; and/or
aligning multiple repeat sequences to obtain a consensus sequence.

5. The method of any preceding claim, wherein the concatemeric first strand has a length of at least 500 bases and contains at least 3 complementary copies of the circRNA.

6. The method of any preceding claim, wherein the sequencing of the concatemeric first strand cDNA in (i) is by long-read sequencing; or wherein the sequencing of the amplification product of the first strand cDNA in (i) is by long-read sequencing.

7. The method of any preceding claim, further comprising size separating the concatemeric first strand cDNA from non-concatemeric cDNA; optionally wherein step (a) does not include RNase treatment of the sample containing circRNA.

8. The method of any preceding claim, further comprising amplifying the cDNA with a DNA polymerase; optionally wherein the DNA polymerase is Phi29; optionally wherein the method further comprises treating the amplified first strand cDNA with a nuclease for removing branching.

9. The method of any preceding claim, wherein the amount of cDNA copies of the circular RNA within a concatemer provides at least 2 fold higher concentration of copies than can be obtained using a retroviral RT.

10. A method for assaying the transcription fidelity of an RNA polymerase, comprising:
(a) selecting a synthetic linear DNA;
(b) transcribing the DNA with a DNA dependent RNA polymerase in a reaction mixture;
(c) producing a circularized RNA from (b);
(d) reverse transcribing the circular RNA (circRNA) with a Group II Intron reverse transcriptase (Intron-RT) to form a population of concatemeric cDNA, and optionally amplifying the cDNA with a DNA polymerase; and
(e) sequencing the population of cDNA, optionally comprising long read DNA sequencing, to determine the transcription fidelity of the RNA polymerase.

11. The method of claim 10, further comprising determining the error rate of the RNA polymerase from the occurrence of errors in the consensus sequences in individual cDNAs.

12. A reaction mixture, comprising:
(a) a circular RNA (circRNA), wherein the circRNA is selected from: a eukaryotic circRNA, and a synthetic circRNA having an artificial sequence; and
b) a Group II bacterial or archaeal Intron reverse transcriptase (Intron-RT).

13. The reaction mixture of claim 12, wherein:
the circRNA is a eukaryotic circRNA; wherein the eukaryotic circRNA has been isolated from a cell or bodily fluid, or wherein the reaction mixture comprises a sample of a cell lysate or bodily fluid comprising the eukaryotic circRNA; or
the eukaryotic circRNA or the synthetic circRNA is a circularized linear RNA.

14. The reaction mixture of claim 12 or claim 13, wherein the circRNA has a size in the range of 20 bases-50 kilobases.

15. The reaction mixture of any of claims 12-14, further comprising a DNA oligonucleotide;
optionally wherein the DNA oligonucleotide is a primer or an adapter; such as wherein the oligonucleotide is a primer comprising: a 3' end having a target-specific complementary sequence for hybridizing to the circular RNA, or a degenerate sequence at the 3' end, and optionally a 5' tail.

16. The reaction mixture of any of claims 12-15, further comprising one or more enzymes selected from the group consisting of a 5'- 3' RNA exonuclease, a 3'- 5' RNA exonuclease, a DNA polymerase, a ligase, a thermolabile proteinase and an endonuclease;
optionally wherein the reaction mixture comprises a DNA polymerase, such as wherein the DNA polymerase is Phi29, Taq, Bst, Bst large fragment, Bsu, Bsu large fragment, *E.coli* Polymerase I, Klenow, Deep Vent, Vent, Pfu, KOD, Tgo or 9°N DNA polymerase.

17. The reaction mixture of any of claims 12-16, further comprising concatemeric first strand cDNAs, wherein each concatemeric cDNA contains multiple repeat units that are complementary copies of circRNA, and wherein the median length of the concatemeric cDNA is at least 3 times the length of the circRNA;
optionally wherein the cDNA in the reaction mix comprises at least 20 complementary copies of the circRNA, or wherein the cDNA contains at least 500 nucleotides; and/or wherein the repeat units in a single cDNA share more than 90% sequence identity.

18. A kit comprising:
a Group II Intron reverse transcriptase enzyme (Intron-RT); and
reagents for circularizing a linear RNA, selected from any of: a splint adapter, an RNA ligase such as T4 RNA ligase, a ribozyme, or chemicals capable of circularizing the RNA.

19. The kit of claim 18, further comprising at least one enzyme selected from the group consisting of: a DNA dependent RNA polymerase; a 5'- 3' RNA exonuclease; a 3'- 5' RNA exonuclease; a DNA polymerase; a ligase; a thermolabile proteinase; and an endonuclease;
optionally wherein one or more of the enzymes is in the same container as the Group II Intron reverse transcriptase (Intron-RT) or wherein one or more of the enzymes is in one or more different containers from the Group II Intron reverse transcriptase enzyme (Intron-RT).

20. The kit of claim 18 or claim 19, wherein at least one of the enzymes in the kit is lyophilized; optionally wherein the lyophilized enzyme is on the surface of a polymer, within a porous polymer matrix, or in a cake within a tube.

## Patentansprüche

1. Ein Verfahren zur Identifikation einer zirkulären RNA (circRNA) in einer Probe durch Charakterisieren eines Erststrang-cDNA-Moleküls, das Folgendes umfasst:
(a) Inkubieren einer Probe, die eine circRNA umfasst, mit einer Gruppe-II-Intron-Reverse-Transkriptase (Intron-RT) und dNTPs, um mittels Rolling-Circle-Reverse-Transkription ein Reaktionsprodukt zu produzieren, das konkatemere Erststrang-cDNA-Moleküle umfasst; und
(b) Charakterisieren der cDNA durch:
(i) Erhalten einer Sequenz der konkatemeren Erststrang-cDNA-Moleküle, wobei die Sequenz eine komplementäre Konsensussequenz von Repetiereinheiten der circRNA ist; wobei dem Schritt des Sequenzierens der cDNA optional ein Schritt des Detektierens der cDNA durch Amplifikation vorausgeht;
oder
(ii) Amplifizieren der konkatemeren Erststrang-cDNA durch eine DNA-Amplifikationsreaktion mittels Verwendung von Primern.

2. Verfahren nach Anspruch 1, wobei die circRNA:
aus einer Probe isoliert wird, die eukaryotische Zellen oder Körperflüssigkeit enthält;
innerhalb eines Zelllysats oder einer Körperflüssigkeit enthalten ist;
zirkularisierte lineare RNA ist;
in vitro synthetisiert wird, wodurch eine künstliche Sequenz entsteht; oder
eine zirkularisierte lineare RNA ist, die ein Transkriptionsprodukt einer DNA ist.

3. Verfahren nach Anspruch 1 oder Anspruch 2, wobei (a) ferner Folgendes umfasst:
Anreichern der circRNA in Gesamt-RNA durch Abbauen von linearer RNA mit einer 5'-3'-RNase und einer 3'-5'-RNase;
Kombinieren von DNA-Oligonukleotid oder einem Ribozym mit der circRNA und Intron-RT, wobei das Oligonukleotid optional ein Primer oder ein Adapter ist;
und/oder
Bilden der konkatemeren cDNA bei einer Temperatur im Bereich von 20°C-60°C, wie etwa wobei (a) das Inkubieren der Probe mit der Intron-RT bei einer Temperatur im Bereich von 50°C-60°C umfasst, um somit die Bildung von RNA Sekundärstruktur zu reduzieren.

4. Verfahren nach einem der Ansprüche 1-3, wobei (b) ferner Folgendes umfasst:
Amplifizieren des kompletten Erststrang-cDNA-Konkatemers unter Verwendung eines Zufallsprimer-Amplifikationsverfahrens, um ein amplifiziertes Konkatemer zu produzieren; und/oder
Alignieren mehrere Repetiersequenzen, um eine Konsensussequenz zu erhalten.

5. Verfahren nach einem der vorhergehenden Ansprüche, wobei der konkatemere Erststrang eine Länge von mindestens 500 Basen aufweist und mindestens 3 komplementäre Kopien der circRNA enthält.

6. Verfahren nach einem der vorhergehenden Ansprüche, wobei das Sequenzieren der konkatemeren Erststrang-cDNA in (i) mittels Long-Read-Sequenzierung erfolgt; oder wobei die Sequenzierung des Amplifikationsprodukts der Erststrang-cDNA in (i) mittels Long-Read-Sequenzierung erfolgt.

7. Verfahren nach einem der vorhergehenden Ansprüche, das ferner das Trennen der konkatemeren Erststrang-cDNA von der nicht konkatemeren cDNA nach Größe umfasst; wobei Schritt (a) optional nicht die RNase-Behandlung der circRNA-haltigen Probe einschließt.

8. Verfahren nach einem der vorhergehenden Ansprüche, das ferner das Amplifizieren der cDNA mit einer DNA-Polymerase umfasst; wobei die DNA-Polymerase optional Phi29 ist; wobei das Verfahren optional ferner das Behandeln der amplifizierten Erststrang-cDNA mit einer Nuklease zur Entfernung von Verzweigung umfasst.

9. Verfahren nach einem der vorhergehenden Ansprüche, wobei die Menge von cDNA-Kopien der zirkulären RNA innerhalb eines Konkatemers eine mindestens 2-fach höhere Konzentration von Kopien umfasst als unter Verwendung einer retroviralen RT erhalten werden kann.

10. Ein Verfahren zum Analysieren der Transkriptionsgenauigkeit einer RNA-Polymerase, das Folgendes umfasst:
(a) Auswählen einer synthetischen linearen DNA;
(b) Transkribieren der DNA mit einer DNA-abhängigen RNA-Polymerase in einem Reaktionsgemisch;
(c) Produzieren einer zirkularisierten RNA aus (b);
(d) Reverse-Transkribieren der zirkulären RNA (circRNA) mit einer Gruppe-II-Intron-Reverse-Transkriptase (Intron-RT), um eine Population von konkatemerer cDNA zu bilden, und optional Amplifizieren der cDNA mit einer DNA-Polymerase; und
(e) Sequenzieren der Population von cDNA, das optional das Long-Read-Sequenzieren umfasst, um die Transkriptionsgenauigkeit der RNA-Polymerase zu bestimmen.

11. Verfahren nach Anspruch 10, das ferner das Bestimmen der Fehlerrate der RNA-Polymerase aus dem Auftreten von Fehlern in den Konsensussequenzen in individuellen cDNAs umfasst.

12. Ein Reaktionsgemisch, das Folgendes umfasst:
(a) eine zirkuläre RNA (circRNA), wobei die circRNA aus Folgenden ausgewählt ist: einer eukaryotischen circRNA, und einer synthetischen circRNA mit einer künstlichen Sequenz; und
(b) eine bakterielle oder archaeale Gruppe-II-Intron-Reverse-Transkriptase (Intron-RT).

13. Reaktionsgemisch nach Anspruch 12, wobei:
die circRNA eine eukaryotische circRNA ist; wobei die eukaryotische circRNA aus einer Zelle oder Körperflüssigkeit isoliert wurde oder wobei das Reaktionsgemisch eine Probe eines Zelllysats oder einer Körperflüssigkeit enthält, die die eukaryotische circRNA umfasst; oder
die eukaryotische circRNA oder die synthetische circRNA eine zirkularisierte lineare RNA ist.

14. Reaktionsgemisch nach Anspruch 12 oder Anspruch 13, wobei die circRNA eine Größe im Bereich von 20 Basen bis 50 Kilobasen aufweist.

15. Reaktionsgemisch nach einem der Ansprüche 12-14, das ferner ein DNA-Oligonukleotid umfasst;
wobei das DNA-Oligonukleotid optional ein Primer oder ein Adapter ist; wie etwa wobei das Oligonukleotid ein Primer ist, der Folgendes umfasst: ein 3'-Ende mit einer zielspezifischen komplementären Sequenz zum Hybridisieren an die zirkuläre RNA oder einer degenerierten Sequenz an dem 3'-Ende, und optional einen 5'-Schwanz.

16. Reaktionsgemisch nach einem der Ansprüche 12-15, das ferner ein oder mehrere Enzyme umfasst, die aus der Gruppe ausgewählt sind, die aus einer 5'-3'-RNA-Exonuklease, einer 3'-5'-RNA-Exonuklease, einer DNA-Polymerase, einer Ligase, einer thermolabilen Proteinase und einer Endonuklease besteht;
wobei das Reaktionsgemisch optional eine DNA-Polymerase umfasst, wie etwa wobei die DNA-Polymerase Phi29, Tag, Bst, Bst Large Fragment, Bsu, Bsu Large Fragment, E.coli-Polymerase I, Klenow, Deep Vent, Vent, Pfu, KOD, Tgo oder 9°N-DNA-Polymerase ist.

17. Reaktionsgemisch nach einem der Ansprüche 12-16, das ferner konkatemere Erststrang-cDNAs umfasst, wobei jede konkatemere cDNA mehrere Repetiereinheiten umfasst, die komplementäre Kopien von circRNA sind, und wobei die mediane Länge der konkatemeren cDNA mindestens das 3-Fache der Länge der circRNA beträgt;
wobei die cDNA in dem Reaktionsgemisch optional mindestens 20 komplementäre Kopien der circRNA umfasst oder wobei die cDNA mindestens 500 Nukleotide umfasst; und/oder wobei die Repetiereinheiten in einer einzelnen cDNA eine mehr als 90%ige Sequenzidentität gemeinsam haben.

18. Ein Kit, der Folgendes umfasst:
ein Gruppe-II-Intron-Reverse-Transkriptase-Enzym (Intron-RT); und
Reagenzien zur Zirkularisierung einer linearen RNA, die aus einem der Folgenden ausgewählt sind: einem Splint-Adapter, einer RNA-Ligase wie etwa T4-RNA-Ligase, einem Ribozym oder Chemikalien, die in der Lage sind, die RNA zu zirkularisieren.

19. Kit nach Anspruch 18, der ferner mindestens ein Enzym umfasst, das aus der Gruppe ausgewählt ist, die aus Folgenden besteht:
einer DNA-abhängigen RNA-Polymerase; einer 5'-3'-RNA-Exonuklease; einer 3'-5'-RNA-Exonuklease; einer DNA-Polymerase; einer Ligase; einer thermolabilen Proteinase; und einer Endonuklease;
wobei eines oder mehrere der Enzyme optional in dem gleichen Behälter wie die Gruppe-II-Intron-Reverse-Transkriptase (Intron-RT) sind oder wobei eines oder mehrere der Enzyme in einem oder mehreren anderen Behältern als das Gruppe-II-Intron-Reverse-Transkriptase-Enzym (Intron-RT) sind.

20. Kit nach Anspruch 18 oder Anspruch 19, wobei mindestens eines der Enzyme in dem Kit lyophilisiert ist;
wobei sich das lyophilisierte Enzym optional auf der Oberfläche eines Polymers, innerhalb einer porösen Polymermatrix oder in einem Kuchen innerhalb eines Röhrchens befindet.

## Revendications

1. Procédé d'identification d'un ARN circulaire (ARNcirc) dans un échantillon par caractérisation d'une molécule d'ADNc de premier brin, comprenant :
(a) l'incubation d'un échantillon comprenant un ARNcirc avec une transcriptase inverse d'intron du groupe II (Intron-RT) et des dNTP, afin de produire par transcription inverse en cercle roulant un produit réactionnel comprenant des molécules concatémériques d'ADNc de premier brin ; et
(b) la caractérisation de l'ADNc par :
(i) obtention d'une séquence des molécules concatémériques d'ADNc de premier brin, la séquence étant une séquence consensus complémentaire d'unités de répétition de l'ARNcirc ; optionnellement, l'étape de séquençage de l'ADNc étant précédée d'une étape de détection de l'ADNc par amplification ;
ou
(ii) amplification de l'ADNc de premier brin concatémérique par une réaction d'amplification de l'ADN à l'aide d'amorces.

2. Procédé selon la revendication 1, dans lequel l'ARNcirc est :
isolé à partir d'un échantillon contenant des cellules eucaryotes ou un fluide corporel ;
présent dans un lysat cellulaire ou un fluide corporel ;
un ARN linéaire circularisé ;
synthétisé in vitro en créant une séquence artificielle ; ou
un ARN linéaire circularisé qui est un produit de transcription d'un ADN.

3. Procédé selon la revendication 1 ou la revendication 2, dans lequel (a) comprend en outre :
un enrichissement de l'ARNcirc en ARN total par dégradation d'ARN linéaire avec une RNase 5'-3' et une RNase 3'-5' ;
la combinaison d'un oligonucléotide d'ADN ou d'un ribozyme avec l'ARNcirc et l'Intron-RT, l'oligonucléotide étant optionnellement une amorce ou un adaptateur ;
et/ou
la formation de l'ADNc concatémérique à une température dans la plage de 20 °C à 60 °C, (a) comprenant par exemple une incubation de l'échantillon avec l'Intron-RT à une température dans la plage de 50 °C à 60 °C afin de réduire la formation de la structure secondaire de l'ARN.

4. Procédé selon l'une quelconque des revendications 1 à 3, dans lequel (b) comprend en outre :
une amplification du concatémère d'ADNc de premier brin de pleine longueur en utilisant un procédé d'amplification à amorce aléatoire, pour produire un concatémère amplifié ; et/ou
l'alignement de multiples séquences répétées pour obtenir une séquence consensus.

5. Procédé selon l'une quelconque des revendications précédentes, dans lequel le premier brin concatémérique a une longueur d'au moins 500 bases et contient au moins 3 copies complémentaires de l'ARNcirc.

6. Procédé selon l'une quelconque des revendications précédentes, dans lequel le séquençage de l'ADNc de premier brin concatémérique dans (i) se fait par séquençage à lecture longue ; ou dans lequel le séquençage du produit d'amplification de l'ADNc de premier brin dans (i) se fait par séquençage à lecture longue.

7. Procédé selon l'une quelconque des revendications précédentes, comprenant en outre une séparation par taille de l'ADNc de premier brin concatémérique de l'ADNc non concatémérique ; optionnellement dans lequel l'étape (a) ne comprend pas de traitement par RNase de l'échantillon contenant l'ARNcirc.

8. Procédé selon l'une quelconque des revendications précédentes, comprenant en outre une amplification de l'ADNc avec une ADN polymérase ; optionnellement dans lequel l'ADN polymérase est Phi29 ; le procédé comprenant optionnellement en outre un traitement de l'ADNc de premier brin amplifié avec une nucléase pour supprimer les ramifications.

9. Procédé selon l'une quelconque des revendications précédentes, dans lequel la quantité de copies d'ADNc de l'ARN circulaire dans un concatémère produit une concentration de copies au moins 2 fois supérieure à celle que l'on peut obtenir en utilisant une transcription inverse rétrovirale.

10. Procédé permettant de tester la fidélité de transcription d'une ARN polymérase, comprenant :
(a) la sélection d'un ADN linéaire synthétique ;
(b) la transcription de l'ADN avec une ARN polymérase dépendante de l'ADN dans un mélange réactionnel ;
(c) la production d'un ARN circularisé à partir de (b) ;
(d) une transcription inverse de l'ARN circulaire (ARNcirc) avec une transcriptase inverse d'intron du groupe II (Intron-RT) pour former une population d'ADNc concatémérique, et optionnellement une amplification de l'ADNc avec une ADN polymérase ; et
(e) un séquençage de la population d'ADNc, comprenant optionnellement un séquençage d'ADN à lecture longue, pour déterminer la fidélité de transcription de l'ARN polymérase.

11. Procédé selon la revendication 10, comprenant en outre une détermination du taux d'erreur de l'ARN polymérase à partir de la survenue d'erreurs dans les séquences consensus d'ADNc individuels.

12. Mélange réactionnel, comprenant :
(a) un ARN circulaire (ARNcirc), l'ARNcirc étant sélectionné parmi : un ARNcirc eucaryote, et un ARNcirc synthétique ayant une séquence artificielle ; et
(b) une transcriptase inverse d'intron du groupe II (Intron-RT) d'origine bactérienne ou archéenne.

13. Mélange réactionnel selon la revendication 12, dans lequel :
l'ARNcirc est un ARNcirc eucaryote ; l'ARNcirc eucaryote ayant été isolé à partir d'une cellule ou d'un fluide corporel, ou le mélange réactionnel comprenant un échantillon d'un lysat cellulaire ou d'un fluide corporel comprenant l'ARNcirc eucaryote ; ou
l'ARNcirc eucaryote ou l'ARNcirc synthétique est un ARN linéaire circularisé.

14. Mélange réactionnel selon la revendication 12 ou la revendication 13, dans lequel l'ARNcirc a une taille dans la plage de 20 bases à 50 kilobases.

15. Mélange réactionnel selon l'une quelconque des revendications 12 à 14, comprenant en outre un oligonucléotide d'ADN ;
optionnellement dans lequel l'oligonucléotide d'ADN est une amorce ou un adaptateur ; par exemple dans lequel l'oligonucléotide est une amorce comprenant : une extrémité 3' ayant une séquence complémentaire spécifique à la cible pour une hybridation à l'ARN circulaire, ou une séquence dégénérée à l'extrémité 3', et optionnellement une queue 5'.

16. Mélange réactionnel selon l'une quelconque des revendications 12 à 15, comprenant en outre une ou plusieurs enzymes sélectionnées dans le groupe constitué d'une ARN exonucléase 5'-3', d'une ARN exonucléase 3'-5', d'une ADN polymérase, d'une ligase, d'une protéinase thermolabile et d'une endonucléase ;
le mélange réactionnel comprenant optionnellement une ADN polymérase, par exemple dans lequel l'ADN polymérase est Phi29, Taq, Bst, un grand fragment de Bst, Bsu, un grand fragment de Bsu, la polymérase I *d'E. coli,* Klenow, Deep Vent, Vent, Pfu, KOD, Tgo ou l'ADN polymérase 9°N.

17. Mélange réactionnel selon l'une quelconque des revendications 12 à 16, comprenant en outre des ADNc de premier brin concatémériques, dans lequel chaque ADNc concatémérique contient de multiples unités de répétition qui sont des copies complémentaires d'ARNcirc, et dans lequel la longueur médiane de l'ADNc concatémérique est d'au moins 3 fois la longueur de l'ARNcirc ;
optionnellement dans lequel l'ADNc dans le mélange réactionnel comprend au moins 20 copies complémentaires de l'ARNcirc, ou dans lequel l'ADNc contient au moins 500 nucléotides ; et/ou dans lequel les unités de répétition dans un même ADNc partagent une identité de séquence de plus de 90 %.

18. Trousse comprenant :
une enzyme transcriptase inverse d'intron du groupe II (Intron-RT) ; et
des réactifs pour circulariser un ARN linéaire, sélectionnés parmi n'importe lesquels de : un adaptateur de type attelle, une ARN ligase telle que l'ARN ligase T4, un ribozyme, ou des substances chimiques capables de circulariser l'ARN.

19. Trousse selon la revendication 18, comprenant en outre au moins une enzyme sélectionnée dans le groupe constitué de :
une ARN polymérase dépendante de l'ADN ; une ARN exonucléase 5'-3' ; une ARN exonucléase 3'-5' ; une ADN polymérase ; une ligase ; une protéinase thermolabile ; et une endonucléase ;
optionnellement dans laquelle une ou plusieurs des enzymes se trouvent dans le même contenant que la transcriptase inverse d'intron du groupe II (Intron-RT), ou dans laquelle une ou plusieurs des enzymes se trouvent dans un ou plusieurs contenants différents de l'enzyme transcriptase inverse d'intron du groupe II (Intron-RT).

20. Trousse selon la revendication 18 ou la revendication 19, au moins une des enzymes dans la trousse étant lyophilisée ;
optionnellement dans laquelle l'enzyme lyophilisée est présente sur la surface d'un polymère, à l'intérieur d'une matrice polymère poreuse, ou dans un gâteau à l'intérieur d'un tube.
